# EUROPEAN PATENT SPECIFICATION

(11) **EP 2 152 742 B1**
(45) Date of publication and mention of the grant of the patent: **23.01.2013**
(21) Application number: 08743270.4
(22) Date of filing: 23.04.2008
(51) Int. Cl.: C07K 14/72, A61K 38/30

(54) **IGF-1R binding proteins and antagonists**
IGF-1R-bindende Proteine und Antagonisten
Protéines de liaison à l'IGF-1R et antagonistes

(30) Priority: 24.04.2007 US 925982 P
(43) Date of publication of application: 17.02.2010
(73) Proprietor: Antyra, Inc., Edison, New Jersey 08818 (US); Novo Nordisk A/S, 2880 Bagsværd (DK)
(72) Inventor: OSTERGAARD, Soren, DK-2880 Bagsvaerd (DK); PILLUTLA, Renuta, Edison, NJ 08818 (US); FLETCHER, Paul, Edison, NJ 08818 (US); SCHAFFER, Lauge, DK-2880 Bagsvaerd (DK); MATHIASEN, Ida Heise, Stenfeldt, DK-2880 Bagsvaerd (DK); GOLDSTEIN, Neil, L., Edison, NJ 08818 (US); SPETZLER, Jane, DK-2880 Bagsvaerd (DK)
(74) Representative: Hart, Deborah Mary
(86) International application number: PCT/US2008/005316
(87) International publication number: WO 2008/133961

(56) References cited:
- US-A1- 2004 023 887
- WETTERAU L A ET AL: "Novel aspects of the insulin-like growth factor binding proteins" MOLECULAR GENETICS AND METABOLISM, ACADEMIC PRESS, SAN DIEGO, CA, US, vol. 68, no. 2, 1 October 1999 (1999-10-01), pages 161-181, XP002306791 ISSN: 1096-7192
- DURAI RAJARAMAN ET AL: "The role of the insulin-like growth factor system in colorectal cancer: review of current knowledge" INTERNATIONAL JOURNAL OF COLORECTAL DISEASE, vol. 20, no. 3, May 2005 (2005-05), pages 203-220, XP002495771 ISSN: 0179-1958
- MITSIADES C S ET AL: "TREATMENT OF HEMATOLOGIC MALIGNANCIES AND SOLID TUMORS BY INHIBING IGF RECEPTOR SIGNALING" EXPERT REVIEW OF ANTICANCER THERAPY, FUTURE DRUGS, LONDON, GB, vol. 5, no. 3, 1 January 2005 (2005-01-01), pages 487-499, XP009055818 ISSN: 1473-7140
- HADDAD TUFIA ET AL: "TARGETING THE INSULIN-LIKE GROWTH FACTOR AXIS AS A CANCER THERAPY" FUTURE ONCOLOGY, FUTURE MEDICINE LTD., LONDON, GB, vol. 2, no. 1, 1 February 2006 (2006-02-01), pages 101-110, XP008079502 ISSN: 1479-6694
- RIEDEMANN J ET AL: "IGF1R signalling and its inhibition." ENDOCRINE-RELATED CANCER DEC 2006, vol. 13 Suppl 1, December 2006 (2006-12), pages S33-S43, XP002495772 ISSN: 1351-0088

## Description

### TECHNICAL FIELD

The invention relates to molecules that bind to human insulin-like growth factor-1 receptor (IGF-1R) and, in at least some aspects, act as IGF-1R antagonists, pharmaceutical compositions comprising such molecules, and methods of making and using such compositions and molecules.

### BACKGROUND OF THE INVENTION

Insulin-like growth factor (IGF) signalling stimulates proliferation and prolongs survival of cells. Research has indicated that high levels of circulating IGF-1 are associated with increased risk of several common cancers including breast, prostate, and pancreatic cancers. A number of therapeutic strategies that target the IGF-1 receptor have demonstrated anti-cancer activity.

International Patent Application WO 03/027246 (and corresponding US Patent Publication No. 2004/0023887), which reflects the prior inventions of at least some of the inventors of the invention described herein, describes, *inter alia,* unique IGF-1R antagonists and pharmaceutical compositions comprising the same, as well as methods of producing and using such molecules and compositions (e.g., in the treatment of cancer) (related principles, methods, molecules, and compositions also are described in WO 01/72771 and US Patent Publication No. 2003/0195147). The inventors of the subject matter described herein have continued the research reflected in the '246 PCT application, and have identified additional IGF-1R binding molecules and IGF-1R antagonists with alternative and/or improved properties with respect to the antagonists described in the '246 PCT application, as well as compositions comprising the same and methods of using such compounds and compositions.

### SUMMARY OF THE INVENTION

The invention is directed to new peptides and proteins that bind to human insulin-like growth factor-1 receptor (HIGF-1R), as well as nucleic acids encoding the same, vectors and cells comprising such nucleic acids, pharmaceutical compositions comprising such compounds, and methods of using any thereof.

In certain aspects, the invention is directed to an isolated peptide, comprising: a peptide capable of binding Insulin-like growth factor 1 Receptor (IGF-1R), wherein the sequence of said peptide comprises an amino acid sequence having at least 98% identity to SEQ ID.NO: 18 (F429).

This disclosure also relates to a peptide, comprising an amino acid sequence having at least 96% identity to SEQ ID. NO: 18 (F429).

In certain embodiments, the sequence of the peptide comprises SEQ ID. NO: 18 (F429).

In certain embodiments, the sequence of the peptide consists of SEQ ID. NO: 18 (F429).

In certain aspects, the invention is directed to a pharmaceutical composition, comprising: a peptide capable of binding IGF-1R in an amount that is effective to reduce angiogenesis and/or cancer progression in a mammalian host, wherein the sequence of the peptide comprises an amino acid sequence having at least 98% identity to SEQ ID. NO: 18 (F429).

In certain embodiments, the pharmaceutical composition comprises a peptide capable of binding IGF-1R in an amount that is effective to reduce angiogenesis and/or cancer progression in a human host.

This disclosure also relates to a pharmaceutical composition comprising a peptide comprising an amino acid sequence having at least 96% identity to SEQ ID. NO: 18 (F429).

In certain embodiments, the pharmaceutical composition comprises a peptide consisting of SEQ ID. NO: 18 (F429).

In certain aspects, the invention is directed to a peptide capable of binding IGF-1R, wherein the sequence of the peptide comprises an amino acid sequence having at least 98% identity to SEQ ID. NO: 18 (F429) for use in a method of treating cancer.

In certain embodiments, the cancer to be treated is one in which IGF-1 and/or IGF-1R is expressed.

In certain embodiments, the cancer to be treated is one in which IGF-1 and/or IGF-1R is over-expressed.

In certain embodiments, the cancer to be treated is pancreatic, colorectal, breast, prostate, ovarian or gastric cancer.

This disclosure also relates to a peptide comprising an amino acid sequence having at least 96% identity to SEQ ID. NO: 18 (F429) for use in a method of treating cancer.

In certain embodiments, the peptide comprises SEQ ID. NO: 18 (F429) for use in a method of treating cancer

In certain embodiments, the peptide consists of SEQ ID. NO: 18 (F429) for use in a method of treating cancer.

In certain embodiments the peptides of the invention are for use in therapy.

In certain embodiments, the subject peptides are used in the production of a medicament.

In certain embodiments, the subject peptides are used in the manufacture of a medicament. The medicament can be for use in a method of treating cancer.

This disclosure is also directed to an isolated peptide, comprising: a peptide capable of binding IGF-1R, wherein the sequence of the peptide comprises an amino acid sequence having at least 96% identity to a sequence selected from the group consisting of SEQ ID. NO: 8 (F292), SEQ ID. NO: 9 (F293), SEQ ID. NO: 196 (F294), SEQ ID. NO: 7 (F259), SEQ ID. NO: 10 (F296), SEQ ID. NO: 11 (F297), SEQ ID. NO: 14 (F392), SEQ ID. NO: 16 (F408), SEQ ID. NO: 22 (F142), SEQ ID. NO: 21 (F230), SEQ ID. NO: 27 (F270), SEQ ID. NO: 26 (F264), SEQ ID. NO: 197 (F265), SEQ ID. NO: 136 (F298), SEQ ID. NO: 192 (F441) and SEQ ID. NO: 28 (F364) and combinations thereof.

In this disclosure, the sequence of said peptide comprises a sequence selected from the group consisting of SEQ ID. NO: 8 (F292), SEQ ID. NO: 9 (F293), SEQ ID. NO: 196 (F294), SEQ ID. NO: 7 (F259), SEQ ID. NO: 10 (F296), SEQ ID. NO: 11 (F297), SEQ ID. NO: 14 (F392), SEQ ID. NO: 16 (F408), SEQ ID. NO: 22 (F142), SEQ ID. NO: 21 (F230), SEQ ID. NO: 27 (F270), SEQ ID. NO: 26 (F264), SEQ ID. NO: 197 (F265), SEQ ID. NO: 136 (F298), SEQ ID. NO: 192 (F441) and SEQ ID. NO: 28 (F364) and combinations thereof.

This disclosure is also directed to a pharmaceutical composition comprising a peptide capable of binding IGF-1R, wherein the sequence of the peptide comprises an amino acid sequence having at least 96% identity to a sequence selected from the group consisting of SEQ ID. NO: 8 (F292), SEQ ID. NO: 9 (F293), SEQ ID. NO: 196 (F294), SEQ ID. NO: 7 (F259), SEQ ID. NO: 10 (F296), SEQ ID. NO: 11 (F297), SEQ ID. NO: 14 (F392), SEQ ID. NO: 16 (F408), SEQ ID. NO: 22 (F142), SEQ ID. NO: 21 (F230), SEQ ID. NO: 27 (F270), SEQ ID. NO: 26 (F264), SEQ ID. NO: 197 (F265), SEQ ID. NO: 136 (F298), SEQ ID. NO: 192 (F441) and SEQ ID. NO: 28 (F364) and combinations thereof in an amount that is effective to reduce angiogenesis and/or cancer progression in a mammalian host.

In this disclosure, the pharmaceutical composition comprises a peptide comprising a sequence selected from the group consisting of SEQ ID. NO: 8 (F292), SEQ ID. NO: 9 (F293), SEQ ID. NO: 196 (F294), SEQ ID. NO: 7 (F259), SEQ ID. NO: 10 (F296), SEQ ID. NO: 11 (F297), SEQ ID. NO: 14 (F392), SEQ ID. NO: 16 (F408), SEQ ID. NO: 22 (F142), SEQ ID. NO: 21 (F230), SEQ ID. NO: 27 (F270), SEQ ID. NO: 26 (F264), SEQ ID. NO: 197 (F265), SEQ ID. NO: 136 (F298), SEQ ID. NO: 192 (F441) and SEQ ID. NO: 28 (F364) and combinations thereof.

In certain aspects, this disclosure is directed to the use of a peptide in the production or preparation of a medicament for treating cancer wherein the peptide comprises an amino acid having at least 96% identity to a sequence or comprising a sequence selected from the group consisting of SEQ ID. NO: 8 (F292), SEQ ID. NO: 9 (F293), SEQ ID. NO: 196 (F294), SEQ ID. NO: 7 (F259), SEQ ID. NO: 10 (F296), SEQ ID. NO: 11 (F297), SEQ ID. NO: 14 (F392), SEQ ID. NO: 16 (F408), SEQ ID. NO: 22 (F142), SEQ ID. NO: 21 (F230), SEQ ID. NO: 27 (F270), SEQ ID. NO: 26 (F264), SEQ ID. NO: 197 (F265), SEQ ID. NO: 136 (F298), SEQ ID. NO: 192 (F441) and SEQ ID. NO: 28 (F364) and combinations thereof.

In certain aspects, this disclosure is directed to an isolated peptide, comprising a peptide capable of binding IGF-1R, wherein the sequence of the peptide comprises a sequence selected from the group consisting of Formula 1, Formula 2, Formula 3 and Formula 4 and any peptide in this application including the peptides disclosed in any of the figures of this application and combinations thereof.

In certain aspects, this disclosure is directed to a pharmaceutical composition, comprising a peptide capable of binding IGF-1R, wherein the sequence of the peptide comprises a sequence selected from the group consisting of Formula 1, Formula 2, Formula 3 and Formula 4 and any peptide in this application including the peptides disclosed in any of the figures of this application and combinations thereof.

In certain aspects, this disclosure is directed to a method of treating cancer, comprising: administering to a mammal in need thereof a therapeutically effective amount of a peptide capable of binding IGF-1R, wherein the sequence of the peptide comprises a sequence selected from the group consisting of Formula 1, Formula 2, Formula 3 and Formula 4 and any peptide in this application including the peptides disclosed in any of the figures of this application and combinations thereof.

In certain aspects, this disclosure is directed to the use of a peptide capable of binding IGF-1R, wherein the sequence of the peptide comprises a sequence selected from the group consisting of Formula 1, Formula 2, Formula 3 and Formula 4 and any peptide in this application including the peptides disclosed in any of the figures of this application and combinations thereof in the production of a medicament.

In certain aspects, this disclosure is directed to the use of a peptide capable of binding IGF-1R, wherein the sequence of the peptide comprises a sequence selected from the group consisting of Formula 1, Formula 2, Formula 3 and Formula 4 and combinations thereof in the preparation of a medicament for treating cancer.

In certain aspects, the disclosure is directed to a composition comprising a peptide antagonist of IGF-1R, wherein the peptide comprises the sequence FYxxLxxL for use in a method of treating cancer, wherein IGF-1 and/or IGF-1R are expressed.

In certain aspects, the disclosure is directed to a composition comprising a peptide antagonist of IGF-1R, wherein the peptide is at least 15 amino acids long and comprises the sequence FYxxLxxL for use in a method of treating cancer, wherein IGF-1 and/or IGF-1R are over-expressed.

In certain embodiments, the composition comprises a peptide antagonist of IGF-1R, wherein the sequence of the peptide comprises SEQ ID. NO: 18 (F429). This disclosure also encompasses a sequence selected from the group consisting of SEQ ID. NO: 20 (RP6), SEQ ID. NO: 3 (RP33/F250), SEQ ID. NO: 13 (F138) and SEQ ID. NO: 198 (RP30) and combinations thereof.

In certain aspects, the disclosure is directed to a pharmaceutical composition, comprising a peptide capable of binding IGF-1R in an amount that is effective to reduce angiogenesis and/or cancer progression in a mammalian host, wherein said peptide comprises the sequence FYxxLxxL.

In certain aspects, the disclosure is directed to a pharmaceutical composition, comprising a peptide capable of binding IGF-1R in an amount that is effective to reduce angiogenesis and/or cancer progression in a mammalian host, wherein said peptide is at least 15 amino acids long and comprises the sequence FYxxLxxL.

In certain aspects, the pharmaceutical composition comprises a peptide comprising a sequence of SEQ ID. NO: 18 (F429). This disclosure also encompasses a sequence selected from the group consisting of SEQ ID. NO: 20 (RP6), SEQ ID. NO: 3 (RP33/F250), SEQ ID. NO: 13 (F138) and SEQ ID. NO: 198 (RP30) and combinations thereof.

In certain embodiments, the disclosure is directed towards the use of a peptide capable of binding IGF-1R in an amount that is effective to reduce angiogenesis and/or cancer progression in a mammalian host, wherein the peptide comprises the sequence FYxxLxxL in the production of a medicament.

In certain embodiments, the disclosure is directed towards the use of a peptide capable of binding IGF-1R in an amount that is effective to reduce angiogenesis and/or cancer progression in a mammalian host, wherein the peptide is at least 15 amino acids long and comprises the sequence FYxxLxxL in the production of a medicament.

In certain aspects, the peptide comprises a sequence of SEQ ID. NO: 18 (F249). This disclosure also encompasses a sequence selected from the group consisting of SEQ ID. NO: 20 (RP6), SEQ ID. NO: 3 (RP33/F250), SEQ ID. NO: 13 (F138) and SEQ ID. NO: 198 (RP30) and combinations thereof.

In certain aspects, the disclosure is directed to the use of a peptide capable of binding IGF-1R in an amount that is effective to reduce angiogenesis and/or cancer progression in a mammalian host, wherein said peptide comprises the sequence FYxxLxxL in the preparation of a medicament for the treatment of cancer.

In certain aspects, the disclosure is directed to the use of a peptide capable of binding IGF-1R in an amount that is effective to reduce angiogenesis and/or cancer progression in a mammalian host, wherein said peptide is at least 15 amino acids long and comprises the sequence FYxxLxxL in the preparation of a medicament for the treatment of cancer.

In certain embodiments, the peptide comprises a sequence selected from the group consisting of SEQ ID. NO: 18 (F429). This disclosure also encompasses a sequence selected from the group consisting of SEQ ID. NO: 20 (RP6), SEQ ID. NO: 3 (RP33/F250), SEQ ID. NO: 13 (F138) and SEQ ID. NO: 198 (RP30) and combinations thereof.

### BRIEF DESCRIPTION OF THE DRAWINGS

Figures 1A and 1B depict data obtained from experiments in which SGBS adipocytes were transfected with HIR-encoding DNA to determine the effect of peptide F293 and (separately) peptide F138 on the incorporation of ³H glucose in the presence of insulin or IGF-1. The results are expressed as increase relative to full insulin response and presented graphically as effect of peptide F138 or peptide F293 or (in combination with insulin or with IGF-1) on an (approximate) ED₂₀ insulin response, with data normalized to a full insulin response, respectively.

Figure 2 depicts the results of a kinase assay performed with peptides F235 and F259 to determine IGF-1R activation. As shown in Figure 2, peptides F235 and F259 show the ability to completely inhibit IGF-1R kinase activation by IGF-1.

Figures 3A and 3B depict the results of studies measuring the activation of HIR or HIGF-1R in adipocytes by stimulating the cells to study the level of tyrosine phosphorylation (Western blot) of the insulin receptor/IGF-1R.

Figure 4 depicts the results of downstream signaling studies in which the tyrosine phosphorylation of IRS signaling (the 180 kDa band on a tyrosine phosphor Western blot) as well as activation of effectors MAPK 44 and 42 and PKB were analyzed by using antibodies specific for their active forms.

Figures 5 and 6 depict the results of cell growth/density studies in which a mitochondrial activity assay was performed with IGF-1 or F138.

Figures 7 and 8 depict the results of downstream signaling studies which tested IRS-1 phosphorylation in the presence of either F429 or F138 +/- (in the presence or absence thereof) 3 nM IGF-1.

Figures 9A-9F depict the dose related increase in cell proliferation of MiaPaCa and MCF-7 cell-based models of cancer to IGF-1, IGF-2, and Insulin.

Figures 10A-10C depict the results of binding and cell proliferation assays which reveal that F250 competes with IGF-1 binding and antagonizes its activity in cell-based cancer models. Figure 10A reflects inhibition of IGF-1 binding as a function of F250 concentration. Figure 10B reflects antagonism of IGF-1 activity in MCF-7 cells by F250. Figure 10C reflects antagonism of IGF-1 activity in MiaPaCa cells by F250.

Figures 11A-11C depict the results of experiments which demonstrate that IGF-1 stimulates phosphorylation in cancer cell models and can be blocked or reduced by candidate peptides of the invention. Figure 11A reflects that IGF-1 stimulates a transient phosphorylation of IRS-1 in MCF7 cells. The results shown in Figure 11B reflect that phosphorylation of IRS-1 in MCF7 cells induced by IGF-1 is dose-dependant.

Figure 12 depicts the results of binding and antagonism assays which exhibits the antagonistic effect of peptides F429, F441, and F408.

Figure 13 and Figure 14 depict the plots of individual and mean plasma concentration of F429 versus time following the incubation of 100 µg/mL F429.

Figure 15 depicts the logarithm of the remaining Test Article F429.

Figures 16A and 16B depict gene arrays in which gene expression changes were analyzed between MiaPaCa cells grown with IGF-1 as compared to those with ANT-429.

Figure 17 provides a list of genes which were shown to be down-regulated in ANT-429 treated cells.

Figures 18A and 18B provide a list of genes that were up-regulated or down-regulated when treated with ANT-429.

Figures 19A and 19B present data demonstrating that ANT-429 inhibits tumor growth.

Figure 20 presents data demonstrating that ANT-429 is not toxic *in vivo.*

Figure 21 presents data demonstrating the stability of ANT-429 in human plasma.

### DETAILED DESCRIPTION OF THE INVENTION

Before describing the present invention in detail, it is to be understood that unless otherwise indicated, this invention is not limited to particular formulations, active and inactive agents, modes of administration, or methods of treatment or use, as such may vary. It is also to be understood that the terminology used herein is for the purpose of describing particular embodiments only, and is not intended to be limiting.

The invention described herein provides novel proteins and peptides that bind to, and typically act as antagonists at, a human IGF-1R, having certain chemical properties as defined by comprising, consisting essentially, or consisting of an amino acid sequence according to one of the formulas provided herein and/or according to a specific amino acid sequence set forth herein.

A "peptide" in the context of this invention means a single chain amino acid sequence compound of any length but preferably consisting of about 15 to about 40, such as about 20 to about 35 (e.g., about 25 to about 30) amino acid residues. A protein in the context of this invention means any protein (whether comprising one or more chains, monomeric or multimeric, etc.) comprising at least one chain that is significantly larger than a peptide (e.g., comprising at least about 40 amino acid residues) that contains an amino acid sequence according to one of the formulas provided herein or that is otherwise specifically provided herein. Peptide and protein aspects of the invention may vary significantly in terms of, e.g., ease of production, stability, administration, etc. Typically, the methods of the invention are practiced with, and the compositions of the invention comprise, a peptide.

The terms "peptide" and "protein" herein both generally encompass (and inherently provide support for) "derivatives" of such amino acid polymers. A "derivative" refers to a protein, peptide, or amino acid sequence in which one or more of the amino acid residues thereof have been artificially chemically modified (e.g., by alkylation, acylation, ester formation, amide formation, or other similar type of modification), such as through covalent association with one or more heterologous substituents (e.g., a lipophilic substituent, a PEG moiety, a peptide side chain linked by a suitable organic moiety linker, etc.). A derivative wherein a heterologous substituent of significant size, such as a PEG moiety, peptide side chain, or the like, is attached to the "backbone" amino acid sequence, the derivative also can be described as a "conjugate." The inclusion of one or more modified amino acids in a protein or peptide of the invention, may be advantageous in, for example, (a) increasing polypeptide serum half-life, (b) reducing polypeptide antigenicity, or (c) increasing polypeptide storage stability. Amino acid (s) can be modified, for example, co-translationally or post-translationally during recombinant production (e.g., N-linked glycosylation at introduced N-X-S/T motifs during expression in mammalian cells) or modified by synthetic means. Non-limiting examples of a modified amino acid include a glycosylated amino acid, a sulfated amino acid, a prenlyated (e.g., famesylated, geranylgeranylated) amino acid, an acetylated amino acid, an acylated amino acid, a PEGylated amino acid, a biotinylated amino acid, a carboxylated amino acid, a phosphorylated amino acid, and the like. References adequate to guide one of skill in the modification of amino acids are replete throughout the literature. Exemplary protocols are found in, e.g., Walker ( 1998) PROTEIN PROTOCOLS ON CD-ROM Humana Press, Towata, NJ. Thus, for example, a modified amino acid that may be included in a derivative can be selected from a glycosylated amino acid, a PEGylated amino acid, a famesylated amino acid, an acetylated amino acid, a biotinylated amino acid, an amino acid conjugated to a lipid moiety, and an amino acid conjugated to an organic derivatizing agent. Proteins and peptides also can be chemically modified by covalent conjugation to a polymer to increase their circulating half-life, for example. Exemplary polymers and methods to attach such polymers to peptides are illustrated in, e.g., U.S. Pat. Nos. 4,766,106; 4,179,337; 4,495,285; and 4,609,546. Additional illustrative polymers include polyoxyethylated polyols and polyethylene glycol (PEG) moieties (e.g., a fusion protein can be conjugated to a PEG with a molecular weight of between about 1,000 and about 40,000, such as between about 2000 and about 20,000, e.g., about 3,000-12,000). Proteins and peptides also or alternatively may be conjugated to a second molecule that is able to impart novel biological/pharmacological properties to the protein derivative, such as a radionuclide, an enzyme substrate, a cofactor, a fluorescent marker, a chemiluminescent marker, another peptide tag, a magnetic particle, or drug. Other examples of derivatized amino acids are described in, e.g., US Patent 6,800,740.

In one aspect, peptides of the invention are provided that have a molecular weight ("MW") of about 1500 to about 6000, such as about 1750-5000. In a more particular aspect, peptides having a MW of about 2000 to about 4000, such as about 2000 to about 3000, or even about 2000 to about 2500 are provided.

Peptides and proteins provided by the invention typically have an affinity (K_{d}) of between about 10⁻⁷ to about 10⁻¹⁵ M for a human IGF-1R. Typically, the affinity is 10⁻⁸ to about 10⁻¹² M, such as about 10⁻¹⁰ to about 10⁻¹² M. For use as a reagent in a competitive binding assay to identify other ligands, the amino acid sequence typically has an affinity for the receptor of between about 10⁻⁵ to about 10⁻¹² M. Select peptides and proteins of the invention have an affinity for HIGF-1R of about 5 x 10⁻⁶ to about 10⁻⁹ to 10⁻¹¹ M (e.g., about 5 x 10⁻⁹ or about 10⁻¹⁰ M).

Usually, the peptides and proteins of the invention have a greater affinity for human IGF-1R (HIGF-1R) than for the human insulin receptor (HIR) and often exhibit such selectivity by at least about 10 fold, at least about 20 fold, at least about 50 fold, at least about 100 fold, or more. In one aspect, the peptide shows no detectable affinity for HIR, e.g., as determined by using the methods described in the Experimental Methods and Data section of this document.

Peptides and proteins provided by the invention can act as IGF-1R antagonists. Antagonism can be measured by any suitable methodology, such as by a detectable reduction in IGF-1R activity and/or signaling. In a more particular aspect, peptides and proteins provided by the invention exhibit IGF-1R antagonism without having a significant, or perhaps even a detectable, effect with respect to insulin or IGF-induced glucose uptake. In a further aspect, peptides and proteins of the invention also desirably exhibit a detectable, and desirably therapeutically beneficial, anti-angiogenic effect and/or other anti-cancer effect in a mammalian host, such as a human patient.

"Analogs" of specific peptides/sequences described herein, having a high level of identity to the specific "parent" (reference) peptide/sequence, but comprising one or more insertions, deletions, additions, and/or substitutions (of amino acid residues in the parent sequence/peptide), can exhibit IGF-1R-binding and in some cases IGF-1R antagonist properties similar to such a parent peptide/sequence (e.g., at least about 33% of, at least about 50% of, or at least about 75% of the affinity and/or activity exhibited by the parent peptide, and in some cases about 100%, or even more than 100% (e.g., at least about 125%) of the activity and/or affinity exhibited by the parent peptide/sequence can be associated with such an analog). Typically, most of the substitutions made are "conservative" in nature, and deletions and/or insertions are avoided. Conservative substitutions can be defined by substitutions within the classes of amino acids reflected in one or more of the following three amino acid classification tables:

**Table 1 - Amino Acid Residue Classes for Conservative Substitutions**

| Amino Acid Class | Amino Acid Residues |
|---|---|
| Acidic Residues | ASP and GLU |
| Basic Residues | LYS, ARG, and HIS |
| Hydrophilic Uncharged Residues | SER, THR, ASN, and GLN |
| Aliphatic Uncharged Residues | GLY, ALA, VAL, LEU, and ILE |
| Non-polar Uncharged Residues | CYS, MET, and PRO |
| Aromatic Residues | PHE, TYR, and TRP |

**Table 2 - Alternative Conservative Amino Acid Residue Substitution Groups**

| | | | |
|---|---|---|---|
| 1 | Alanine (A) | Serine (S) | Threonine (T) |
| 2 | Aspartic acid (D) | Glutamic acid (E) | |
| 3 | Asparagine (N) | Glutamine (Q) | |
| 4 | Arginine (R) | Lysine (K) | |
| 5 | Isoleucine (I) | Leucine (L) | Methionine (M) |
| 6 | Phenylalanine (F) | Tyrosine (Y) | Tryptophan (W) |

**Table 3 - Alternative Physical and Functional Classifications of Amino Acid Residues**

| | |
|---|---|
| Alcohol group-containing residues | S and T |
| Aliphatic residues | I, L, V, and M |
| Cycloalkenyl-associated residues | F, H, W, and Y |
| Hydrophobic residues | A, C, F, G, H, I, L, M, R, T, V, W, and Y |
| Negatively charged residues | D and E |
| Polar residues | C, D, E, H, K, N, Q, R, S, and T |
| Small residues | A, C, D, G, N, P, S, T, and V |
| Very small residues | A, G, and S |
| Residues involved in turn formation | A, C, D, E, G, H, K, N, Q, R, S, P, and T |
| Flexible residues | E, Q, T, K, S, G, P, D, E, and R |

Even more conservative amino acid residue substitution groupings include: valine-leucine-isoleucine, phenylalanine-tyrosine, lysine-arginine, alanine-valine, and asparagine-glutamine. Additional groups of amino acids can also be formulated using the principles described in, e.g., Creighton (1984) PROTEINS: STRUCTURE AND MOLECULAR PROPERTIES (2d Ed. 1993), W.H. Freeman and Company. In some instances it can be useful to further characterize substitutions based on two or more of such features (e.g., substitution with a "small polar" residue, such as a Thr residue, can represent a highly conservative substitution in an appropriate context).

It also can be the case that known synthetic, rare, or modified amino acid residues having known similar physiochemical properties to those identified in one of the above-described groupings can be used as a "conservative" substitute for a particular amino acid residue in a sequence. For example, a D-Arg residue may serve as a substitute for a typical (L-) Arg residue. It also can be the case that a particular substitution can be described in terms of two or more of the above described classes (e.g., a substitution with a small and hydrophobic residue would mean with residues that are found in both of the above-described classes or other synthetic, rare, or modified residues that are known in the art to have similar physiochemical properties to such residues meeting both definitions).

In many cases, analogs herein are described with respect of the substitution of a particular residue. In such cases, a conservative substitution is judged in respect of the residue that normally occurs in the position. In certain cases, analogs also may be described herein by a substitution of a residue with one or more particular residues that typically have been determined by prior study (as described herein). In such cases, a conservative substitution can be judged by both the residue being substituted and the residues identified as being suitable for substitution of that residue. In general, any suitable amino acid residue can replace a substituted residue. Typically, a residue that substitutes another residue is one of the twenty residues that are frequently incorporated in human proteins, though artificial, rare, and derivatized amino acid residues also can be incorporated into peptides/sequences of the invention. Suitability is typically judged by retention of biological function. Those skilled in protein engineering will also be able to recognize certain substitutions are not suitable due to improper introduction of regions of flexibility or rigidity; removal of functionally significant residues; or removal of residues that provide important structural characteristics.

Substantial changes in protein/domain/sequence function can be made by selecting substitutions that are less conservative than those shown in the defined groups, above. Thus, in some aspects, an analog can include one or more non-conservative residues are included. For example, non-conservative substitutions can be made which more significantly affect the structure of the peptide in the area of the alteration; the charge or hydrophobicity of the molecule at the target site; or the bulk of the side chain. The substitutions which generally are expected to produce the greatest changes in the peptide's properties are those where 1) a hydrophilic residue, e.g., seryl or threonyl, is substituted for (or by) a hydrophobic residue, e.g., leucyl, isoleucyl, phenylalanyl, valyl, or alanyl; 2) a cysteine or proline is substituted for (or by) any other residue; 3) a residue having an electropositive side chain, e.g., lysyl, arginyl, or histidyl, is substituted for (or by) an electronegative residue, e.g., glutamyl or aspartyl; or 4) a residue having a bulky side chain, e.g., phenylalanine, is substituted for (or by) a residue that does not have a side chain, e.g., glycine. Accordingly, these and other nonconservative substitutions can be introduced into peptide analogs where significant changes in function/structure is desired and such changes avoided where conservation of structure/function is desired.

Those skilled in the art will be aware of additional principles useful in the design and selection of peptide analogs. For example, residues in surface positions of a peptide typically a strong preference for hydrophilic amino acids. Steric properties of amino acids can greatly affect the local structures that a protein adopts or favors. Proline, for example, exhibits reduced torsional freedom that can lead to the conformation of the peptide backbone being locked in a turn and with the loss of hydrogen bonding, often further resulting in the residue appearing on a surface loop of a protein. In contrast to Pro, Gly has complete torsional freedom about a main peptide chain, such that it is often associated with tight turns and regions buried in the interior of the protein (e.g., hydrophobic pockets). The features of such residues often limit their involvement in secondary structures. However, residues typically involved in the formation of secondary structures are known. For example, residues such as Ala, Leu, and Glu (amino acids without much bulk and/or polar residues) typically are associated with alpha-helix formation, whereas residues such as Val, Ile, Ser, Asp, and Asn can disrupt alpha helix formation. Residues with propensity for beta-sheet structure formation/inclusion include Val and Ile and residues associated with turn structures include Pro, Asp, and Gly. The skilled artisan can consider these and similar known amino acid properties in the design and selection of suitable peptide analogs, such that suitable analogs can be prepared with only routine experimentation.

Frequently, conservation in terms of hydropathic/hydrophilic properties also is substantially retained in a analog peptide as compared to a parent peptide (e.g., the weight class, hydropathic score, or both of the sequences are at least about 50%, at least about 60%, at least about 70%, at least about 75%, at least about 80%, at least about 85%, at least about 90%, at least about 95%, or more (e.g., about 65-99%) retained). Methods for assessing the conservation of the hydropathic character of residues/sequences are known in the art and incorporated in available software packages, such as the GREASE program available through the SDSC Biology Workbench (see also, e.g., Kyte and Doolittle et al., J. Mol. Biol. 157:105-132(1982); Pearson and Lipman, PNAS (1988) 85:2444-2448, and Pearson (1990) Methods in Enzymology 183:63-98 for a discussion of the principles incorporated in GREASE and similar programs).

Thus, in making substitutions, deletions, insertions, additions, and the like, the hydropathic index of amino acids may be considered (in addition to other factors). Each amino acid has been assigned a hydropathic index on the basis of their hydrophobicity and charge characteristics, these are: isoleucine (+4.5); valine (+4.2); leucine (+3.8); phenylalanine (+2.8); cysteine/cysteine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The importance of the hydropathic amino acid index in conferring interactive biological function on a protein is understood in the art. Kyte et al., J. Mol. Biol., 157:105-131 (1982). It is known that certain amino acids may be substituted for other amino acids having a similar hydropathic index or score and still retain a similar biological activity. In making changes based upon the hydropathic index, the substitution of amino acids whose hydropathic indices are within ±2 is typically preferred, those that are within ±1 are commonly particularly preferred, and those within ±0.5 typically are even more particularly preferred.

It is also understood in the art that, e.g., the substitution of like amino acids, can be made effectively on the basis of hydrophilicity, particularly where the biologically functionally equivalent protein or peptide thereby created is intended for use in immunological embodiments, as in the present case. The greatest local average hydrophilicity of a protein, as governed by the hydrophilicity of its adjacent amino acids, correlates with its immunogenicity and antigenicity, i.e., with a biological property of the protein.

The following hydrophilicity values have been assigned to amino acid residues: arginine (+3.0); lysine ('3.0); aspartate (+3.0±1); glutamate (+3.0±1); serine (+0.3); asparagine (+0.2); glutamine (+0.2); glycine (0); threonine (-0.4); proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). In making changes based upon similar hydrophilicity values, the substitution of amino acids whose hydrophilicity values are within ±2 is typically preferred, those that are within ±1 commonly are particularly preferred, and those within ±0.5 usually are even more particularly preferred.

It also is advantageous that structure of the analog peptide is substantially similar to the structure of the parent peptide. Methods for assessing similarity of peptides in terms of conservative substitutions, hydropathic properties, weight conservation, and similar considerations are described in e.g., International Patent Applications WO 03/048185, WO 03/070747, and WO 03/027246. Exemplary methods for producing functional analog proteins and sequences (e.g., by "DNA shuffling," "rational design" methods, alanine scanning techniques, and random mutagenesis methods) also are described in these and other references cited herein. Structural determinations can be made by any suitable technique, such as nuclear magnetic resonance (NMR) spectroscopic structure determination techniques, which are well-known in the art (See, e.g., Wuthrich, NMR of Proteins and Nucleic Acids, Wiley, New York, 1986; Wuthrich, K. Science 243:45-50 (1989); Clore et al., Crit. Rev. Bioch. Molec. Biol. 24:479-564 (1989); Cooke et al. Bioassays 8:52-56 (1988)), typically in combination with computer modeling methods (e.g., by use of programs such as MACROMODEL™, INSIGHT™, and DISCOVER™, to obtain spatial and orientation requirements for structural analogs. Using information obtained by these and other suitable known techniques, structural analogs can be designed and produced through rationally-based amino acid substitutions, insertions, and/or deletions. Such structural analogs may be useful in practicing methods of the invention. A number of scientific publications have been devoted to the prediction of secondary structure. See Moult J., Curr. Op. in Biotech., 7(4):422-427 (1996), Chou et al., Biochemistry, 13(2):222-245 (1974); Chou et al., Biochemistry, 113(2):211-222 (1974); Chou et al., Adv. Enzymol. Relat. Areas Mol. Biol, 47:45-148 (1978); Chou et al., Ann. Rev. Biochem., 47:251-276 and Chou et al., Biophys. J., 26:367-384 (1979). Various computer programs are currently available to assist with predicting secondary structure. One method of predicting secondary structure is based upon homology modeling. The recent growth of the protein structural data base (PDB) has provided enhanced predictability of secondary structure, including the potential number of, folds within a polypeptide's or protein's structure. See Holm et al., Nucl. Acid. Res., 27(1):244-247 (1999); see also Brenner et al., Curr. Op. Struct. Biol., 7(3):369-376 (1997) (for a discussion of related principles). Additional methods of predicting secondary structure include "threading" techniques (see, e.g., Jones, D., Curr. Opin. Struct. Biol., 7(3):377-87 (1997); Sippl et al., Structure, 4(1):15-9 (1996)), "profile analysis" (Bowie et al., Science, 253:164-170 (1991); Gribskov et al., Meth. Enzymol., 183:146-159 (1990); Gribskov et al., Proc. Nat. Acad. Sci., 84(13):4355-4358 (1987)), and "evolutionary linkage" methods (See Home, supra, and Brenner, supra).

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include a peptide with an N-terminal methionyl residue or fusion to an epitope tag. Other insertion analogs of peptide molecules include the fusion to (typically to the N- or C-terminus) of the peptide amino acid sequence of an enzyme, another polypeptide, or a PEG, which increases the serum half-life of the chain.

Typically, advantageous sequence changes are those that (1) reduce susceptibility to proteolysis, (2) reduce susceptibility to oxidation, (3) alter binding affinity of the analog sequence (typically desirably increasing affinity), and/or (4) confer or modify other physicochemical or functional properties on the associated analog sequence/analog peptide.

Typically, an analog of a specific peptide or sequence described herein can be characterized as a protein, peptide, or sequence that exhibits at least about 50%, typically at least about 75%, such as about 100%, or more, of at least one of the defined functional characteristics of the disclosed peptide/sequence disclosed herein (e.g., IGF-IR binding and/or IGF-1 R antagonism).

Peptides and proteins of the invention, also may be derivatized by the addition of any one or more derivatives (e.g., PEG moieties, acyl moieties, etc.).

In a further aspect, peptides and proteins of the invention, be characterized by exhibiting an at least about 15%, such as an at least about 20%, at least about 30%, at least about 50%, at least about 75% or more (e.g., about 80%) reduction in IGF-1R kinase activity.

In an additional aspect, peptides and proteins of the invention also or alternatively can be characterized as exhibiting a significant reduction in IGF-1R-associated MAPK44 signaling.

In an additional aspect, peptides and proteins of the invention also or alternatively can be characterized as exhibiting a significant reduction in IGF-1R-associated MAPK42 signaling.

In an additional aspect, peptides and proteins of the invention also or alternatively can be characterized as exhibiting a significant reduction in IGF-1R-associated IRS- I signaling.

In another aspect, peptides and proteins of the invention also or alternatively can be characterized based on the ability to completely inhibit IGF-1R kinase activation by IGF-1.

In still a further facet, peptides and proteins of the invention also or alternative can be characterized on the basis of specifically inhibiting IGF-1R tyrosine phosphorylation (by exhibiting relatively low levels of insulin receptor tyrosine phosphorylation).

Peptides and proteins of the invention can be further characterized according to any of the particular formulas or sequences described herein, a description of specific examples of which immediately follows below.

### FORMULA 1 PEPTIDES AND PROTEINS

In a first aspect, the disclosure provides novel proteins and peptides that bind to, and typically act as antagonists at, human IGF-1R, comprising (or consisting or consisting essentially of) an amino acid sequence according to formula 1.

Formula 1 is defined as follows:
X₁SFYSCLESLYX₂X₃PAEKSRGQWJ₄X₅CRX₆X₇ (SEQ ID NO:I), wherein X₁ represents an optionally present E residue; X₂ represents any suitable residue (but typically is selected from an N, A, Q, or T residue); X₃ represents any suitable amino acid residue (but typically represents a small residue, a very small residue, or even more particularly a G or A residue (or D-Ala ("a") residue); X₄ represents a D or E residue; X₅ represents any suitable residue (but typically represents a small residue, a very small residue, or even more particularly a G or A residue); X₆ represents an optionally present K or E residue; and X₇ represents an optionally present S or K residue, wherein the peptide or sequence is not F249
(SFYSCLESLVNGPAEKSRGQWDGCR - SEQ ID NO:2), F250
(SFYSCLESLVNGPAEKSRGQWDGCRK - SEQ ID NO:3), or RP33-IGF
(SFYSCLESLVNGPAEKSRGQWDGCRKK - SEQ ID NO:4).

Formula 1 sequences are highly similar to F249, F250, and RP33-IGF (each disclosed in the '246 PCT application), which have previously been demonstrated to bind to IGF-1R and exhibit IGF-1R antagonism. In view of these facts, combined with the finding that a number of other formula 1 sequences exhibit IGF-1R binding, and in some cases antagonism, it can be predicted that a number of such sequences can be identified by routine experimentation.

In one exemplary aspect, the peptide or sequence is or comprises F215 (i.e., ESFYSCLESLVNGPAEKSRGQWDGCRE - SEQ ID NO: 5). F215 peptide has been determined to have a binding affinity to IGF-1R of 0.8-3x 10⁻⁷ M. In another aspect, the disclosure provides an analog of F215 (or a protein comprising the same) having at least about 88%, at least about 92%, or at least about 96% identity to SEQ ID NO:5, but is not one of the peptides specifically described herein or in the '246 PCT, '771 PCT, or '147 US patent applications.

In one exemplary aspect, the peptide or sequence is or comprises RP30 (i.e., SFYSCLESLVNGGAERSDGQWEGCR - SEQ ID NO: 198). In another aspect, the disclosure provides an analog of RP30 (or a protein comprising the same) having at least about 88%, at least about 92%, or at least about 96% identity to SEQ ID NO:198, but is not one of the peptides specifically described herein or in the '246 PCT, '771 PCT, or '147 US patent applications.

In another illustrative facet, the peptide or sequence is or comprises F258 (i.e., SFYSCLESLVAGPAEKSRGQWEGCR - SEQ ID NO:6). In another aspect, the disclosure provides an analog of F258 (or a protein comprising such an analog) that has at least about 88%, 92%, or 96% identity to SEQ ID NO:6, but is not one of the peptides that is specifically described herein or in the '246 PCT, '771 PCT, or '147 US patent applications. F258 peptide has been shown to have a similar affinity to F249 (about 2x10⁻⁷M), but greater stability.

Another facet of the disclosure is embodied in a peptide or protein that consists, consists essentially of, or comprises F259, SFYSCLESLVAGPAEKSRGQWEGCRK (SEQ ID NO:7). F259 peptide has been shown to have an affinity to IGF-1R 8x10⁻⁸ M, antagonistic effect on IGF-1 dependent growth in SW480 and MCF-7 cells with IC₅₀ 2x10⁻⁶ M, and greater stability than F250. In another aspect, the disclosure provides an analog of F259 (or a protein comprising such an analog) that has at least about 88%, 92%, or 96% identity to SEQ ID NO:7, but is not one of the peptides otherwise specifically described herein or in the '246 PCT, '771 PCT, or '147 US patent applications.

In yet another, the disclosure provides a protein or a peptide comprising, consisting, or consisting essentially of F292, SFYSCLESLVTGPAEKSRGQWEGCRK (SEQ ID NO:8), with binding affinity to IGF-1R of 1x10⁻⁷ M. In another aspect, the disclosure provides an analog of F292 (or a protein comprising such an analog) that has at least about 88%, 92%, or 96% identity to SEQ ID NO:8, but is not one of the peptides otherwise specifically described herein or in the '246 PCT, '771 PCT, or '147 US patent applications.

A further exemplary aspect of the invention is embodied in proteins and peptides that comprise, consist, or consist essentially of F293 (SFYSCLESLVQGPAEKSRGQWEGCRK- SEQ ID NO:9). F293 peptide has been determined to have a binding affinity to IGF-1R of 9x10⁻⁸ M, antagonistic effect on IGF-1 dependent growth in SW480 with IC₅₀ 1-20x10⁻⁷ M and in MCF-7 cells with IC₅₀ 1x10⁻⁵ M. In SW480 an inhibition of basal growth was observed with F293 (one experiment only). IGF-1 binding to IGF-1R on L6 (with mainly symmetric IGF-1R) and in L6 hIR (with mainly hybrid IGF-1R/IR) could be displaced by 1.5x10⁻⁷ M, and phosphorylation of IGF-1R and MAPK42/44 in response to IGF-1 treatment was inhibited with IC₅₀ 5x10⁻⁷ M with F293. Inhibition of phosphorylation by F293 was more effective on IGF-1 induced phosphorylation as compared to insulin induced phosphorylation in L6-hIR. Glucose uptake in SGBS cells in response to insulin/IGF-1 is not affected by doses <5x 10⁻⁶M of F293. In another aspect, the disclosure provides an analog of F293 (or a protein comprising such an analog) that has at least about 88%, 92%, or 96% identity to SEQ ID NO:9, but is not one of the peptides otherwise specifically described herein or in the above-cited '246 PCT, '771 PCT, or '147 US patent applications.

Additionally, the disclosure provides proteins and peptides that comprise, consist, or consist essentially of F296 (SFYSCLESLVNAPAEKSRGQWEGCRK - SEQ ID NO:10). F296 peptide has been determined to have a binding affinity to IGF-1R of 6x10⁻⁸ M. In another aspect, the disclosure provides an analog of F296 (or a protein comprising such an analog) that has at least about 88%, 92%, or 96% identity to SEQ ID NO: 10, but is not one of the peptides specifically described herein or in the '246 PCT, '771 PCT, or '147 US patent applications.

The disclosure also provides proteins and peptides that comprise, consist, or consist essentially of F297 (SFYSCLESLVNaPAEKSRGQWEGCRK - SEQ ID NO:11). F297 peptide has been demonstrated to have a binding affinity to IGF-1R of 5x10⁻⁸ M with a being D-Ala. In another aspect, the disclosure provides an analog of F297 (or a protein comprising such an analog) that has at least about 88%, 92%, or 96% identity to SEQ ID NO:11, but is not one of the peptides specifically described herein or in the '246 PCT, '771 PCT, or '147 US patent applications.

In still another aspect, the disclosure provides proteins and peptides that comprise, consist, or consist essentially of F294 (SFYSCLESLVNAPAEKSRGQWDGCRK - SEQ ID NO:196). In another aspect, the disclosure provides analogs of F294 having at least 92% or at least 96% identity to SEQ ID NO:196, with the proviso that the analog is not one of the peptides explicitly disclosed herein or in the '246 PCT, '771 PCT, or '147 US patent applications.

### FORMULA 2 PEPTIDES AND PROTEINS

In another aspect, the disclosure provides novel proteins and peptides that bind to, and typically act as antagonists at, human IGF-1R, comprising (or consisting or consisting essentially of) an amino acid sequence according to formula 2.

Formula 2 is defined as follows:
X₈X₉FYGCLLDLSLGVPSX₁₀GWX₁₁X₁₂X₁₃CITX₁₄X₁₅ (SEQ ID NO:12), wherein X₈ represents an optionally present Arg (R) residue; X₉ represents any suitable residue (typically a polar residue and commonly a D, N, or Q residue); X₁₀ represents any suitable residue (typically an F or L residue); X₁₁ represents any suitable amino acid residue (typically a polar residue, and more typically a basic residue, or more particularly still an R, K, or D-Arg ("r") residue); X₁₂ represents any suitable amino acid residue (typically a polar residue, and more typically a basic residue, or more particularly still an R, K, or D-Arg ("r")); X₁₃ represents any suitable amino acid residue (typically a polar residue, and more typically a basic residue, or more particularly still an R, K, or D-Arg ("r")); X₁₄ represents an optionally present A residue; and X₁₅ represents an optionally present R residue, wherein the amino acid sequence is not and does not comprise F138 (QFYGCLLDLSLGVPSFGWRRRCITA - SEQ ID NO: 13).

Formula 2 sequences are highly similar to F138, which is described in the '246 PCT application. F138 has been demonstrated to have a binding affinity to IGF-1R of 6-10x10⁻⁹ M. Moreover, F138 has been demonstrated to have an antagonistic effect on IGF-1 dependent growth in SW480, with an IC₅₀ of 1.5x10⁻⁷ M; in the range of about 1-5x10⁻⁷ M in MCF-7 the IC₅₀ was 5x10⁻⁷ M. F138 also inhibits basal growth of SW480 at doses > 1x10⁻⁷ M. In SW480, the inhibitory effect of IGF-2 dependent growth was similar to the effect observed with IGF-1 dependent growth in association with F138. IGF-1 binding to IGF-1R on L6 could be displaced by 1x10⁻⁷ M of F138. In L6 cells (with mainly symmetric IGF-1R) and in L6 hIR (with mainly hybrid IGF-1R/IR) IGF-1 induced phosphorylation of IGF-1R, IRS-1, MAPK42/44 and PKB/Akt was inhibited by peptide F138 with IC₅₀ 1-6x10⁻⁷ M. Some selectivity for inhibiting IGF-1 induced tyrosine phosphorylation as compared to insulin induced phosphorylation is seen in L6-hIR in association with F138. Moreover. glucose uptake in SGBS cells in response to insulin/IGF-1 is not affected by doses <5x 10⁻⁶ M peptide F138. Based on these properties, as well as that a number of other formula 2 peptides have been identified that bind IGF-1R, and in some cases exhibit IGF-1R antagonism, additional specific formula 2 sequences that exhibit binding to IGF-1R and also likely exhibit IGF-1R antagonism can be expected to be identified with routine experimentation.

In a particular exemplary aspect, the invention provides a formula 2 protein or peptide that comprises, consists, or consists essentially of sequence F391 (QFYGCLLDLSLGVPSFGWmCITA - SEQ ID NO: 208). Peptide F391 has been * demonstrated to have a binding affinity to IGF-1R of 8x10⁻⁷M. In another aspect, the disclosure provides an analog of F391 (or a protein comprising such an analog) that has at least about 88%, 92%, or 96% identity to F391, but is not one of the peptides specifically described herein or in the '246 PCT, '771 PCT; or '147 US patent applications.

Another representative set of formula 2 proteins and peptides is embodied in proteins and peptides that comprise, consist, or consist essentially of sequence F392 (QFYGCLLDLSLGVFISFGWKKKCITA - SEQ ID NO: 14). Peptide F392 has been demonstrated to have a binding affinity to IGF-1R of 2x10⁻⁸ M. Moreover, peptide F392 was demonstrated to exhibit an antagonistic effect on IGF-1 dependent growth of SW480 with IC₅₀ of 8x10⁻⁷ M. In another aspect, the disclosure provides an analog of F392 (or a protein comprising such an analog) that has at least about 88%, 92%, or 96% identity to SEQ ID NO:14, but is not one of the peptides specifically described herein or in the '246 PCT, '771 PCT, or '147 US patent applications.

In a further illustrative aspect, the disclosure provides a protein or peptide that comprises, consists, or consists essentially of sequence F407 (RQFYGCLLDLSLGVPSFGWRRRCITAR - SEQ ID NO:15). Peptide F407 has been demonstrated to bind IGF-1R with binding of 7x10⁻⁸ M. In another aspect the disclosure provides an analog of F407 (or a protein comprising such an analog) that has at least about 88%, 92%, or 96% identity to SEQ ID NO:15, but is not one of the peptides specifically described herein or in the '246 PCT, '771 PCT, or '147 US patent applications.

In still another particular aspect, the disclosure provides a protein or peptide that comprises, consists or consists essentially of sequence F408 (NFYGCLLDLSLGVPSFOWRRRCITA - SEQ ID NO:1 16). Peptide F408 has been disclosure provides an analog of F408 (or a protein comprising such an analog) that has at least about 88%, 92%, or 96% identity to SEQ ID NO:16, but is not one of the peptides specifically described herein or in the '246 PCT, '771 PCT, or '147 US patent applications.

Another exemplary set of formula 2 peptides and proteins comprise sequence F428 (DFYGCLLDLSLGVPSLGWRRRCIT- SEQ ID NO:17). Peptide 428 has been determined to have a binding affinity to IGF-1R of 1x10⁻⁹ M. Moreover, peptide 428 has been determined experimentally to have an antagonistic effect on IGF-1 dependent growth of SW480 with IC₅₀ 5-10x10⁻⁷ M. In another aspect, the disclosure provides an analog of F428 (or a protein comprising such an analog) that has at least about 88%, 92%, or 96% identity to SEQ ID NO:17, but is not one of the peptides specifically described herein or in the '246 PCT, `771 PCT, or '147 US patent applications.

In yet a further aspect, the invention provides peptides and proteins that consist of, or comprise sequence F429 (DFYGCLLDLSLGVPSLGWRRRCITA - SEQ ID NO: 18). Peptide F429 has been determined to have a binding affinity to IGF-1R of 6x10⁻¹⁰ M. Furthermore, peptide F429 has been shown to have an antagonistic effect on IGF-1 dependent growth of SW480 with IC₅₀ 3-10x10⁻⁷ M. In another aspect, the invention provides an analog of F429 (or a protein comprising such an analog) that has at least about 98%% identity to SEQ ID NO: 18, but is not one of the peptides specifically described herein or in the '246 PCT, `771 PCT, or 147 US patent applications.

In another aspect, the disclosure provides a derivative of F138 (F138P), which comprises an N-terminal pyroglutamate residue. F138P has been demonstrated to have a binding affinity to IGF-1R that is similar to F138. F138P has been shown to exhibit an antagonistic effect on IGF-1 dependent growth in SW480 with an IC₅₀ of 5x10⁻⁷ M. F138P also inhibits basal growth of SW480 at doses >1x10⁻⁷ M.

### FORMULA 3 PROTEINS AND PEPTIDES

In another aspect, the disclosure further provides proteins and peptides that bind to, and typically act as antagonists at, human IGF-1R, comprising (or consisting or consisting essentially of) an amino acid sequence according to formula 3.

Formula 3 is defined as follows:
X₁₆X₁₇FYSCLASLX₁₈X₁₉GX₂₀X₂₁X₂₂X₂₃X₂₄X₂₅GX₂₆WERCRX₂₇X₂₈ (SEQ ID NO:19),
wherein X₁₆ represents an optionally present E residue; X₁₇ represents a T or an S residue; X₁₈ represents any suitable residue (but typically is a hydrophobic residue, an aliphatic uncharged residue, an aliphatic residue, or more particularly still an L or V residue); X₁₉ represents any suitable residue (but typically is a small residue and/or a hydrophobic residue, such as a T or an A residue); X₂₀ represents any suitable residue (but typically is a small residue, such as a T or a P residue); X₂₁ represents any suitable residue (but typically is a flexible residue, such as a P or an R residue); X₂₂ represents any suitable residue (e.g., a Q, E, or W residue); X₂₃ represents any suitable residue (but typically is a flexible residue, e.g., a P or Q residue); X₂₄ represents any suitable residue (but typically is a polar residue, e.g., an N or K residue); X₂₅ represents any suitable residue (but typically is a hydrophobic and/or flexible residue, such as, e.g., an R or G residue); X₂₆ represents any suitable residue (but typically is a small residue, e.g., an S, A, or P residue); X₂₇ represents an optionally present flexible and/or polar residue (e.g., an E, R, or K residue); and X₂₈ represents an optionally present flexible and/or polar residue (e.g., a K or E residue), wherein the sequence is not (and does not comprise) RP6 (TFYSCLASLLTGTPQPNRGPWERCR - SEQ ID NO:20) (disclosed in the '246 PCT application).

In one exemplary aspect, the formula 3 peptide or protein comprises, consists of, or consists essentially of sequence F230 (ESFYSCLASLVAGTPWPKGGSWERCREE - SEQ ID NO:21). Peptide F230 was experimentally determined to have a binding affinity to IGF-1R of 2-5x10⁻⁸ M. In another aspect, the disclosure provides an analog of F230 (or a protein comprising such an analog) that has at least about 80%, 84%, 87%, 90%, 93%, or 96% identity to SEQ ID NO:21, but is not one of the peptides specifically described herein or in the '246 PCT, '771 PCT, or '147 US patent applications.

In another exemplar facet, the disclosure provides formula 3 peptides and proteins that comprise, consist of, or consist essentially of sequence F142 (TFYSCLASLLTGPREQNRGAWERCRR - SEQ ID NO:22). Peptide F142 has been determined to have a binding affinity to IGF-1R of 4x10⁻⁸ M. In another aspect, the disclosure provides an analog of F142 (or a protein comprising such an analog) that has at least about 80%, 85%, 88%, 92%, or 96% identity to SEQ ID NO:22, but is not one of the peptides specifically described herein or in the '246 PCT, '771 PCT, or 147 US patent applications.

### ADDITIONAL IGF1R ANTAGONISTS COMPRISING A FORMULA 4 SEQUENCE

In another aspect, the disclosure further provides proteins and peptides that bind to, and typically act as antagonists at, human IGF-1R, comprising (or consisting or consisting essentially of) an amino acid sequence according to formula 4.

Formula 4 is defined as follows:
X₂₉X₃₀DCX₃₁X₃₂RPCGDAX₃₃X₃₄FYX₃₅WFX₃₆QQX₃₇SX3₈ (SEQ ID NO:23), wherein X₂₉ represents any suitable residue (e.g., a Y residue) but frequently is a Q residue, X₃₀ represents any suitable residue; X₃₁ represents any suitable residue (e.g., W), but typically is an R residue; X₃₂ represents any suitable residue (but typically is a small residue, e.g., an A, D, or G residue); X₃₃ represents any suitable residue (e.g., an A, P, E, or D residue); X₃₄ represents any suitable residue, but typically is an N or an S (or other small and/or polar residue); X₃₅ represents any suitable residue, but typically is a D or E; X₃₆ represents any suitable residue, but typically is a D or a V residue (or other small residue); X₃₇ represents any suitable residue (but typically is a hydrophobic residue, e.g., an A or an R residue); and X₃₈ represents any optionally present E or D residue (typically an E residue), wherein the sequence is not and does not comprise C1 or D112 (CWARPCGDAANFYDWFVQQAS - SEQ ID NO:24)
(disclosed in the `246 PCT application under both names).

In one illustrative particular aspect, the disclosure provides a formula 4 protein or peptide comprising, consisting of, or consisting essentially of sequence F263 (VQDDCRGRPCGDADSFYEWFDQQAS - SEQ ID NO:25). F263 peptide has been determined to have a binding affinity to IGF-IR of 3x10⁻⁸ M. In another aspect, the disclosure provides an analog of F263 (or a protein comprising such an analog) that has at least about 88%, 92%, or 96% identity to SEQ ID NO:25, but is not one of the peptides specifically described herein or in the '246 PCT, '771 PCT, or '147 US patent applications.

In another illustrative aspect, the disclosure provides formula 4 peptides and proteins comprising, consisting of, or consisting essentially of sequence F264 (RQWDCRGRPCGDAESFYEWFDQQRS - SEQ ID NO:26). Peptide F264 has been determined to have a binding affinity to IGF-1R of 4x10⁻⁸ M. Moreover, peptide F264 has been demonstrated to have an antagonistic effect on IGF-1 dependent growth in SW480 cells with IC₅₀ >1x10⁻⁶ M. In another aspect, the disclosure provides IGF-1R-binding peptides (and in at least some cases IGF-1R antagonists) that exhibit at least about 80%, such as at least about 88%, such as at least 92%, such as at least about 96% identity (but less than 100% identity) to SEQ ID NO:26, but are not described specifically herein or in the '246 PCT, `771 PCT, or '147 US applications.

Another exemplary embodiment is provided in peptides and proteins that comprise, consist of, or consist essentially of sequence F270 (ESYGDCRDRPCGDAPNFYDWFVQQASE - SEQ ID NO:27). Peptide F270 has been determined to have a binding affinity to IGF-1R of 7x 10⁻⁸ M. Moreover, peptide F270 has been determined to exhibit an antagonistic effect on IGF-1 dependent growth in SW480 with IC₅₀ 20x10⁻⁷ M. However, agonistic effects on MCF-7 were seen (in one experiment only). In another aspect, the disclosure provides an analog of F270 (or a protein comprising such an analog) that has at least about 80%, 85%, 89%, 92%, or 96% identity to SEQ ID NO:27, but is not one of the peptides specifically described herein or in the '246 PCT, `771 PCT, or '147 US patent applications.

In yet another aspect, the disclosure provides peptides and proteins comprising, consisting of, or consisting essentially of sequence F265 (VQRDCRGRPCGDAASFYDWFDQQRS - SEQ ID NO:197). In a further aspect, the disclosure provides an analog of F265 (or a protein comprising such an analog) that has at least about 80%, 88%, 92%, or 96% identity to SEQ ID NO:197, but is not one of the peptides specifically described herein or in the '246 PCT, `771 PCT, or '147 US patent applications.

### F364 AND ANALOGS THEREOF

The disclosure also provides proteins that comprise sequence F364 (FVQDDCRGRPCGDADSFYEWFDQQAGYGSSSRRAPQT- SEQ ID NO:28) and the disclosure provides peptides that consist of F364 or that consist essentially of F364. F364 peptide has been determined to bind IGF-1R with binding affinity of 5x10⁻⁸ M. In another aspect, the disclosure provides IGF-1R-binding peptides that exhibit at least about 80%, for example at least about 85%, such as at least about 90%, such as at least about 95%, such as at least about 97% identity to SEQ ID NO:28, and are not specifically described herein or in the '246 PCT, `771 PCT, or '147 US applications.

### ADDITIONAL IGF-1R-BINDING PEPTIDES/PROTEINS

The disclosure provides additional IGF-1R-binding peptides described in the tables provided in the Exemplary Experimental Methods and Data section of this document (e.g., peptides defined by and/or proteins comprising at least one of SEQ ID NOs:29-54 and 56-195 set forth in Table 4). Analogs of such peptides, having high levels of identity thereto, e.g., at least about 82% (e.g., about 85% or more), at least about 86% (e.g., about 90% or more), or at least about 92% or at least about 96% (e.g., about 95% or more) identity thereto, having similar IGF-1R-binding and/or antagonism properties, also are provided as a further facet of the disclosure (with the proviso that such analogs are not explicitly disclosed herein or in the '246 PCT, `771 PCT, or '147 US applications. As reflected by peptides disclosed in Table 4, multimers of any of the sequences provided herein also can exhibit useful biological properties. Such peptides and proteins are another feature of the disclosure. An additional feature of the disclosure is fusion proteins comprising one or more of the sequences disclosed herein linked to another functional moiety (e.g., an anti-cancer (e.g., a toxic protein) or anti-angiogenic agent (e.g., pigment epithelium-derived factor (PEDF)) or a sequence that promotes detection (e.g., a green fluorescent protein or an epitope tag sequence)), which may be conjugated to one or more sequences of the invention directly or by a linker, which may be, e.g., a "flexible" amino acid sequence linker (e.g., GSGS (SEQ ID NO:55) or a chemical moiety, such as a Lig or Pox moiety - described below).

### NUCLEIC ACIDS, VECTORS, CELLS, AND METHODS OF PRODUCING PEPTIDES/PROTEINS

In another aspect, the invention provides an isolated nucleic acid comprising a sequence encoding at least one of any of the above-described peptides or proteins of the invention. The nucleic acid may be of any suitable composition. For example, the nucleic acid may be a DNA molecule (single stranded or double stranded), an RNA molecule (single stranded or double stranded), a hybrid DNA/RNA molecule, or other nucleic acid molecule comprising an expressible nucleic acid sequence coding for one of the above-described peptides. The nucleic acid may include other modifications and/or features, such as, for example, a phosphothioate backbone.

In an additional facet, the invention provides vectors that comprise a nucleic acid according to the immediately foregoing aspect. A "vector" refers to a delivery vehicle that (a) promotes the expression of a peptide/protein-encoding nucleic acid sequence, (b) promotes the production of the peptide/protein therefrom, (c) promotes the transfection/transformation of target cells therewith, (d) promotes the replication of the nucleic acid sequence, (e) promotes stability of the nucleic acid, (f) promotes detection of the nucleic acid and/or transformed/transfected cells, and/or (g) otherwise imparts advantageous biological and/or physiochemical function to the peptide/protein-encoding nucleic acid. A vector in the context of this invention can be any suitable vector, including chromosomal, non-chromosomal, and synthetic nucleic acid vectors (a nucleic acid sequence comprising a suitable set of expression control elements). Examples of such vectors include derivatives of SV40, bacterial plasmids, phage DNA, baculovirus, yeast plasmids, vectors derived from combinations of plasmids and phage DNA, and viral nucleic acid (RNA or DNA) vectors. In one exemplary aspect, a nucleic acid is comprised in a naked DNA or RNA vector, including, for example, a linear expression element (as described in, e.g., Sykes and Johnston (1997) Nat Biotech 17: 355-59), a compacted nucleic acid vector (as described in, e.g., US Patent 6,077, 835 and/or International Patent Application WO 00/70087), a plasmid vector such as pBR322, pUC 19/18, or pUC 118/119, a "midge" minimally-sized nucleic acid vector (as described in, e.g., Schakowski et al. (2001) Mol Ther 3: 793-800), or as a precipitated nucleic acid vector construct, such as a CaPO₄-precipitated construct (as described in, e.g., International Patent Application WO 00/46147, Benvenisty and Reshef (1986) Proc Natl Acad Sci USA 83: 9551-55, Wigler et al. (1978), Cell 14:725, and Coraro and Pearson (1981) Somatic Cell Genetics 7:603). Such nucleic acid vectors and the usage thereof are well known in the art (see, e.g., US Patents 5,589,466 and 5,973,972).

The vector can be selected based on its ability to be expressed in any suitable cell type (e.g., a mammalian cell, a yeast cell, etc.). In one aspect, the vector is suitable for expression of the peptide/protein in a bacterial cell. Examples of such vectors include, for example, vectors which direct high level expression of fusion proteins that are readily purified (e.g., multifunctional E. coli cloning and expression vectors such as BLUESCRIPT (Stratagene), pIN vectors (Van Heeke & Schuster, J Biol Chem 264: 5503-5509 (1989); pET vectors (Novagen, Madison WI) ; and the like). An expression vector also or alternatively can be, for example, a vector suitable for expression in a yeast system. Any vector suitable for expression in a yeast system can be employed. Suitable vectors for use in, e.g., Saccharomyces cerevisiae include, for example, vectors comprising constitutive or inducible promoters such as alpha factor, alcohol oxidase and PGH (reviewed in, e.g., Ausubel, *supra,* and Grant et al., Methods in Enzymol 153: 516-544 (1987)).

A vector can comprise or be associated with any suitable promoter, enhancer, and other expression-facilitating elements. Examples of such elements include strong expression promoters (e.g., a human CMV IE promoter/enhancer, an RSV promoter, SV40 promoter, SL3-3 promoter, MMTV promoter, or HIV LTR promoter), effective poly (A) termination sequences, an origin of replication for plasmid product in E. coli, an antibiotic resistance gene as a selectable marker, and/or a convenient cloning site (e.g., a polylinker). Vectors also can comprise an inducible promoter as opposed to a constitutive promoter such as CMV IE (the skilled artisan will recognize that such terms are actually descriptors of a relative degree of gene expression under certain conditions). In one aspect, the invention provides a nucleic acid comprising a sequence encoding a peptide/protein which is operatively linked to a tissue specific promoter which promotes expression of the sequence in target tissue, such as cancer-associated tissues. Examples of such cancer-related tissue specific promoter systems are described in, e.g., Fukazawa et al., Cancer Res. 2004 Jan 1;64(1):363-9; Latham et al., Cancer Res. 2000 Jan 15;60(2):334-41; and Shirakawa et al., Mol Urol. 2000 Summer;4(2):73-82.

In another aspect, the nucleic acid is positioned in and/or delivered to the host cell or host animal via a viral vector. Any suitable viral vector can be used in this respect, and several are known in the art. A viral vector can comprise any number of viral polynucleotides, alone or in combination with one or more viral proteins, which facilitate delivery, replication, and/or expression of the nucleic acid of the invention in a desired host cell. The viral vector can be a polynucleotide comprising all or part of a viral genome, a viral protein/nucleic acid conjugate, a virus-like particle (VLP), or an intact virus particle comprising viral nucleic acids and the nucleic acid of the invention. A viral particle viral vector can comprise a wild-type viral particle or a modified viral particle. The viral vector can be a vector which requires the presence of another vector or wild-type virus for replication and/or expression (i.e., a viral vector can be a helper-dependent virus), such as an adenoviral vector amplicon. Typically, such viral vectors consist essentially of a wild-type viral particle, or a viral particle modified in its protein and/or nucleic acid content to increase transgene capacity or aid in transfection and/or expression of the nucleic acid (examples of such vectors include the herpes virus/AAV amplicons). Typically, a viral vector is similar to and/or derived from a virus that normally infects humans. Suitable viral vector particles in this respect, include, for example, adenoviral vector particles (including any virus of or derived from a virus of the adenoviridae), adeno-associated viral vector particles (AAV vector particles) or other parvoviruses and parvoviral vector particles, papillomaviral vector particles, flaviviral vectors, alphaviral vectors, herpes viral vectors, pox virus vectors, retroviral vectors, including lentiviral vectors. A viral vector, or other vector, often can be characterized as being replication-deficient. Examples of such viruses and viral vectors are well known in the art.

Other features of the disclosure include recombinant cells, such as yeast, bacterial, and mammalian cells (e.g., immortalized mammalian cells) comprising such a nucleic acid, vector, or combinations of either or both thereof. For example, in one exemplary aspect the invention provides a cell comprising a non-integrated nucleic acid, such as a plasmid, cosmid, phagemid, or linear expression element, which comprises a sequence coding for expression of a peptide/protein according to one of the various aspects of the invention.

In yet another aspect, the disclosure provides a method of producing a peptide or protein according to any of the above-described aspects of the invention that includes transforming/transfecting a cell with a nucleic acid coding for expression of the peptide/protein or a vector comprising the same, typically culturing the cell under conditions suitable for expression of the nucleic acid, and collecting the expression product therefrom (e.g., from a cell lysate or cell media in the case of a secreted product), typically in association with and/or followed by one or more purification methods to obtain an isolated protein/peptide (e.g., centrifugation, chromatography purification, and/or filtering). Methods for transfection/transformation, culturing, and purifying proteins and peptides are known in the art and, accordingly, need not be described here.

Peptides provided by the invention also can be produced by chemical "synthesis" techniques. A number of methods of chemical synthesis are known and available and any suitable type of such method can be used for this purpose. Examples of such techniques include exclusive solid phase synthesis, partial solid phase methods, fragment condensation, classical solution synthesis. In addition, recombinant and synthetic methods of peptide production can be combined to produce semi-synthetic peptides. Thus, for example, the chains can be prepared by solid phase peptide synthesis as described by Merrifield, 1963, J. Am. Chem. Soc. 85:2149; 1997. In one embodiment, synthesis is carried out with amino acids that are protected at the alpha-amino terminus. Trifunctional amino acids with labile side-chains can also be protected with suitable groups to prevent undesired chemical reactions from occurring during the assembly of the peptides. The alpha-amino protecting group can be selectively removed to allow subsequent reaction to take place at the amino-terminus. The conditions for the removal of the alpha-amino protecting group do not remove the side-chain protecting groups. Other principles relevant to such methods are described in, e.g., Merrifield R B. Angew Chem Int Ed Engl. 1985;97:799-810, Methods Enzymol. 1997;289:3-13; Hackeng et al., Proc. Natl. Acad. Sci. USA, Vol. 96, pp. 10068-10073, August 1999; Goeddel et al., Proc. Natl. Acad. Sci. USA, 76, 106-110, 1979; Hunkapillar et al., 1984, Nature (London) , 310: 105-111; and Becker et al., Proc Natl Acad Sci USA. 2003 April 29; 100(9): 5075-5080.

### PHARMACEUTICAL COMPOSITIONS

Compounds (proteins, peptides) of the invention and/or any secondary agents (e.g., one or more additional anti-cancer and/or anti-angiogenic compounds/compositions) can be formulated with any number of suitable carriers, diluents, excipients, and the like (See e.g., Powell et al. "Compendium of excipients for parenteral formulations" PDA J Pharm Sci Technol. 52:238-311 (1998) - the terms "vehicle" and "carrier" can be used to refer to collectively all and, by reference, independently each such type of agent throughout the description of the invention and/or functionality enhancers (e.g., stabilizers, surfactants, wetting agents, emulsifying agents, preservatives, fillers, salt(s), solubilizers, detergents, anti-aggregating agents (e.g., anti-aggregating amino acid formulations) dispersion media, isotonic agents, tissue fixatives, chelating agents, buffers, antibacterial agents, antioxidants, colorants, flavoring agents, absorption delaying agents, controlled release agents, etc.) appropriate for the indicated route of administration (and contemplated storage, etc.). Such additional ingredients, of course, should not adversely affect the overall stability of the pharmaceutical formulation of the present invention. Suitable carriers, diluents, adjuvants, as well as functionality enhancers and modes of administration/formulation of such compositions are well known in the pharmaceutical arts. See, e.g., Remington: The Science and Practice of Pharmacy, 19th edition, 1995. See also, e.g., Berge et al., J. Pharm. Sci., 6661), 1-19 (1977); Wang and Hanson, J. Parenteral. Sci. Tech: 42, S4-S6 (1988), US Patents 6,165,779 and 6,225,289. Additional relevant principles, methods, and agents are described in, e.g., Urquhart et al., Lancet, 16, 367 (1980), Lieberman et al., PHARMACEUTICAL DOSAGE FORMS-DISPERSE SYSTEMS (2nd ed., vol. 3,1998); Ansel et al., PHARMACEUTICAL DOSAGE FORMS & DRUG DELIVERY SYSTEMS (7th ed. 2000); Martindale, THE EXTRA PHARMACOPEIA (31st edition), Remington's PHARMACEUTICAL SCIENCES (16th-20th editions); The Pharmacological Basis Of Therapeutics, Goodman and Gilman, Eds. (9th ed.-1996); Wilson and Gisvolds' TEXTBOOK OF ORGANIC MEDICINAL AND PHARMACEUTICAL CHEMISTRY, Delgado and Remers, Eds. (10th ed. -1998), and U.S. Patents 5,708,025 and 5,994,106. Principles of formulating pharmaceutically acceptable compositions also are described in, e.g., Platt, Clin. Lab Med., 7:289-99 (1987), Aulton, PHARMACEUTICS: THE SCIENCE OF DOSAGE FORM DESIGN, Churchill Livingstone (New York) (1988), EXTEMPORANEOUS ORAL LIQUID DOSAGE PREPARATIONS, CSHP (1998), and "Drug Dosage," J. Kans. Med. Soc., 70 (I), 30-32 (1969).

Compositions of the invention can be formulated in any suitable form, such as liquid, semi-solid and solid dosage forms, such as liquid solutions (e.g., injectable and infusible solutions), dispersions or suspensions, and the like. The optimal form for any composition depends on the intended mode of administration, the nature of the composition or combination, and therapeutic application or other intended use. A typical mode for delivery for a composition of the invention is by parenteral administration (e.g., intravenous administration). In one aspect, a composition of the invention is administered to a human patient by intravenous infusion or injection.

Pharmaceutically acceptable compositions typically are sterile, dissolve sufficient amounts of the compound of the invention (and any present secondary agents), are stable under conditions for manufacture and storage, and not harmful to the subject for the proposed application (or at least not harmful to a number, usually a substantial majority (at least about 70%, 80%, 85%, 90%, 95%, 97%, 98%, 99%, etc.), of similar subjects as may be determined by, e.g., clinical trials). A composition so provided by the invention (and/or used in the various methods described elsewhere herein) can be subjected to conventional pharmaceutical operations such as sterilization, purification, etc. (such that the active ingredients thereof can be considered at least substantially isolated or isolated).

In one embodiment of the invention the pharmaceutical formulation is an aqueous formulation, i.e. formulation comprising water. Such formulation is typically a solution or a suspension. In a further embodiment of the invention the pharmaceutical formulation is an aqueous solution. The term "aqueous formulation" is defined as a formulation comprising at least 50% w/w water. Likewise, the term "aqueous solution" is defined as a solution comprising at least 50% w/w water, and the term "aqueous suspension" is defined as a suspension comprising at least 50% w/w water.

In another embodiment the pharmaceutical formulation is a freeze-dried formulation, whereto the physician or the patient adds solvents and/or diluents prior to use.

An injectable pharmaceutical product typically is considered "acceptable for therapeutic application" if it is sterile, substantially pyrogen-free, and has no medically unacceptable effects. For example, the product should not produce a medically unacceptable immunological reaction when injected into a human subject. Medically unacceptable effects can be determined by the skilled person in the field of medicine. By purifying the components of the compositions, formulating such components according to the principles described herein and known in the art, and using standard testing procedures, compositions meeting these and/or the other characteristics described herein can be obtained without undue experimentation or effort.

### APPLICATIONS INCLUDING THERAPEUTIC USES/METHODS

The compounds and compositions provided by this invention are useful in a variety of different applications.

In one exemplary aspect, certain compounds and compositions of the invention have anti-cancer properties. As such, at least some compounds and compositions of the invention can be used in the treatment of cancer (an aspect of the invention further described below). The term "treating" in its various grammatical forms in relation to the present invention refers to preventing, curing, reversing, attenuating, alleviating, minimizing, suppressing or halting the deleterious effects of a disease (cancer) state, disease (cancer) progression, disease (cancer) causative agent or other abnormal cancerous, precancerous, or neoplastic condition.

In another illustrative aspect, compounds and compositions of the invention exhibit anti-angiogenic properties. In this respect, such compounds and compositions can be used as substitutes for and/or secondary agents for combination with other known anti-angiogenic agents in similar applications (e.g., in one aspect compounds or compositions of the invention can be administered or otherwise delivered to a patient for treatment of diabetic retinopathy - e.g., by injection into the eye and/or by inclusion in eye drops - or, more generally, for the reduction/prevention of retinal neovascularization).

In a further facet, peptides of the invention can be used as a reference/tool for screening of new chemical entities with antagonistic effects on IGF-1R (examples of such screening methods are described in the '246 PCT, `771 PCT, or '147 US patent applications).

In still another aspect, the peptides of the invention can be used as a reference for CDR/variable region sequence optimization in modified anti-IGF-1R antibodies (by substitution of "native" CDR/variable region residues/sequences/motifs with residues/sequences/motifs of the peptides/sequences disclosed herein).

In another aspect, IGF-1R-binding peptides/sequence of the invention can be used as targeting agents for fusion proteins.

Peptides/proteins of the invention also can be used to purify IGF-1R molecules.

Peptides/proteins of the invention can also be used as diagnostic agents, e.g., for tracking IGF-1R distribution in the body or other media (e.g., in the case of a fusion protein comprising a detectable portion, such as a fluorescent protein portion, and an IGF-1R-binding portion).

In respect of cancer treatment, IGF-1R antagonists provided by the invention can be used in the treatment of a number of different types of cancers, including, but not limited to, breast, prostate, colorectal, and ovarian cancers.

In a particular aspect, an IGF-1R antagonist compound or composition of the invention is used as a treatment of pancreatic cancer or breast cancer. In a particular aspect, the IGF-1R antagonist used in such a method is selected, in part, on the basis of exhibiting an IC₅₀ in the nanomolar range, or picomolar range (e.g., about 600 picomolar) and/or an ED₅₀ (e.g., an ED₅₀ of about 10⁻⁹) with respect to pancreatic cancer cells or breast cancer cells in culture. Typically, the IGF-1R antagonist compound or composition will exhibit inhibition of the growth of pancreatic tumors in nude mice or other suitable animal model (and optimally in human patients).

The compounds of the invention can be delivered in any suitable dose and by any suitable delivery regimen. In one aspect, the compound or composition of the invention delivered to treat cancer is administered subcutaneously, intravenously, or intratumorally by injection or infusion.

An exemplary dosage for an animal model (e.g., nude mice or human patients) is about 3.75 mg/kg, dosing twice a week, for a period of at least about 4 weeks. It is understood that this dosage may be translated to a daily, weekly , bi-weekly, monthly, or yearly regimen. For example, the patient may receive a specific dosage ranging from 20-16 mg/kg or 15-10 mg/kg or 9-5 mg/kg or 4-1 mg/kg or 1 mg/kg-100 µg/kg or from 100 µg/kg-10 µg/kg twice a week or over a period of days, weeks, months, or years such as 1 day, 3 days, 5 days, I week, 2 weeks, 3 weeks, 1 month, 2 months, 3 months, 4 months, 5 months, 6 months, 7 months, 8 months, 9 months, 10 months, 11 months, I year, 2 years, 3 years, 4 years, 5 years and the like.

Typically, the amount of IGF-1R antagonist used in a therapeutic method of the invention is an amount that has been determined to be an effective amount (a therapeutic effective amount and/or prophylactically effective amount).

Compositions of the invention may include a "therapeutically effective amount" or a "prophylactically effective amount" of a compound of the invention (or first and second amounts in the case of a combination composition comprising a compound of the invention and a second agent). A "therapeutically effective amount" refers to an amount effective, when delivered in appropriate dosages and for appropriate periods of time, to achieve a desired therapeutic result in a host (e.g., the inducement, promotion, and/or enhancement of a physiological response associated with reduced angiogenesis or cancer progression). A therapeutically effective amount may vary according to factors such as the disease state, age, sex, and weight of the individual, and the ability of the compound (or compound/secondary agent) to elicit a desired response in the individual. A therapeutically effective amount is also one in which any toxic or detrimental effects of the peptide/molecule of the invention are outweighed by the therapeutically beneficial effects. A "prophylactically effective amount" refers to an amount effective, at dosages and for periods of time necessary, to achieve the desired prophylactic result (e.g., a reduction in the likelihood of developing a disorder, a reduction in the intensity or spread of a disorder, an increase in the likelihood of survival during an imminent disorder, a delay in the onset of a disease condition, a decrease in the spread of an imminent condition as compared to in similar patients not receiving the prophylactic regimen, etc.). Typically, because a prophylactic dose is used in subjects prior to or at an earlier stage of disease, the prophylactically effective amount will be less than the therapeutically effective amount. The term "treatment" herein refers to the delivery of an effective amount of a therapeutically active compound of the invention with the purpose of preventing any symptoms or disease state to develop or with the purpose of easing, ameliorating, or eradicating (curing) such symptoms or disease states already developed. The term "treatment" is thus meant to include prophylactic treatment. However, it will be understood that therapeutic regimens and prophylactic regimens of the invention also can be considered separate and independent aspects of this invention. As such, wherever the term is used herein it is to be understood as also providing support for such separate prophylactic and palliative/curative applications.

Other types of cancers that can be treated by administration or delivery of an effective amount of a compound or composition of the invention. As a non-limiting example, the cancer may be carcinoma, sarcoma, myeloma, leukemia, and lymphoma, and mixed types of cancers, such as adenosquamous carcinoma, mixed mesodermal tumor, carcinosarcoma, and teratocarcinoma. Representative cancers include, but are not limited to, bladder cancer, lung cancer, breast cancer, colon cancer, rectal cancer, endometrial cancer, ovarian cancer, head and neck cancer, prostate cancer, and melanoma. Specifically included are AIDS-related cancers (e.g., Kaposi's Sarcoma, AIDS-related lymphoma), bone cancers (e.g., osteosarcoma, malignant fibrous histiocytoma of bone, Ewing's Sarcoma, and related cancers), and hematologic/blood cancers (e.g., adult acute lymphoblastic leukemia, childhood acute lymphoblastic leukemia, adult acute myeloid leukemia, childhood acute myeloid leukemia, chronic lymphocytic leukemia, chronic myelogenous leukemia, hairy cell leukemia, cutaneous T-cell lymphoma, adult Hodgkin's disease, childhood Hodgkin's disease, Hodgkin's disease during pregnancy, mycosis fungoides, adult non-Hodgkin's lymphoma, childhood non-Hodgkin's lymphoma, non-Hodgkin's lymphoma during pregnancy, primary central nervous system lymphoma, Sezary syndrome, cutaneous T-cell lymphoma, Waldenström's macroglobulinemia, multiple myeloma/plasma cell neoplasm, myelodysplastic syndrome, and myeloproliferative disorders).

Also included as targets for treatment by practice of this inventive method include brain cancers (e.g., adult brain tumor, childhood brain stem glioma, childhood cerebellar astrocytoma, childhood cerebral astrocytoma, childhood ependymoma, childhood medulloblastoma, supratentorial primitive neuroectodermal and pineal, and childhood visual pathway and hypothalamic glioma), digestive/gastrointestinal cancers (e.g., anal cancer, extrahepatic bile duct cancer, gastrointestinal carcinoid tumor, colon cancer, esophageal cancer, gallbladder cancer, adult primary liver cancer, childhood liver cancer, pancreatic cancer, rectal cancer, small intestine cancer, and gastric cancer), musculoskeletal cancers (e.g., childhood rhabdomyosarcoma, adult soft tissue sarcoma, childhood soft tissue sarcoma, and uterine sarcoma), and endocrine cancers (e.g., adrenocortical carcinoma, gastrointestinal carcinoid tumor, islet cell carcinoma (endocrine pancreas), parathyroid cancer, pheochromocytoma, pituitary tumor, and thyroid cancer).

Further included as targets for the practice of the inventive methods described herein are neurologic cancers (e.g., neuroblastoma, pituitary tumor, and primary central nervous system lymphoma), eye cancers (e.g., intraocular melanoma and retinoblastoma), genitourinary cancers (e.g., bladder cancer, kidney (renal cell) cancer, penile cancer, transitional cell renal pelvis and ureter cancer, testicular cancer, urethral cancer, Wilms' tumor and other childhood kidney tumors), respiratory/thoracic cancers (e.g., non-small cell lung cancer, small cell lung cancer, malignant mesothelioma, and malignant thymoma), germ cell cancers (e.g., childhood extracranial germ cell tumor and extragonadal germ cell tumor), skin cancers (e.g., melanoma, and merkel cell carcinoma), gynecologic cancers (e.g., cervical cancer, endometrial cancer, gestational trophoblastic tumor, ovarian epithelial cancer, ovarian germ cell tumor, ovarian low malignant potential tumor, uterine sarcoma, vaginal cancer, and vulvar cancer), and unknown primary cancers.

Specific breast cancers that can be treated by practice of the invention include, but are not limited to, non-invasive cancers, such as ductal carcinoma *in situ* (DCIS), intraductal carcinoma lobular carcinoma *in situ* (LCIS), papillary carcinoma, and comedocarcinoma, or invasive cancers, such as adenocarcinomas, or carcinomas, e.g., infiltrating ductal carcinoma, infiltrating lobular carcinoma, infiltrating ductal and lobular carcinoma, medullary carcinoma, mucinous (colloid) carcinoma, comedocarcinoma, Paget's Disease, papillary carcinoma, tubular carcinoma, and inflammatory carcinoma. Specific prostate cancers may include adenocarcinomas and sarcomas, or pre-cancerous conditions, such as prostate intraepithelial neoplasia (PIN). Specific lung cancers include those relating to tumors such as bronchial carcinoid (bronchial adenoma), chondromatous hamartoma (benign), solitary lymphoma, and sarcoma (malignant) tumors, as well as lung cancers relating to multifocal lymphomas. Bronchogenic carcinomas may present as squamous cell carcinomas, small cell carcinomas, non-small cell carcinomas, or adenocarcinomas. It is understood that for specific cancers, the peptides may be delivered directly to the affected areas or organs such as, for example, spleen, liver, prostate, ovary, colon, or the central nervous system.

IGF-1R antagonist peptides and proteins of the invention may be administered individually, or in combination with other IGF-1 or IGF-1R antagonists or inhibitors. Alternatively, the disclosed IGF-1R antagonist peptides can be used in combination with other cancer therapies, e.g., surgery, radiation, biological response modification, immunotherapy, hormone therapy, and/or chemotherapy. For prostate cancers, non-limiting examples of chemotherapeutic agents include docetaxel, paclitaxel, estramustine, etoposide, vinblastine, mitoxantrone, and paclitaxel. For breast cancers, non-limiting examples of chemotherapeutic and biological agents include cyclophosphamide, methotrexate, 5-fluorouracil, doxorubicin, tamoxifen, paclitaxel, docetaxel, navelbine, capecitabine, mitomycin C, Interferons, interleukin-2, lymphocyte-activated killer cells, tumor necrosis factors, and monoclonal antibodies (e.g., mAb to HER-2/neu receptor (trastuzumab) Herceptin®). For lung cancers, non-limiting examples of chemotherapeutic and biological agents include, but are not limited to, platinum compounds (e.g., cisplatin or carboplatin), vinca alkaloids (e.g., vinorelbine, vincristine, or vinblastine), taxines (e.g., docetaxel or paclitaxel), and various topoisomerase inhibitors.

In another aspect, IGF-1R antagonists of the invention can be used to reduce one or more aspects of cancer progression or tumor progression in a mammalian host, such as a human patient. "Cancer progression" generally refers to any event or combination of events that promote, or which are indicative of, the transition of a normal, non-neoplastic cell to a cancerous, neoplastic cell; the migration of such neoplastic cells; and the formation, growth, and spread of tumors therefrom (which latter aspect can be referred to as tumor progression). Examples of such events include phenotypic cellular changes associated with the transformation of a normal, non-neoplastic cell to a recognized pre-neoplastic phenotype, and cellular phenotypic changes that indicate transformation of a pre-neoplastic cell to a neoplastic cell. Methods of the invention can be used to reduce any aspect of cancer or tumor progression associated with IGF-1R. In a particular exemplary aspect, a reduction of cancer progression means a reduction in the increase (growth) and/or survival of preneoplastic and/or neoplastic cells.

### KITS

Also provided are kits for practicing the subject methods. The subject kits may vary greatly in regards to the components included. The subject kits at least include the peptides and/or proteins of the present invention that bind to human insulin-like growth factor-1 receptor (HIGF-1R). In certain aspects, the subject kits may further include nucleic acids encoding the peptides or proteins of the present invention, vectors and cells comprising such nucleic acids, and pharmaceutical compositions comprising such compounds.

In certain embodiments, the subject kits include instructions for a patient to carry out administration to treat cancer.

The instructions may be recorded on a suitable recording medium or substrate. For example, the instructions may be printed on a substrate, such as paper or plastic, etc. As such, the instructions may be present in the kits as a package insert, in the labeling of the container of the kit or components thereof (i.e., associated with the packaging or sub-packaging) etc. In other embodiments, the instructions are present as an electronic storage data file present on a suitable computer readable storage medium, e.g. CD-ROM, diskette, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, this means for obtaining the instructions is recorded on a suitable substrate

Some or all components of the subject kits may be packaged in suitable packaging to maintain sterility. In many embodiments of the subject kits, the components of the kit are packaged in a kit containment element to make a single, easily handled unit, where the kit containment element, e.g., box or analogous structure, may or may not be an airtight container, e.g., to further preserve the sterility of some or all of the components of the kit.

### EXAMPLES

This invention is further illustrated by the following examples which should not be construed as limiting.

The following exemplary experimental methods and data is presented to better illustrate various aspects of the invention, but in no event should be viewed as limiting the scope of the invention.

### Brief Description of Materials and Methods

Experiments used to obtain the data provided herein were performed using standard methods, similar to those previously described in the above-cited '246 PCT, `771 PCT, and '147 US patent applications. As such, some of the specific methods used in obtaining this data are only briefly described here.

### EXAMPLE 1

### IGF-1R Affinity Studies

IGF-1R affinity studies were generally performed as follows. Human IGF-1R (HIGF-1R) was incubated with ¹²⁵ I-labeled human IGF-1 at various concentrations of test substance and the K_{d} was calculated. HIGF-1R was purified from a cell culture of BHK cells stably transfected with IGF-1R cDNA. The day before harvest cells were moved to serum free conditions and before harvest the cells were rinsed with and ice cold PBS solution. The cells were solubillized in cold lysis buffer (4ml lysis buffer/1x10⁸ cells); vortexed vigorously to lysis in lysis buffer (50 mM Hepes (pH 8.0); 150 mM NaCl; 1% Triton X-100; 2 mM EDTA; 10µg/ml aprotinin; 0.5 mM PEFABLOC (Roche)); and the lysate centrifuged at 4° C, 20000 rpm, for 15 minutes. The HIGF-1R portion was absorbed to WGA-agarose beads and subjected to chromatography purification (Pharmacia biotech No 17-0760-01) (column washed with 20 x column volumes wash buffer - 50 mM Hepes (pH 8.0); 150 mM NaCl; 0.1% Triton X-100; 0.5 mM PEFABLOC (Roche)). The HIGF-1R portion was then eluted with 0.5 M n-Acetyl Glycosamine (NAG), 10% Glycerol in elution buffer (50 mM Hepes (pH 8.0); 150 mM NaCl; 0.1% Triton X-100; 0.5 mM PEFABLOC (Roche); 0.5 M n-acetyl glucosamine; 10% glycerol). The eluate was tested for IGF-1 receptor tyrosine kinase activity and IGF-1 binding activity (described further below) and the fraction exhibiting such activity (corresponding to the HIGF-1R portion) was collected for use in the experiments described herein. For affinity measurements, the receptor concentration was generally chosen to give 30-60% binding of 2000 cpm (3 µM) of its ¹²⁵ I-labeled ligand (Tyr31-¹²⁵I-IGF1) and a dilution series of the substance to be tested was added. After equilibration for 2 days at 4°C, each sample (200 µl) typically was precipitated by addition of 400 µl 25% PEG 6000, centrifuged, washed with 1 ml 15% PEG 6000, and counted in a gamma-counter. In some cases, human insulin receptor (HIR) affinity for select test peptides was similarly measured.

In certain cases, a scintillation proximity assay (SPA assay) was used to determine IGF-1R affinity. Briefly, anti-mouse SPA beads were mixed with an HIGF-1R antibody (24-31) and WGA-purified HIGF-1R from transfected cells. The binding buffer was 0.1 M Hepes, pH 7.8, 0.1 M NaCl, 10 mM MgCl₂, 0.025% Tween-20, 0.5% BSA. To this mixture was added ¹²⁵I-IGF1 and a dilution series of IGF1 or the peptide to be determined. After 16 hours incubation at room temperature, the 96-well plates were centrifuged and counted in a topcounter and the IC50 for binding was used as a measure of K_{d}.

The results of these affinity studies on a number of peptides and peptide derivatives are presented in Table 4. Through this investigation, a number of new peptides exhibiting high affinity for IGF-1R were identified.

### EXAMPLE 2

### IGF-1R Activation Studies

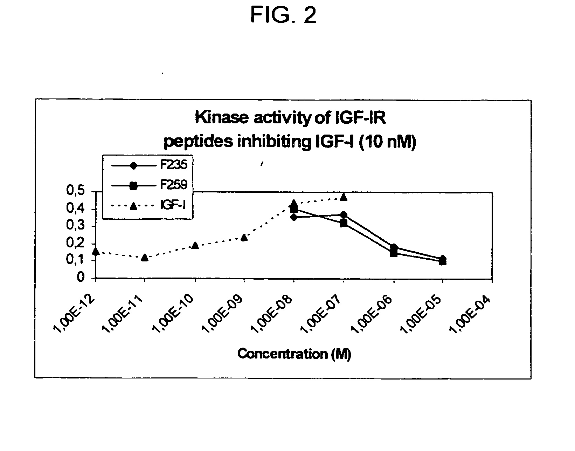

For determining IGF-1R activation with test peptides, a kinase assay, generally using the following conditions was employed. A solution of each test peptide in kinase buffer (Hepes 100 mM (pH 8,0), MgCl₂ 8 mM, MnCl₂ 6 mM, Triton X100 0.1% v/v, BSA 0.2 % w/v, 4 µg/ml IRS-1 peptide (Biotin-KSRGDYMTMQIG in PBS+ 1 mg/ml)) was prepared. Samples were mixed in a microtiter plate in triplicate. 50 µl 2 x kinase buffer was added into each well. IGF-1 was to the appropriate wells (10⁻⁷M, 10⁻⁸M, 10⁻⁹M, 5x10⁻¹⁰M, 10⁻¹⁰M and 10⁻¹¹M). Volume was adjusted to 88 µL, before addition of purified HIGF-1R (2 µl/well). Plates were shaken and incubated for 50 min. at room temperature (RT). DTT and 10 µl/well ATP were added and the wells incubated for 10 min. before stopping the reaction by adding 10 µl stop-buffer (50 mM EDTA (pH 8.0))/well and shaking. The samples were then transferred to streptavidin coated plates and incubated for 1 hour at RT. The coated plates were aspirated and washed 3 times with TBS-T. 100 µl P-tyr Ab-PO (Phosphotyrosine-RC20:HRPO (1:1000) in TBS-T) was added and each plate was incubated for 30 min at RT. The plates were aspirated and washed 3 times with TBS-T. 100 µl TMB-One-substrate (Kem-En-Tec Diagnostics A/S - Denmark) (preheated to RT) was then added and the plates further incubated for approximately 20 min at RT. This reaction was stopped by adding 10 µl H₂SO₄ and the plates were read in an ELISA-reader at 450 nM within 5-10 min. thereafter. The results of studies performed with peptides F235 and F259 are graphically presented in Fig. 2. Table 7 includes additional results from such experiments.

As shown in Figure 2, peptides F235 and F259 show the ability to completely inhibit IGF-1R kinase activation by IGF-1. Both peptides show similar potency with IC₅₀ of 2-3 x 10⁻⁷ M. Several of the peptides listed in Table 7 also inhibit IGF-IR activation by IGF-1, supporting the conclusion that these peptides do not only bind the receptor but work as IGF-1R antagonists. Only the peptides indicated as having no effect did riot function as antagonists. For several of the antagonistic peptides the autophosphorylation was inhibited to a level below the level of basal autophosphorylation of the receptor, with no IGF-1 added (data not shown). This data reflects that peptides of the invention can act as IGF-1R antagonists.

### EXAMPLE 3

### Cell Growth/Density Studies

For cell growth/density studies, a mitochondrial activity assay was used, as exemplified by the following. Cells (SW480, MCF-7S8) were propagated in complete medium (DME: F12, 10 % FCS (SW 480) or 5 % FCS (MCF-7S8, P/S)), trypsinized, and seeded in 96 well plates as 10,000 cells/well in medium with 0.5 % FCS. All wells in the periphery were omitted and only filled with medium. After plating overnight, the volume in the wells was adjusted to 50 µL and the cells were dosed with IGF-1 and/or test peptide by adding 50 µL medium with twice the desired concentration. All treatments are made in triplicates. In each experiment a dose response curve for IGF-1 was made (IGF-1: 100, 10, 2, 1, 0.1, 0.01 nM final). Treatment was continued for 48 hours (SW480 cells) or 72 hours (MCF-7S8 cells) before quantification of the cell density. Media was exchanged each day. Media and peptides were diluted in DMSO. When measuring cell density, the volume of each well was adjusted to 100 µL. Pre-diluted WST-1 (1 ml + 1.5 ml DME:F12) was added as 25 µL to each well including background samples. Three wells with media only served as background controls. The cells were incubated for 90 min protected against light in an incubator. An ELISA reader was used read the absorbance at 450 nm (620 nm reference).

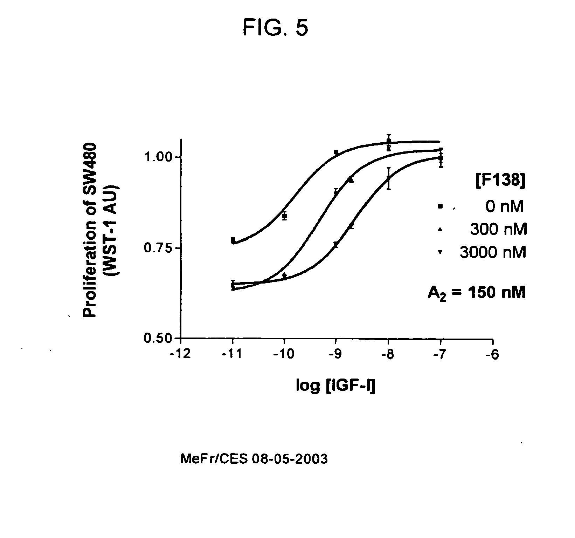

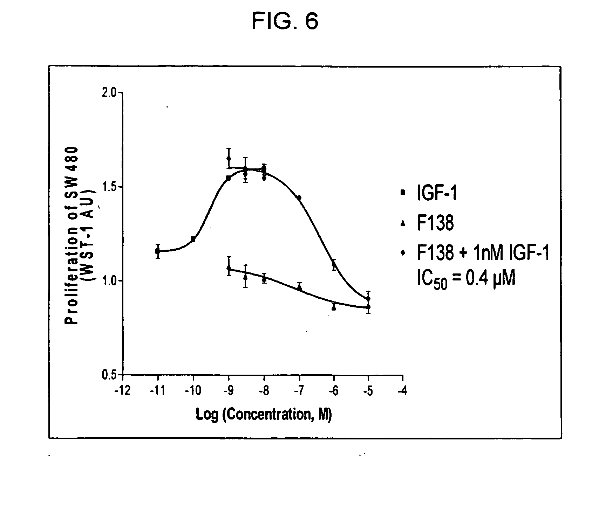

The results of such experiments are presented in Tables 5 and 6 and are graphically presented for peptide F138 in Figures 5 and 6 (at different doses and in association with IGF-1, respectively) (the peptide effect on cell density in SW480 and MCF-7S8 cells reported in Table 5 was measured with peptide treatment alone as well as in parallel with peptide competing with IGF-1 (10 nM) (repeated experiments performed with certain peptides also noted in applicable rows), whereas Table 6 only present data from treatment of SW480 with peptide in competition with IGF-1 (10 nM); empty cells means no data was collected for the indicated parameters; ED₅₀ means efficacy dose for 50% of maximal effect; IC₅₀ means inhibitory concentration for 50% inhibition). Peptides for which the IC₅₀ could not be determined are reported as slightly antagonistic (if some tendency for effect was seen at high dose) or as no effect if no change in cell density was seen.

The antagonistic effect of F138 and F293 peptides inhibiting IGF-1 stimulated growth of L6 cells was tested in a similar way. The stimulation of growth by IGF-1 at 3 nM could be inhibited by both peptides. F138 inhibited growth more potently with IC₅₀ of 4x10⁻⁶ M, whereas F293 inhibited with IC₅₀ > 1x10⁻⁵ M.

These results demonstrate that peptides provided by the invention exhibit IGF-1R antagonist properties, and in some cases are potent IGF-1R antagonists. These results demonstrate that peptides provided by the invention can exhibit anti-proliferative effects in cancer cells and, accordingly, can be useful as, or in the development of, novel anti-cancer therapeutic agents.

### EXAMPLE 4

### Assessment of Insulin Agonist Activity

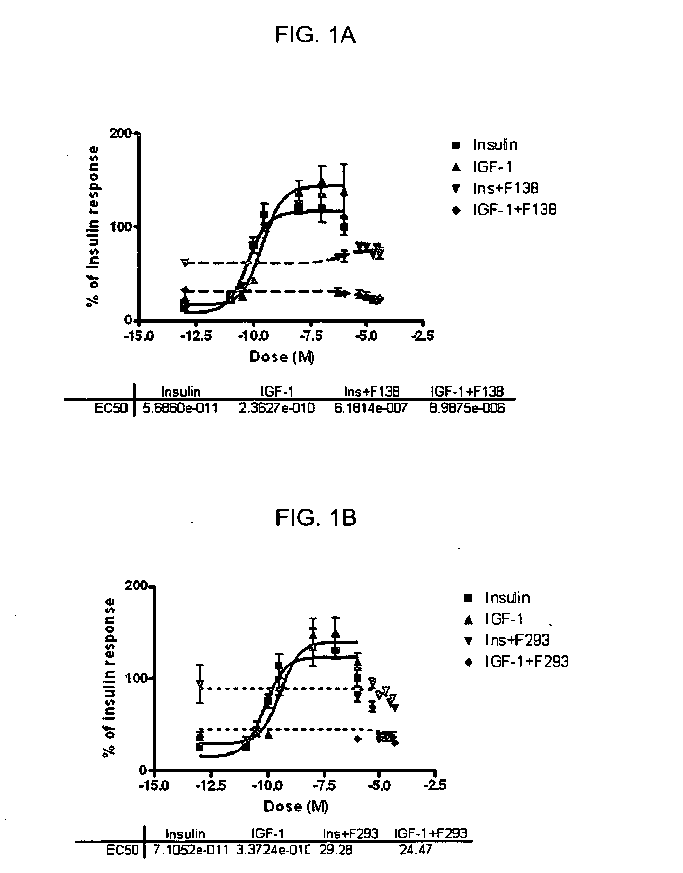

Insulin increases uptake of ³H glucose into adipocytes and its conversion into lipid. Incorporation of ³H into the lipid phase was determined by partitioning of lipid phase into a scintillant mixture, which excludes water-soluble ³H products. Using a method substantially similar to that described in Example 4 of the '246 PCT application, in SGBS adipocytes transfected with HIR-encoding DNA, the effect of peptide F293 and (separately) peptide F138 on the incorporation of ³H glucose in the presence of insulin or IGF-1 was determined (and compared to controls). The results are expressed as increase relative to full insulin response. Data obtained from these experiments are presented graphically in Figures 1A and 1B, as effect of peptide F138 or peptide F293 or (in combination with insulin or with IGF-1) on an (approximate) ED₂₀ insulin response, with data normalized to a full insulin response, respectively. The results of these experiments demonstrate that neither peptide F138 nor peptide F293 has any substantial effect on glucose uptake induced by insulin or IGF-1. These results demonstrate that peptides of the invention exhibit specificity for blocking IGF-1R and not insulin receptor, despite the significant similarities in these receptors (as described in the '247 PCT application).

### EXAMPLE 5

### Phosphorylation of HIR and/or HIGF-IR

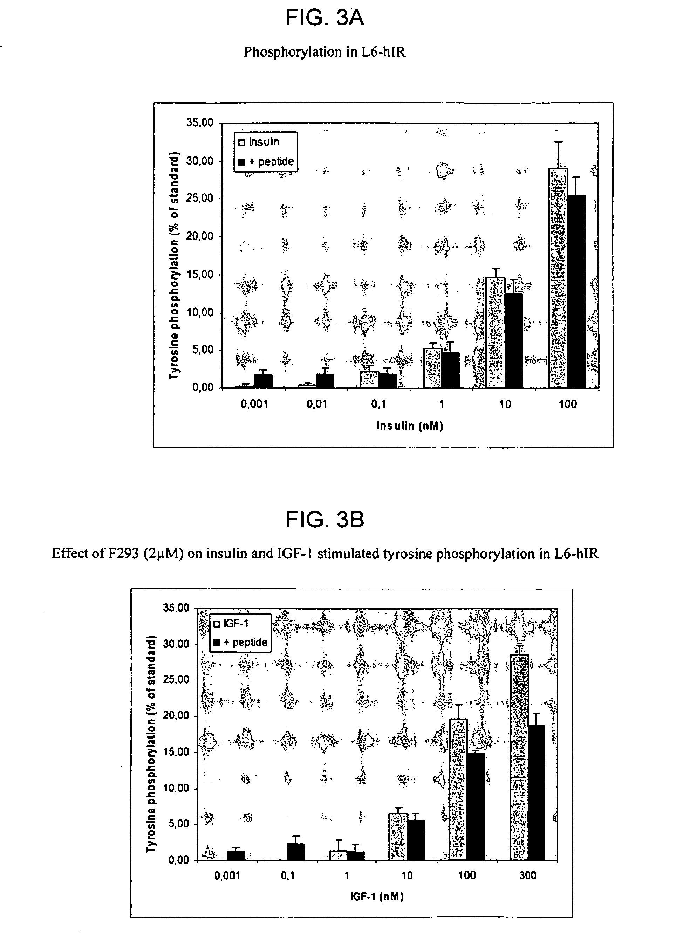

The activation of HIR or HIGF-1R in adipocytes was measured by stimulating the cells and studying the level of tyrosine phosphorylation (Western blot) of the insulin receptor/IGF-1R. This was done by incubating L6-hIR (a cell clone expressing physiological levels of the insulin receptor, i.e., 100,000 receptors/cell) as well as high levels of IGF-1R and incubating these cells with increasing amounts of insulin or IGF-1 alone or in presence of the test peptide for 10 min. The IGF-1R will be present in symmetric homodimer complex as well as in the heterodimer with half insulin receptor. Compared to the basal level (set to 1) stimulation for 10 min with increasing concentrations of insulin leads to increasing tyrosine phosphorylation of the insulin receptor in the L6-hIR cells and more potently than IGF-1 in these cells (Figures 3A, 3B). Using 2 µM F293 results in marginal effect for insulin stimulated tyrosine phosphorylation, whereas the tyrosine phosphorylation by IGF-1 is inhibited. These results suggest that F293 specifically inhibits IGF-1R tyrosine phosphorylation and only for the hybrid (IGF-1R/1R) receptors stimulated by insulin at high concentration is phosphorylation seen (as a marginal effect), whereas stimulation by IGF-1 more clearly become inhibited in the receptor activation.

### EXAMPLE 6

### Downstream Signaling Studies

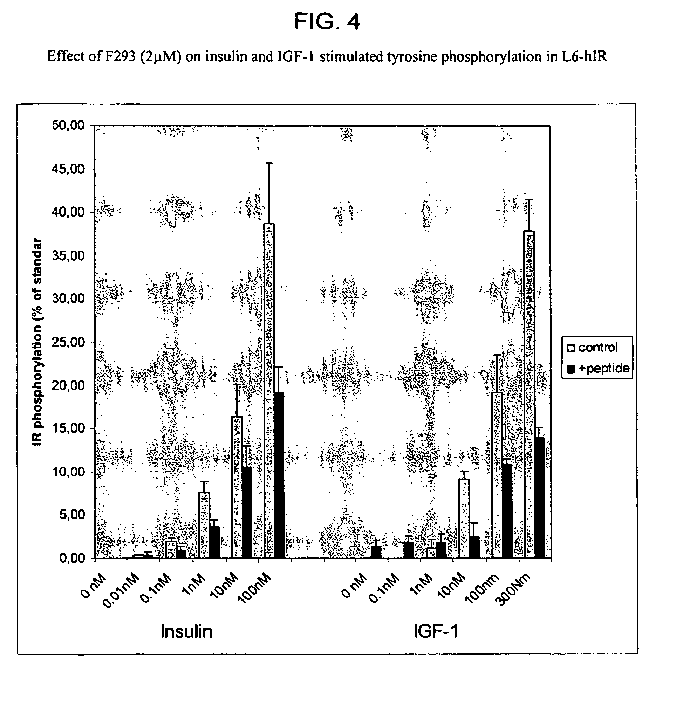

The tyrosine phosphorylation of IRS signaling (the 180 kDa band on a tyrosine phosphor Western blot) as well as activation of effectors MAPK 44 and 42 and PKB were analyzed by using antibodies specific for their active forms using methods substantially similar to those described in Example 14 of the '246 PCT application. IRS is tyrosine phosphorylated by both insulin and IGF-1 at 10 min, with roughly similar effects (see Fig. 4). The presence of peptide F293 clearly inhibits the IGF-1 stimulated IRS-1 phosphorylation most markedly again suggesting the specificity of F293 for binding IGF-1R and not insulin receptor (see Fig. 4).

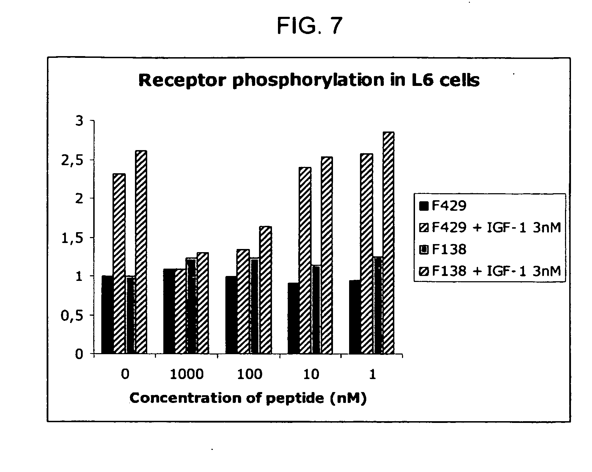

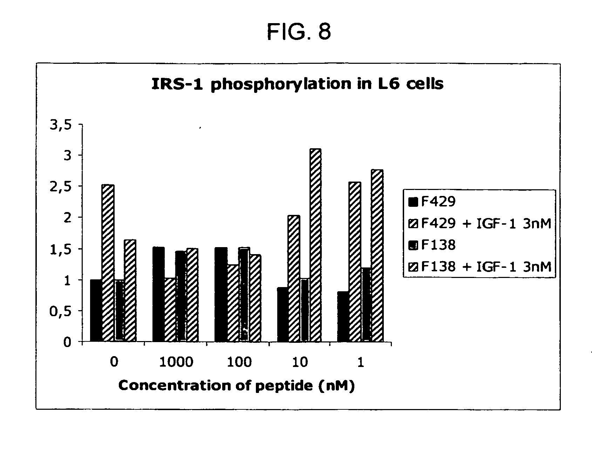

Similarly testing IRS-1 phosphorylation in presence of either F429 or F138 +/- 3 nM IGF-1 show the potency of F429 is higher than F138 for inhibiting the down stream signaling through IRS-1 upon IGF-1 stimulation, without affecting basal level (Fig. 7 and Fig. 8).

The effect of select peptides on downstream signaling (MAPK44, MAPK42, PKB/Akt, IRS-1, and IGF-1R) was also measured by studying the signaling in L6 cells predominantly expressing IGF-1R and very limited insulin receptors. The results of these experiments are presented in Table 8 (numbers are given as IC₅₀ for down regulation of the stimulation by 3 nM IGF-1 to the basal level). The antagonistic effect of the peptides can be seen to affect all the downstream signaling from the IGF-1R.

These data demonstrate that peptides of the invention are able to down regulate IRS-1 downstream signaling and further reflects the IGF-1R specific nature of peptides provided by the invention.

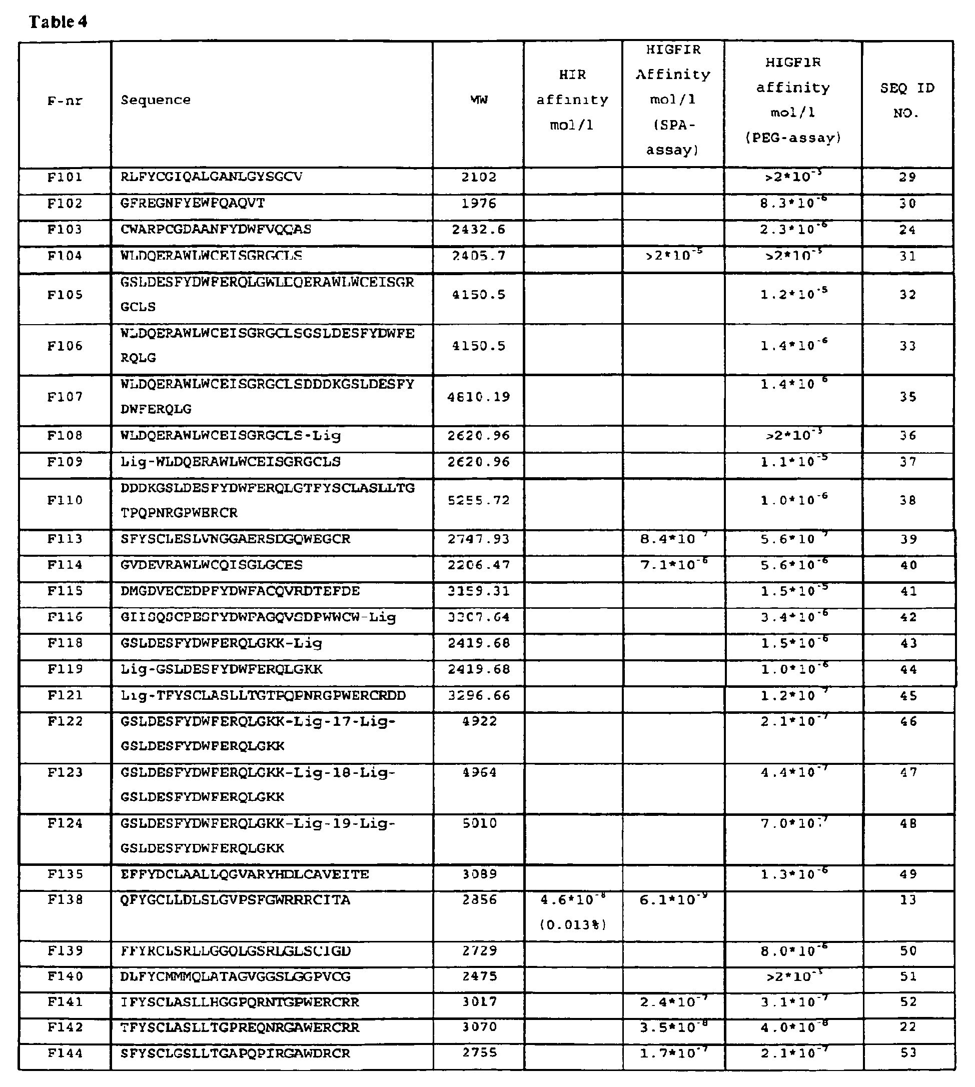

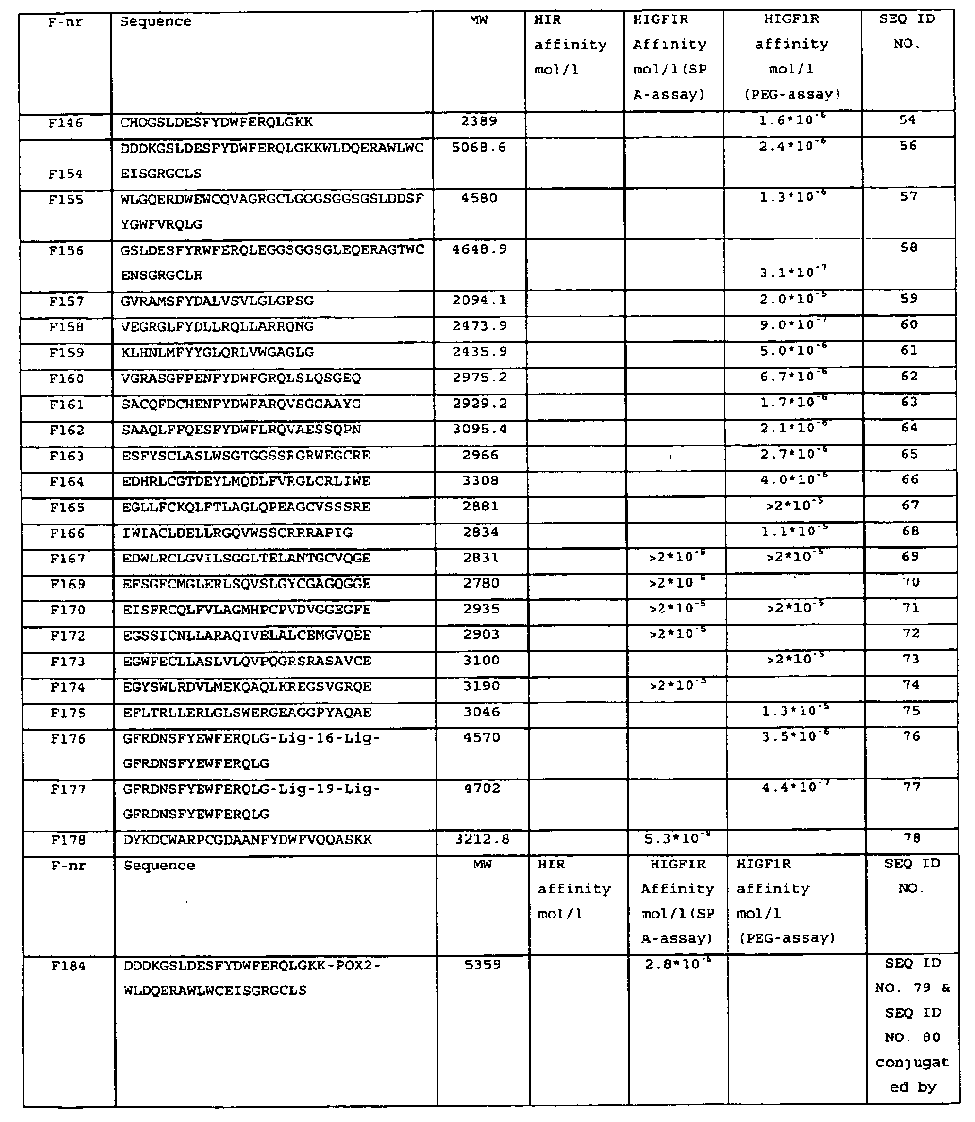

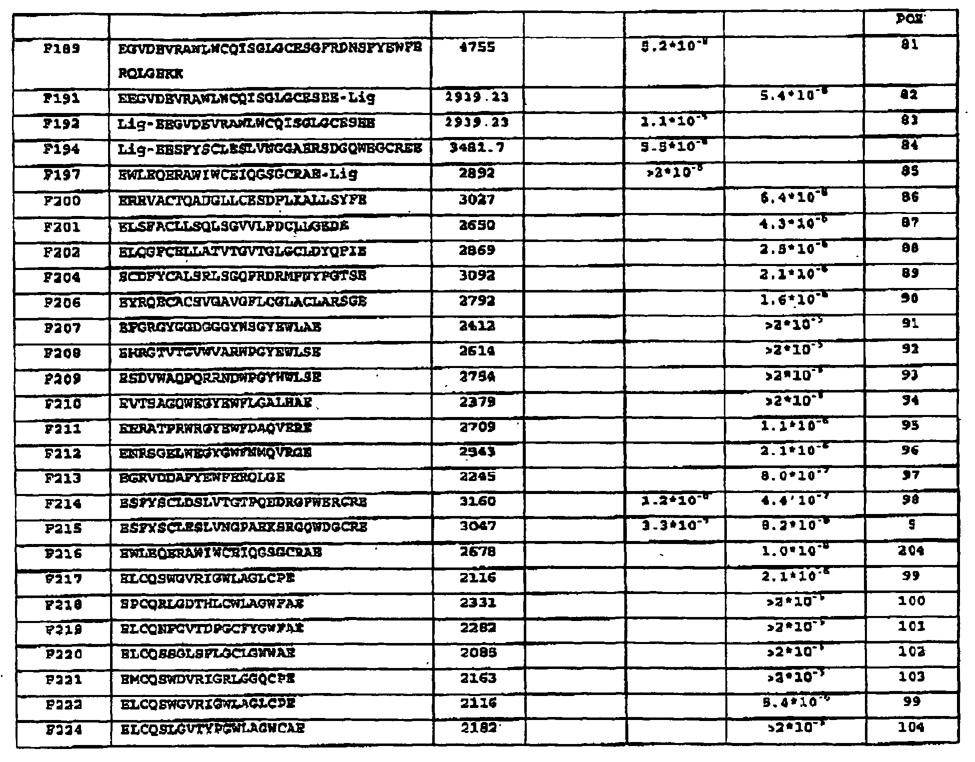

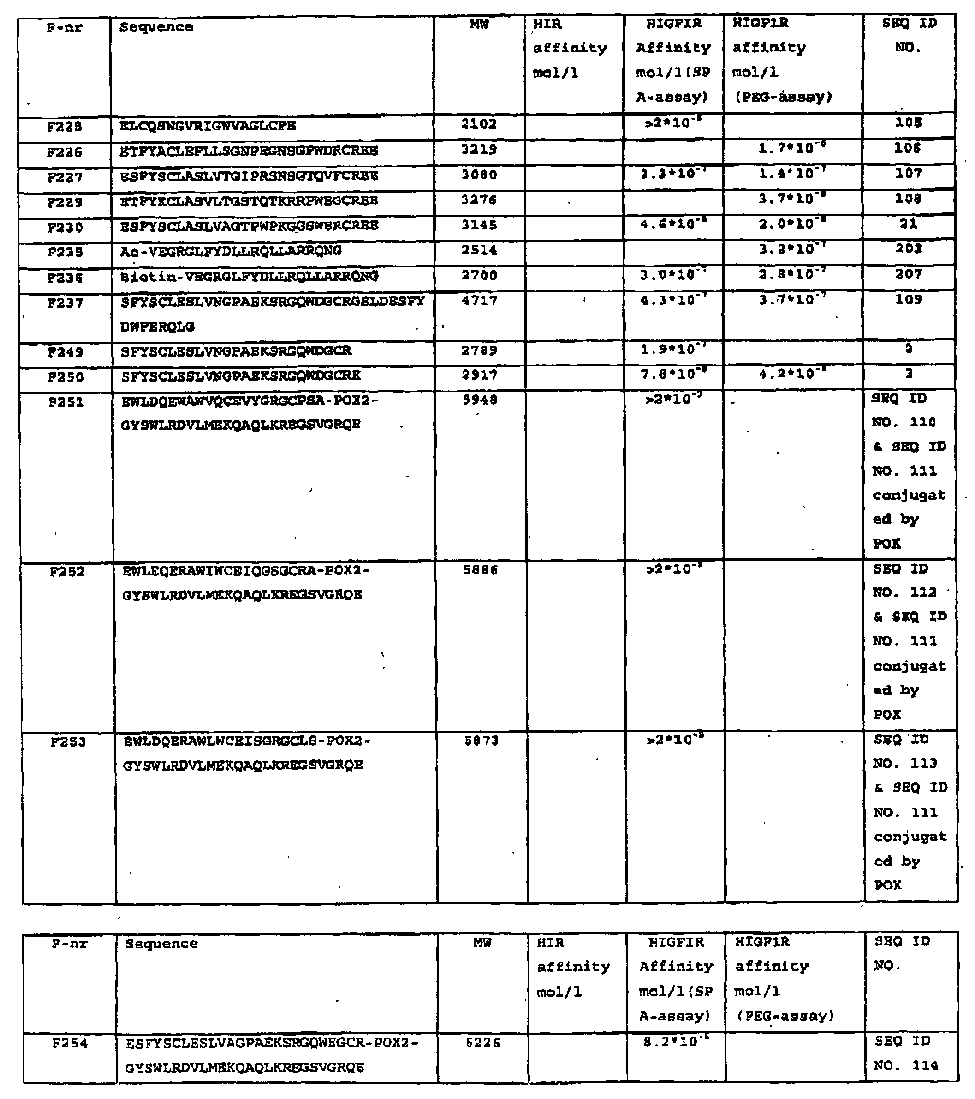

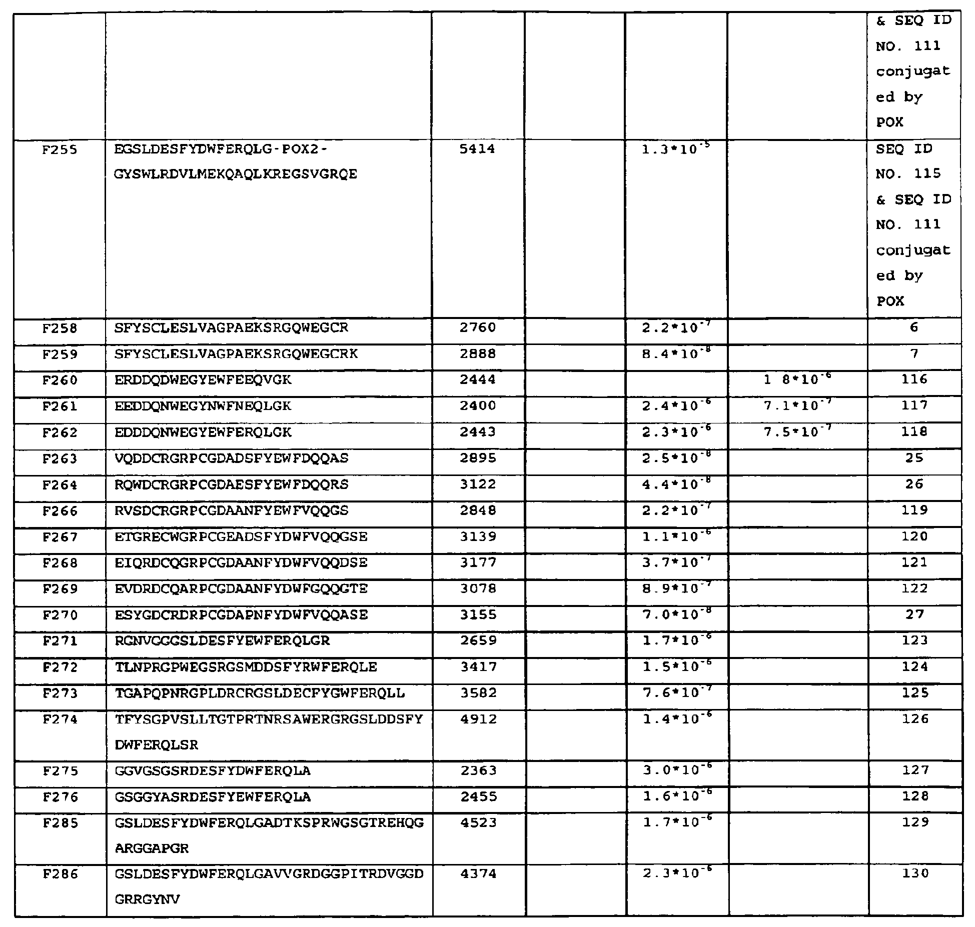

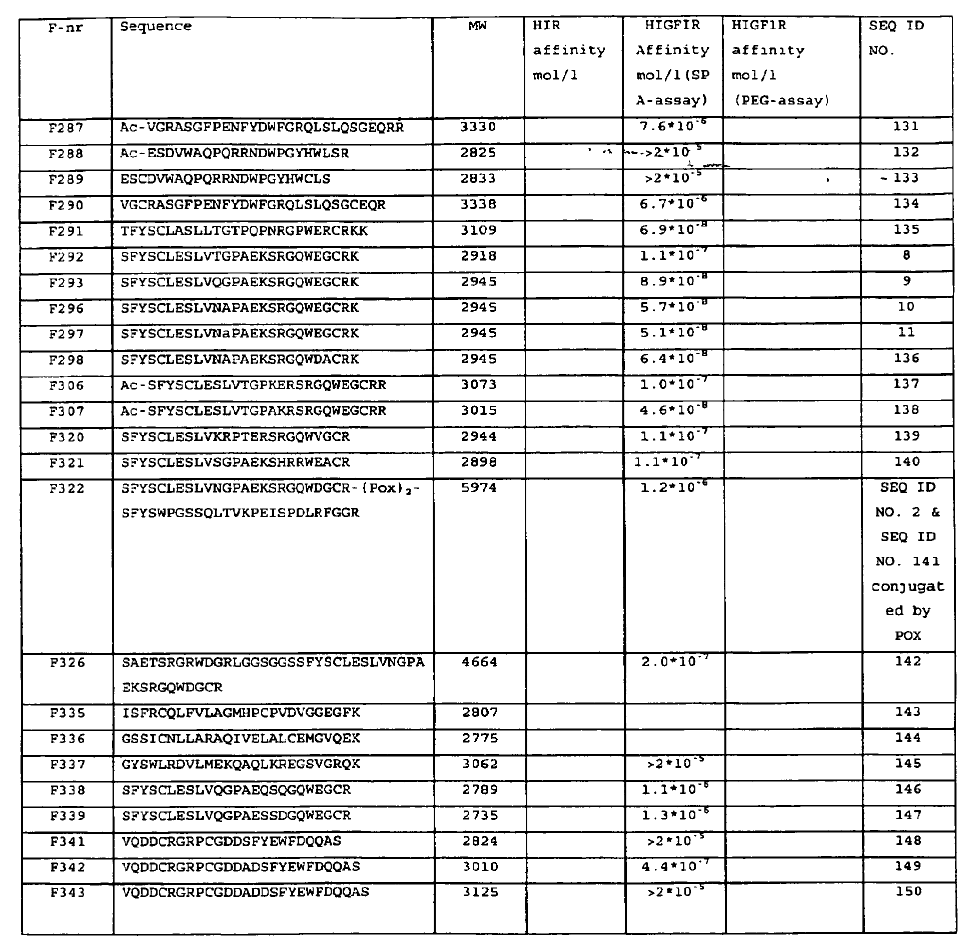

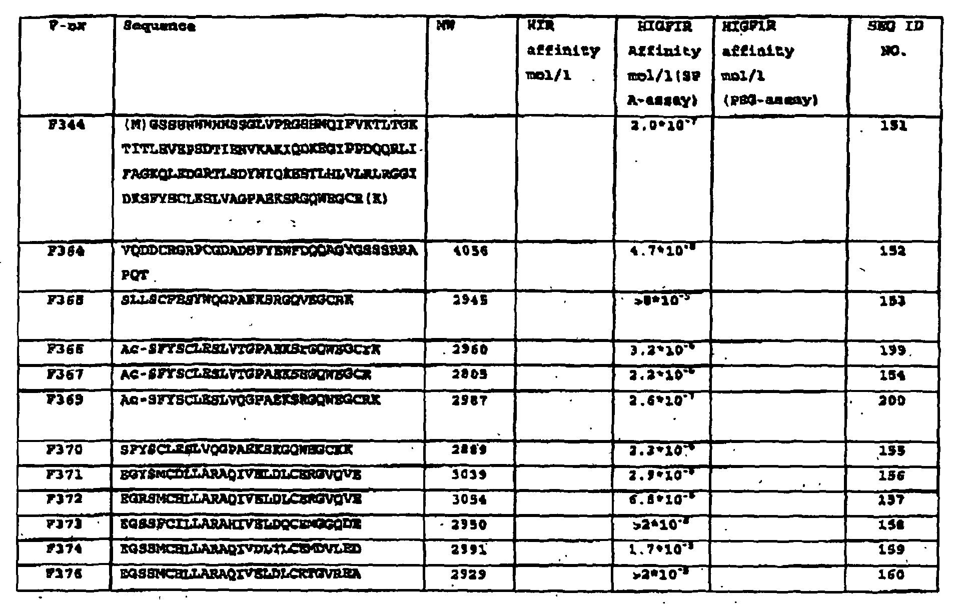

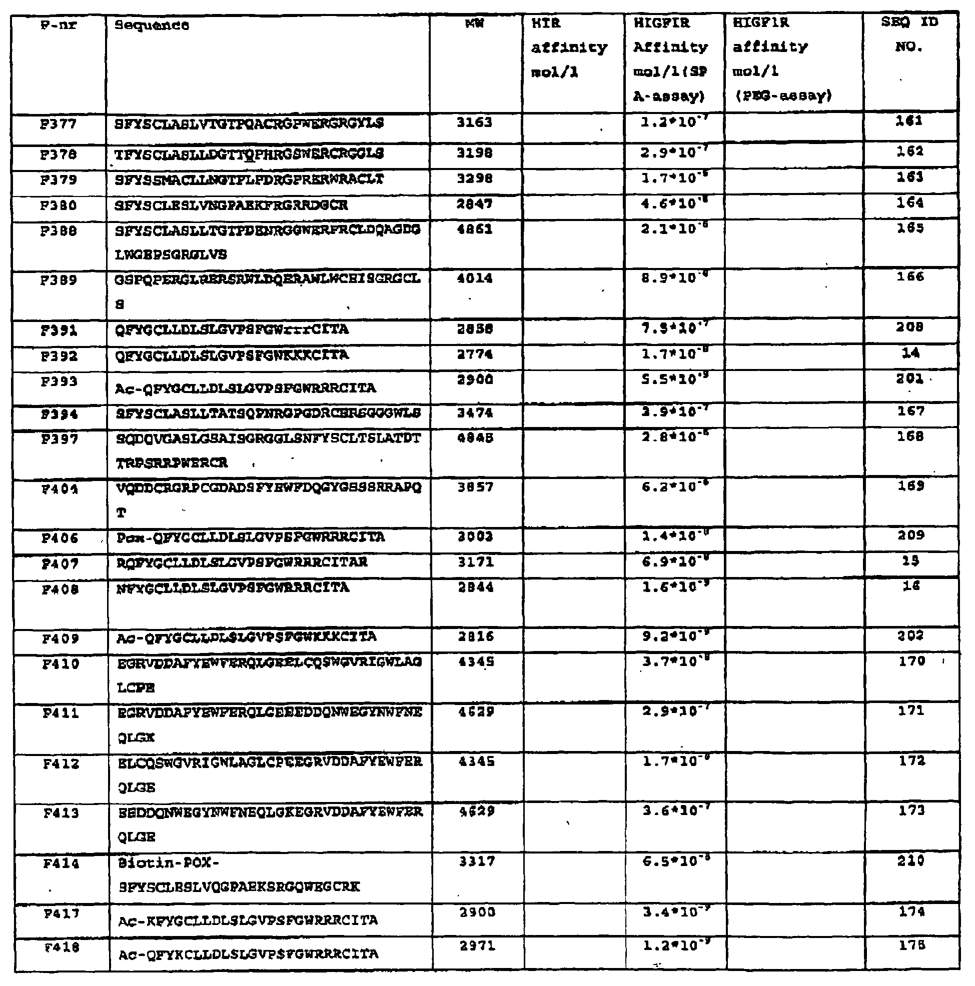

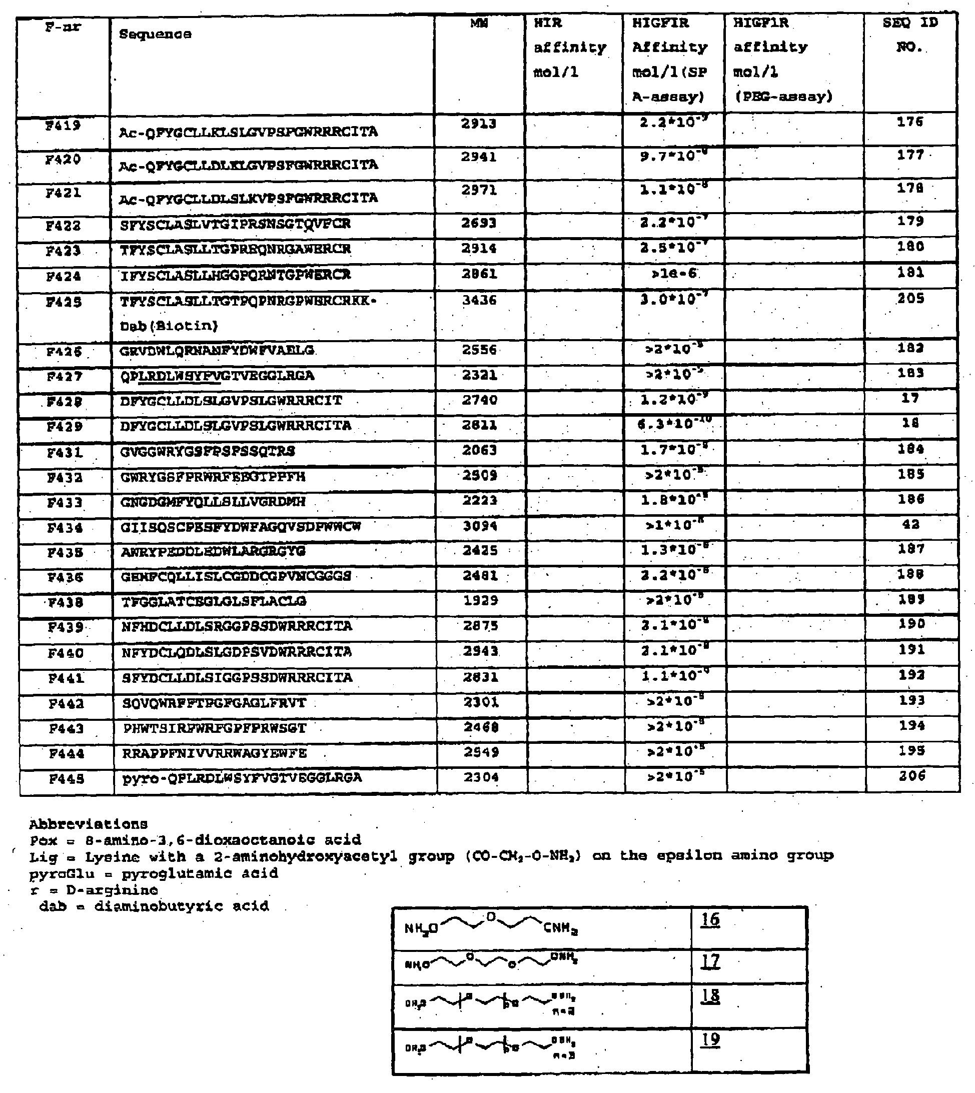

**Table 5**

The following data reflect the results of cell density and kinase assays performed on selected test peptides. IC₅₀ in cell density assays are indicated by two values i.e. the concentration needed to blunt the IGF-1 effect using 10 nM IGF-1 and by the concentration needed to inhibit basal level in 0.5 % FCS.

| F-nr | IC₅₀ in SW480 (M) in presence of IGF-1/alone | IGF-1 ED₅₀ (M) in same experiment | IC₅₀ in MCF-7S8 | IGF-1 ED₅₀ in same experiment |
|---|---|---|---|---|
| F108 | Slightly agonistic | 2x10⁻⁹ | | |
| F109 | Slightly agonistic | 2x10⁻⁹ | | |
| F110 | Slightly agonistic | 2x10⁻⁹ | | |
| F115 | No effect | 2x10⁻⁹ | | |
| F116 | No effect | 2x10⁻⁹ | | |
| F118 | No effect | 2x10⁻⁹ | | |
| F122 | No effect | 2x10⁻⁹ | | |
| F123 | Antagonistic | 2x10⁻⁹ | | |
| F124 | Antagonistic | 2x10⁻⁹ | | |
| F138 | Antagonistic | | 5x10⁻⁷/no effect | 5x10⁻¹⁰ |
| F139 | Antagonistic | 2x10⁻⁹ | | |
| F140 | Slightly antagonistic | 2x10⁻⁹ | | |
| F144 | No effect | 2x10⁻⁹ | | |
| F146 | Slightly agonistic | 2x10⁻⁹ | | |
| F154 | Slightly antagonistic | 2x10⁻⁹ | | |
| | | | | |

| F-nr | IC₅₀ in SW480 (M) in presence of IGF-1/alone | IGF-1 ED₅₀ (M) in same experiment | IC₅₀ in MCF-7S8 | IGF-1 ED₅₀ in same experiment |
|---|---|---|---|---|
| F155 | Slightly agonistic | 2x10⁻⁹ | | |
| F156 | No effect | 2x10⁻⁹ | | |
| F157 | No effect | 2x10⁻⁹ | | |
| F158 | No effect Slightly antagonistic | 2x10⁻⁹ 2x10⁻⁹ | | |
| F159 | Slightly antagonistic | 2x10⁻⁹ | | |
| F160 | Slightly antagonistic | 2x10⁻⁹ | | |
| F161 | No effect | 2x10⁻⁹ | | |
| F162 | No effect | 2x10⁻⁹ | | |
| F165 | No effect | 5x10⁻⁹ | | |
| F167 | Slightly agonistic | 5x10⁻⁹ | Agonistic | 5x10⁻⁹ |
| | Slightly agonistic | 5x10⁻⁹ | | |
| | Slightly agonistic | 5x10⁻⁹ | | |
| F169 | 2x10⁻⁶/no effect | 5x10⁻⁹ | 2x10⁻⁶/no effect | 2x10⁻⁹ |
| F170 | No effect | 5x10⁻⁹ | 2x10⁻⁶/no effect | 2x10⁻⁹ |
| | Slightly agonistic | 5x10⁻⁹ | | |
| | No effect | 5x10⁻⁹ | | |
| F175 | No effect | 5x10⁻⁹ | | |
| F201 | No effect | 5x10⁻⁹ | | |
| F202 | Slightly antagonistic | 1x10⁻⁹ | antagonistic | 1x10⁻⁹ |
| | No effect | 5x10⁻⁹ | no effect | 2x10⁻⁹ |
| | >10⁻⁵/no effect | 5x10⁻⁹ | | |
| F204 | Slightly agonistic | 1x10⁻⁹ | | |
| | Slightly agonistic | 5x10⁻⁹ | | |
| F207 | No effect | 5x10⁻⁹ | 2x10⁻⁶/no effect | 2x10⁻⁹ |
| | 2x10⁻⁶/no effect * | 5x10⁻⁹ | Agonistic | 5x10⁻⁹ |
| F210 | No effect | 1x10⁻⁹ | Slightly antagonistic | 1x10⁻⁹ |
| | No effect | 5x10⁻⁹ | | |
| F211 | No effect | 5x10⁻⁹ | No effect | 1x10⁻⁹ |
| F212 | No effect | 1x10⁻⁹ | No effect | 1x10⁻⁹ |
| | Slightly agonistic | 5x10⁻⁹ | | |
| F213 | No effect | 1x10⁻⁹ | Slightly antagonistic | 1x10⁻⁹ |
| | No effect or antagonistic | 5x10⁻⁹ | 2x10⁻⁶/no effect | 2x10⁻⁹ |
| | 2x10⁻⁶/no effect | 5x10⁻⁹ | No effect | 5x10⁻⁹ |
| F235 | No effect | 5x10⁻⁹ | 5x10⁻⁶/no effect | 2x10⁻⁹ |
| | No effect | 5x10⁻⁹ | No effect | Not det. |
| | | | No effect | 5x10⁻⁹ |
| F249 | >1x10⁻⁵/>1x10⁻⁵ | 1x10⁻⁹ | | |
| F250 | | | | |
| | 2x10⁻⁶/no effect | 5x10⁻⁹ | 2x10⁻⁶/no effect | 2x10⁻⁹ |
| | | | agonistic | 5x10⁻⁹ |
| | 1x10⁻⁶/no effect | 1x10⁻⁹ | agonistic | 1x10⁻⁹ |
| | | | slightly agonistic | 1x10⁻⁹ |

| F-nr | IC₅₀ in SW480 (M) in presence of IGF-1/alone | IGF-I ED₅₀ (M) in same experiment | IC₅₀ in MCF-7S8 | IGF-1 ED₅₀ in same experiment |
|---|---|---|---|---|
| F259 | Slightly antagonistic | 5x10⁻⁹ | 5x10⁻⁶/no effect | Not det. |
| | | | slightly antagonistic | 5x10⁻⁹ |
| | 2x10⁻⁶/no effect | 1x10⁻⁹ | | |
| | | | 2x10⁻⁶/no effect | 1x10⁻⁹ |
| F263 | >1x10⁻⁶/>1x10⁻⁶ | 1x10⁻⁹ | | |
| | >1x10⁻⁶/>1x10⁻⁶ | 5x10⁻¹⁰ | | |
| F264 | >1x10⁻⁵/>1x10⁻⁵, | 1x10⁻⁹ | | |
| F267 | Slightly agonistic | 1x10⁻⁹ | | |
| | | | | |
| F268 | No effect | 1x10⁻⁹ | | |
| F269 | 5x10⁻⁷/>1x10⁻⁵ | 1x10⁻⁹ | | |
| F270 | 2x10⁻⁶ | 1x10⁻⁹ | Agonistic | 1x10⁻⁹ |
| | agonistic? | | No effect | 1x10⁻⁹ |
| F287 | No effect | 1x10⁻⁹ | | |
| F288 | No effect | 1x10⁻⁹ | | |
| F292 | 1x10⁻⁷/1x10⁻⁶ | 1x10⁻⁹ | 1x10⁻⁶/no effect | 1x10⁻⁹ |
| | | | No effect | 1x10⁻⁹ |
| F293 | 1x10⁻⁷/1x10⁻⁷ | 1x10⁻⁹ | 1x10⁻⁵/>1x10⁻⁵ | 1x10⁻⁹ |
| | 2x10⁻⁶/no effect | 1x10⁻⁹ | | |
| F320 | No effect | 1x10⁻⁹ | | |
| F344 | | | 1x10⁻⁷/>1x10⁻⁵ | 5x10⁻¹⁰ |

The data set forth in the following table (Table 6) reflect inhibition of IGF-1 (10 nM)-induced proliferation of SW480 cells by selected test peptides. Numbers are IC₅₀ values in µM for the peptide.

**Table 6**

| F-nr | 13262-014 | 13262-015 | 13262-016 | 13262-025 | 13262-027 | 13262-029 | 13717-001 | 13717-005 | 13717-019 | 13717-027 |
|---|---|---|---|---|---|---|---|---|---|---|
| F113 | | >10 | >10 | | | | | | | |
| F138 | | 0.3 | 1 | 3 | 1 | 1 | 0.4 | | | |
| F141 | | >10 | | | | | | | | |
| F227 | | >10 | | | | | | | | |
| F236 | | >10 | | | | | | | | |
| F259 | >10 | | | | | | | | | |
| F292 | >10 | | | | | | | | | |
| F293 | | 10 | >10 | | | | | | | |
| F320 | >10 | | | | | | | | | |
| F321 | >10 | | | | | | | | | |
| F344 | >10 | | | | | | | | | |
| F347 | | | | | 1 | 1 | 3 | 3 | | |
| F364 | | >10 | | | | | | | | |
| F369 | | >10 | >10 | | | | | | | |
| F391 | | | | >10 | >10 | | | | | |
| F392 | | | | >10 | 1 | | | | | |
| F393 | | | | | 1 | 1 | | | | |
| F348 | | | | | | | 1 | 1 | | |
| F349 | | | | | | | 3 | 3 | | |
| F350 | | | | | | | >10 | | | |
| F353 | | | | | | | >10 | | | |
| F359 | | | | | | | >10 | | | |
| F360 | | | | | | | >10 | | | |
| F363 | | | | | | | >10 | | | |
| F346 | | | | | | | >10 | >10 | | |
| F405 | | | | | | | | | >10 | >10 |
| F428 | | | | | | | | | 1 | 1 |
| F429 | | | | | | | | | 0.5 | 1 |

**Table 7 - IGF-1R kinase activation by 10nM IGF-1 in presence of indicated peptides**

| F number | IC₅₀ in kinase assay (M) |
|---|---|
| F138 | 1-2x10⁻⁷ |
| F167 | 1x10⁻⁶ |
| F213 | No effect |
| F207 | 2x10⁻⁷ |
| F235 | 2x10⁻⁷ |
| F250 | 2-5x10⁻⁷ |
| F259 | 1-3x10⁻⁷ |
| F269 | 8x10⁻⁷ |
| F293 | 1x10⁻⁷ |
| F365 | No effect |

### Table 8 - Effects of Select Peptides on IGF-1R-Associated Downstream Signaling

Effect of antagonistic peptides was measured by studying the signaling in L6 cells predominantly expressing IGF-1R and very limited insulin receptors. Numbers are given as IC₅₀ for down regulation of the stimulation by 3 nM IGF-1 to the basal level.

| treatment | PKB/Akt | MAPK44 | MAPK42 | IGF-IR | IRS-1 |
|---|---|---|---|---|---|
| F320 | No data | 1x10⁻⁶ | 1x10⁻⁶ | 2x10⁻⁶ | >2x10⁻⁶ |
| F138 | 3x10⁻⁷ | 3x10⁻⁷ | 3x10⁻⁷ | 3x10⁻⁷ | 3x10⁻⁷ |
| | 1x10⁻⁷ | 1x10⁻⁷ | 3x10⁻⁷ | 6x10⁻⁸ | No data |
| F293 | > 1x10⁻⁶ | 5x10⁻⁷ | 1x10⁻⁶ | 5x10⁻⁷ | > 1x10⁻⁶ |
| F320 | No data | 1x10⁻⁶ | 1x10⁻⁶ | > 1x10⁻⁶ | > 1x10⁻⁶ |
| F429 | 1x10⁻⁷ | 1x10⁻⁷ | 3x10⁻⁷ | 5x10⁻⁸ | 4x10⁻⁸ |

### EXAMPLE 7

The following additional studies help to further exemplify and support various aspects of the invention.

### Materials and Methods

The following materials and methods were employed in conducting the additional studies:

Cells and Reagents. MCF7 and MiaPaCa cells were obtained from the American Type Culture Collection ("ATCC") (Manassas VA). Cells were routinely grown in RPMI1640 medium supplemented with 10% fetal bovine serum and 1% glutamax. The extra-cellular domain of IGF-1R was obtained as a recombinant protein from R&D Systems (Minneapolis MN).

Preparation of whole-cell lysates, immunoprecipitation, and Western blot analysis. For qualitative IRS-1 phosphorylation analysis, MCF7 cells in monolayer cultures (about 80% of confluency) were used. After about 20 hours of starvation in serum-free RPMI medium (GibcoBRL), cells were stimulated for 10 min in the same medium containing IGF-I (Peprotech), or IGF-1 plus synthetic peptides (Research Genetics), or neither of above as a negative control. After treatment, cells were rinsed twice with ice-cold PBS containing 0.2 mM PMSF and 1 mM Na₃VO₄ (all from SIGMA). Cells were scraped into the same buffer and pelleted by centrifugation at 200 g for 3 min. Lysis was done in RIPA buffer (0.8766 % NaCl, 0.11 % SDS, 0.5 % deoxycholic acid (all from SIGMA), 1 % Triton X-100, (Boehringer Mannheim)) containing phosphatase inhibitor cocktails 1 and 2 (SIGMA) and protease cocktail inhibitor tablet (Boehringer Mannheim) for 5 min on ice. Cell lysates were cleared by centrifugation for 5 min at 14 000 g and the resulting supernatant was snap-frozen in EtOH-dry ice and stored at - 80° C. The protein concentration was determined using the D_{C} Protein Assay Kit (Bio-Rad Laboratories).

Immunoprecipitations were performed with pre-cleared lysates for 4 hours at 40° C using 0.3 - 0.5 mg of total protein with 1 µg of polyclonal anti-IRS-1 antibody (Upstate Biotechnology), and 25 µL of Protein A/agarose slurry (SIGMA). Agarose beads with immobilized proteins were washed 3 times with IP wash buffer (50 mM Tris pH 7.5 (GibcoBRL), 150 mM NaCl, 1 mM Na₃VO₄, 0.2 mM PMSF). Protein elutions and denaturation were done for 3 min at 95° C in 30 µL of Laemmle sample buffer (Bio-Rad Laboratories) with 0.5 M β-Mercaptoethanol (SIGMA).

Immunoprecipitates were subjected to SDS-PAGE on 4-15 % Tris-HCl Ready Gels and transferred to Trans-Blot Transfer Medium nitrocellulose membranes (both from Bio-Rad Laboratories). Membranes were blocked with PBS-Tween 20 (SIGMA) containing 2 % non-fat milk. For detection of IRS-1 protein, blots were incubated with Anti-IRS-1 antibody (secondary antibody - goat Anti-rabbit IgG, HRP-conjugate). For detection of phosphorylated IRS-1, blots were incubated with monoclonal Anti-Phosphotyrosine (4G10) HRP-conjugated antibody (all antibodies are from Upstate Biotechnology). Blots were exposed to an enhanced chemi-fluorescence substrate (ECL Western Blotting Analysis System, Amersham Pharmacia Biotech). Films were developed and fluorescent signal was visualized for qualitative analysis.

Cell Assays. MCF7 or MiaPaCa cells were plated in 96 well plates at a concentration of 3 x 10³ cells/well in 75 µL of serum-free RPMI-1640 and incubated overnight at 37°C. Dilutions of the peptides were prepared in a separate working plate and 75 µL added to the cells. For antagonist assays, IGF-1 was added to each well at 10 times its ED₅₀ (~50 nM). Plates were incubated for 48-72 hours at 37°C. Viability was measured by the addition of 10 L WST-1/well (Roche) as per the manufacturer's instructions.

Binding Assays. Relative potencies of hot spot peptide ligands (HPs) (or "candidate peptides" or simply "candidates") as compared to IGF-1 were analyzed in a competition system utilizing biotinylated-human IGF-1 (b-hIGF-1) and His-tagged soluble recombinant human IGF-IR (srhlGF-IR-his; R&D systems, Inc., Minneapolis, MN). Detection of the receptor ligand interaction was measured in an amplified luminescent proximity homogeneous assay (ALPHAScreen; BioSignal-Packard, Montreal). The assay was performed in 384-well Nunc™white polystyrene microplates (Nalge Nunc International, Naperville, IL) with a final volume of 40 µL. Final incubation conditions were 1 nM b-hIGF-I, 10 nM srhIGF-IR-his, 0.025 M HEPES (pH 7.4 at 25°C), 0.100 M NaCl, 0.1 % BSA (Cohn Fraction V; Sigma Chemical Co., St. Louis, MO), 10 µg/mL nickel conjugated acceptor beads, and 10 µg/mL streptavidin conjugated donor beads. For the first step of the assay hIGF-I (PeproTech, Inc., Rocky Hill, NJ), b-hIGF-I (see below), and candidates were incubated for 2 hours at room temperature. Each concentration of competitor was assayed in duplicate. Non-specific binding was determined in the presence of 3 x 10⁻⁵ M hIGF-1. The second step of the assay was to add acceptor beads and the incubation continued for 0.5 hr. The final step was to add donor beads and the incubation continued for an additional 1hr. At the end of the incubation period the fluorescence signal at 520 nm was read on a Fusion-α HT plate reader (Packard BioScience Company, Meriden, CT). Primary data were background corrected, normalized to buffer controls and then expressed as % Specific Binding. The data were fit to a four-parameter non-linear regression analysis (y = min + (max-min)/(1+10^((logIC₅₀-x)*Hillslope)) ), which was used to determine IC₅₀ values. The Z'-factor for this assay is greater than 0.7 (Z' = 1-(3σ++3σ-)/|µ+-µ-|) and the signal-to-background (S/B) ratio was between 40 and 70.

Human IGF-1 was biotinylated on free amino groups using Pierce EZ-Link™ Sulfo-NHS-LC-Biotinylation Kit (PN #21430, Pierce, Rockford, IL). Human IGF-1, at 2 mg/mL in PBS, pH 7.2, was incubated at room temperature for 30 minutes with a 20-fold excess of sulfo-NHS-LC-biotin over theoretical total free amino groups. Unreacted biotins were removed by extensive dialysis (Pierce Slide-A-Lyzer® Dialysis Cassettes) against PBS, and degree of conjugation was determined by HABA (2-(4'-hydroxyazobenzene) benzoic acid) assay (Pierce product literature #21430). The number of biotins/hIGF-1 varied between 3 and 5.

### Results of Additional Studies

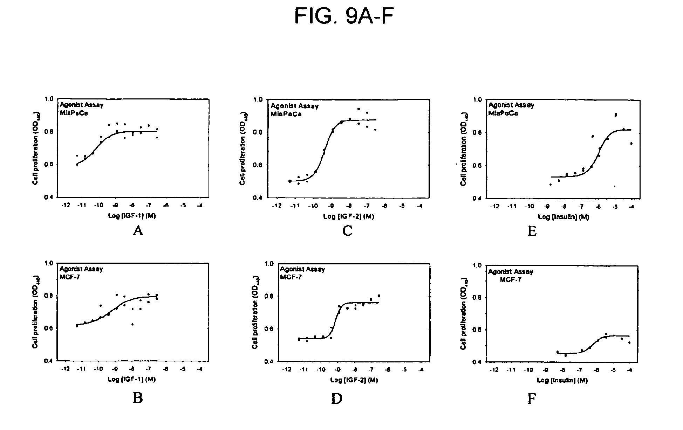

The dose related increase in cell proliferation of MiaPaCa and MCF-7 cell-based models of cancer to IGF-1, IGF-2, and Insulin, are shown in Figure 9 (9A-9F). Cells were treated for 72 h with IGF-1, IGF-2, or insulin, as described above. Specifically, Figure 9A, B shows the effects of IGF-1 on the growth of MiaPaCa (human pancreatic cancer) and MCF7 (human breast cancer) cells, respectively; Figure 9C, D shows the effects of IGF-2 on MiaPaCa and MCF7, respectively; Figure 9E, F shows the effects of insulin on MiaPaCa and MCF7, respectively. These data indicate that both MiaPaCa and MCF7 cells are responsive to the effects of all three hormones and can be used as models to demonstrate the biological effects of the IGF-1R antagonists.

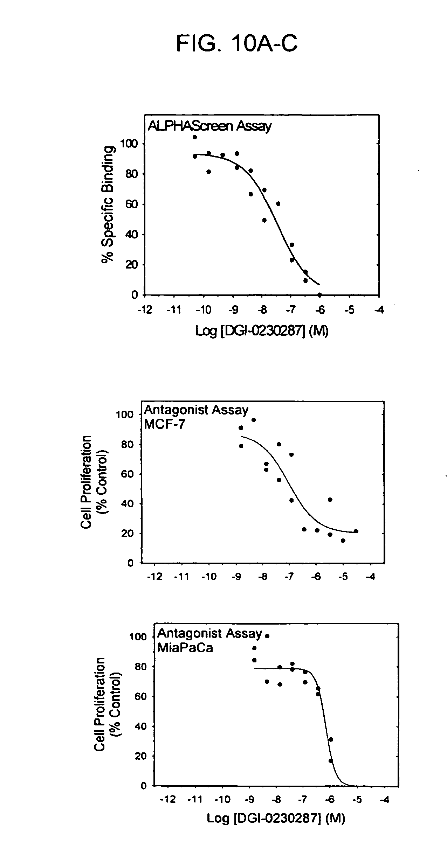

As shown in Figure 10, binding and cell proliferation assays reveal that F250 competes IGF-1 binding and antagonizes its activity in cell-based cancer models. Specifically, Figure 10A reflects inhibition of IGF-1 binding as a function of F250 concentration. Competition experiments were carried out using the ALPHAScreen assay format as described above (data are presented as percent inhibition and to determine binding kinetic parameters data were fit to a four-parameter logistic equation).

Figure 10B reflects antagonism of IGF-1 activity in MCF-7 cells by F250. The cells were treated as described in the materials and methods section. F250 was added to cells in the presence of 5 x10⁻⁸ M IGF-1 and incubated for 72h, then cell number was determined. The data are presented as percent inhibition and to determine binding kinetic parameters data were fit to a four-parameter logistic equation.

Figure 10C reflects antagonism of IGF-1 activity in MiaPaCa cells by F250. The MiaPaCa cells were treated as described in the materials and methods section; F250 was added to cells in the presence of 5 x 10⁻⁸ MIGF-1 and incubated for 72h; then cell number was determined. This data is presented as percent inhibition and to determine binding kinetic parameters data were fit to a four-parameter logistic equation.

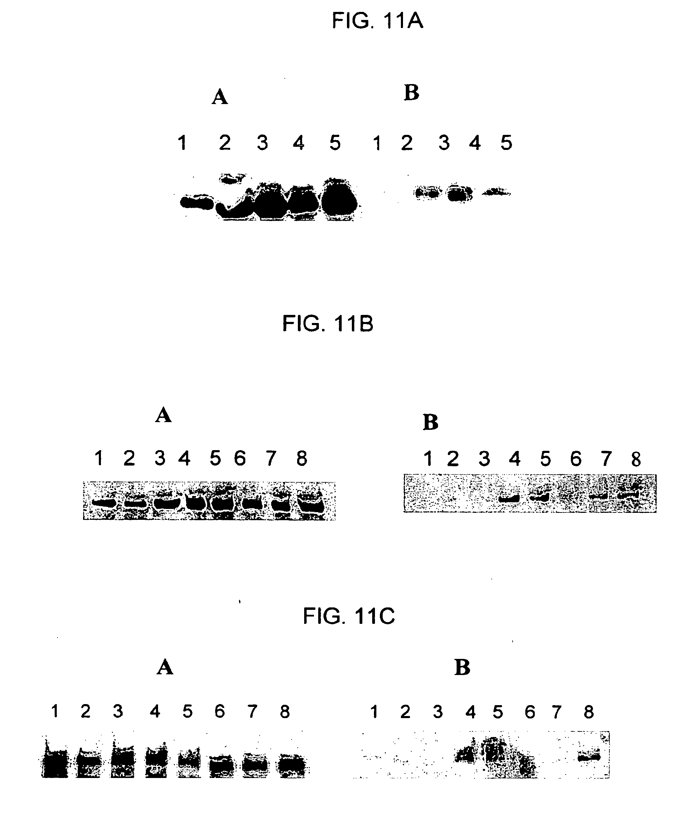

The results of the experiments presented in Figure 11 demonstrate that IGF-1 stimulates phosphorylation in cancer cell models, which can be blocked or reduced by candidate peptides of the invention. Specifically, the data shown in Figure 11a reflect that IGF-1 stimulates a transient phosphorylation of IRS-1 in MCF7 cells. These cells were stimulated with 10 nM IGF-1 for 0, 2,10, 30, 60 min. 0.5 mg total protein was immunoprecipitated for each analysis as described above. Part A of the figure is a Western blot analysis of endogenous IRS-1 and part (B) is a Western blot analysis of phosphorylated IRS-1 [(1) no addition; (2) 2 minutes; (3) 10 minutes; (4) 30 minutes; (5) 60 minutes]. The results shown in Figure 11B reflect that phosphorylation of IRS-1 in MCF7 cells induced by IGF-1 is dose-dependant. These cells were exposed for 10 min to increasing concentrations of IGF-1. 0.5 mg total protein was immunoprecipitated and for each analysis. Stimulation by 0.50 nM IGF-1 resulted in a sub-maximal level of phosphorylation that could consistently be visualized in Western blot analysis: (A). Western blot analysis of endogenous IRS-1; (B). Western blot analysis of phosphorylated IRS-1 [(1) no addition; (2) 0.05 nM IGF-1; (3) 0.1 nM IGF-1; (4) 5 nM IGF-1; (5) 1 nM IGF-1; (6) 0.5 nM IGF-1; (7) 10 nM IGF-1; (8) 50 nM IGF-1]. Figure 11C illustrates a blockade of IGF-1-induced phosphorylation of IRS-1 in MCF cells by candidate peptides (HPs) RP6KK and F250. Cells were stimulated for 10 minutes with 0.5 nM IGF-1 in the presence or absence of peptides (30 µM); 0.3 mg total protein was immunoprecipitated for each analysis. IGF-1-induced phosphorylation of IRS-1 was inhibited by RP6KK and F250 peptides and not inhibited by two irrelevant (control) peptides KCB7 and DGI3-D8. Specifically, part (A) of Figure 11C is a Western blot analysis of expressed IRS-1 and part (B) is a Western blot analysis of phosphorylated IRS-1 [(1) No addition; (2) Irrelevant Peptide 1; (3) Irrelevant Peptide 2; (4) IGF-1 + Irrelevant Peptide 1; (5) IGF-1 + Irrelevant Peptide 2; (6) IGF-1 + RP6KK; (7) IGF-1 +F250; (8) IGF-1].

### EXAMPLE 8

### Additional Binding and Antagonism Assays

Additional binding and antagonism assays using candidate peptides were performed. For antagonism assays the following method was generally used - cells MCF7 cells were "starved" (cultured in 0.1% FBS) and plated at 103 cells/ well. IGF-1 ED₈₀ (1 nM)) and candidate peptide were added to wells simultaneously. The dose response of peptides was evaluated (analysis started at 30 µM). Assays were performed for 72 hrs. Cell viability was determined by luminescence assay and IC₅₀ was calculated using standard techniques.

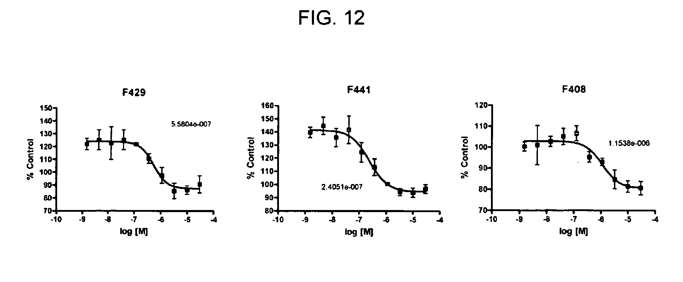

The results of these studies are reflected in the following tables and Figure 12 (which exhibits the antagonistic effect of peptides F429, F441, and F408).

**Table 9 - Binding and Antagonism Properties of Select Peptides**

| ID | Sequence | Kd | MCF7 IC₅₀ | Activity |
|---|---|---|---|---|
| F408 | NFYGCLLDLSLGVPSFGWRRRCITA | 1.6 x 10⁻⁹ | 1.3 x 10⁻⁶ | Antagonist |
| | | | 1.15 x 10⁻⁶ | |
| F429 | DFYGCLLDLSLGVPSLGWRRRCITA | 6.3 x 10⁻¹⁰ | 6.85 x 10⁻⁸ | Antagonist |
| | | | 5.58 x 10⁻⁷ | |
| | | | 1.2 x 10⁻⁸ | |
| F441 | SFYDCLLDLSIGGPSSDWRRRCITA | | 2.4 x 10⁻⁷ | Antagonist |
| | | | 1.8 x 10⁻⁷ | |

Table 10 - Variants of F 138

Variants of peptide F138 were generated and selected based on having improved properties in terms of IGF-1R binding and antagonism as reflected in the following table:

| ID | Sequence | Binding | Cell |
|---|---|---|---|
| | | (nM) | (nM) |
| F429 | DFYGCLLDLSLGVPSLGWRRRCITA | 0.63 | 12-558 |
| F441 | SFYDCLLDLSIGGPSSDWRRRCITA | | 180-240 |
| F408 | NFYGCLLDLSLGVPSFGWRRRCITA | 1.6 | ~1225 |
| F138 | QFYGCLLDLSLGVPSFGWRRRCIT | 6 | 3000 |

### Table 11 - Additional Binding and Cell Data for Select Candidate Peptides

Other peptides were similarly analyzed and determined to be antagonists or candidates:

| ID | Sequence | Binding (nM) | Cell |
|---|---|---|---|
| | | AlphaScreen | (nM) |
| F293 | SFYSCLESLVQGPAEKSRGQWEGCRK | 9 | 3 |
| F250 | SFYSCLESLVNGPAEKSRGQWDGCRK | 78 | 5-10,000 |
| F113 | SFYSCLESL VNGGAERSDGQWEGCR | 560 | 1000 |
| RP6 | TFYSCLASLLTGTPQPNRGPWERCR | 2700 | 9000 |

**Table 12 - Additional Binding and Antagonism Data for Select Peptides**

| ID No | Sequence | IG-1 Displacement | AlphaScreen | Antagonism |
|---|---|---|---|---|
| | | Binding Affinity | Binding Affinity | MCF7 Cells |
| | | IC50 = M | IC50 = M | IC50 = M |
| IGF-1 | | | 2.4E-10 | 2.3E-10 (ED50) |
| F249 | SFYSCLESL VNGPAEKSRGQWDGCR | 1.9E-07 | 2.8E-08 | 7.9E-06 |
| F255 | EGSLDESFYDWFERQLG-POX2-GYSWLRDVLMEKQAOLKREGSVGRQE | 1.3E-05 | 1.6E-06 | 1.8E-05 |
| F263 | VQDDCRGRPCGDADSFYEWFDQQAS | 2.5E-08 | 1.4E-08 | 5.6E-06 |
| F264 | RQWDCRGRPCGDAESFYEWFDQQRS | 4.4E-08 | 1.0E-08 | 3.6E-06 |
| F267 | ETGRECWGRPCGEADSFYDWFVQQGSE | 1.1E-06 | 1.9E-06 | agonist |
| F268 | EIQRDCQGRPCGDAANFYDWFVQQDSE | 3.7E-07 | 2.3E-06 | 2.7E-05 |
| F269 | EVDRDCQARPCGDAANFYDWFGQQGTE | 8.9E-07 | 5.1E-07 | 2.1E-05 |
| F270 | ESYGDCRDRPCGDAPNFYDWFVQQASE | 7.0E-08 | 3.6E-08 | 1.4E-05 |
| F271 | RGNVGGGSLDESFYEWFERQLGR | 1.7E-06 | 5.6E-06 | |
| F272 | TLNPRGPWEGSRGSMDDSFYRWFERQLE | 1.5E-06 | 5.0E-06 | 4.8E-09 |
| F273 | TGAPQPNRGPLDRCRGSLDECFYGWFERQLL | 7.6E-07 | 5.0E-07 | 1.0E-09 |
| F274 | TFYSGPVSLLTGTPRTNRSAWERGRGSLDDSFYDWFERQLSR | 1.4E-06 | 2.0E-06 | |
| F275 | GGVGSGSRDESFYDWFERQLA | 3.0E-06 | 4.3E-06 | 6.9E-07 |
| F276 | GSGGYASRDESFYEWFERQLA | 1.6E-06 | 5.1E-06 | |
| F287 | Ac-VGRASGFPENFYDWFGRQLSLQSGEQRR | 7.6E-06 | 8.4E-06 | |
| F288 | Ac-ESDVWAQPQRRNDWPGYHWLSR | >2e-5 | 3.2E-07 | |
| F292 | SFYSCLESLVTGPAEKSRGQWEGCRK | 1.1E-07 | 9.0E-09 | 1.1E-09 |
| F293 | SFYSCLESLVQGPAEKSRGQWEGCRK | 8.9E-08 | 9.2E-09 | 3.1E-09 |
| F296 | SFYSCLESLVNAPAEKSRGQWEGCRK | 5.7E-08 | 7.5E-09 | 3.1E-09 |
| F297 | SFYSCLESLVNaPAEKSRGQWEGCRK | 5.1E-08 | 8.7E-09 | 4.4E-09 |
| F298 | SFYSCLESLVNAPAEKSRGQWDACRK | 6.4E-08 | 5.2E-09 | 3.4E-09 |

### EXAMPLE 9

In this study, the stability of Test Article F429 in CD-1 mouse plasma was evaluated following incubation at 37°C for twelve sampling time points. Plasma levels of Test Article F429 were determined by LC-MS/MS, and the results were processed and interpreted (half-life).

### Materials

Test Article F429 was supplied as an aqueous solution and stored at -80°C before it was used. Test Article F429 is a peptide with a molecular weight of 2812.34. This peptide contains 25 amino acids, and its sequence is [H]DFYGCLLDLSLGVPSLGWRRRCITA[OH]. The CD-1 mouse plasma was purchased from Bioreclamation Inc. (Hicksville, NY) and stored at 2-8°C before it was used.

### Test Article Preparation

Test Article F429 solution was prepared in aqueous solution at a concentration of 2.0 mg/mL and shipped to Absorption Systems frozen at-80°C.

### Study Design

The study design is shown in Table 13 below. A 150-µL aliquot of Test Article F429 (2.0 mg/mL) was added into 2850 µL of CD-1 mouse plasma, for a final concentration of Test Article F429 at 100 µg/mL. The sample was mixed and immediately put into a 37°C reciprocal shaking water bath (Precision, Winchester, VA). Triplicate 75-µ aliquots were sampled at twelve sample time points (blank test article, 1, 2, 5, 10, 15, 20, 30, 60 min and 2, 4 and 24 hrs). The aliquots were immediately frozen at -80°C.

Table 13 - Study Design

| Treatment Group | Test Article | Route | N=3 per time point | Stock Solution (mg/mL) | Final F429 Cone. (µg/mL) | Vehicle* | Sampling |
|---|---|---|---|---|---|---|---|
| | | | | | | | Time Points |
| 1 | F429 | In Vitro | 36 | 2.0 | 100 | 100% SWFI | Predose (0), 1, 2. 5, 10. 15, 20, 30 min. 1, 2, 4 & 24 hours incubation |

| | | | | | | | |
|---|---|---|---|---|---|---|---|
| * SWFI - Sterile water for injection. | | | | | | | |

### Analysis of Plasma Samples-MS Infusion to Determine Ionization Type (Positive or Negative ESI/APCI)

A stock solution of 1.0 mg/mL F429 was prepared in DMSO. Working solutions of 100 µg/mL and 10 µg/mL were prepared from the 1.0 mg/mL stock solution.

F429 (100 µg/mL) in 3:1 (v/v) acetonitrile:H₂O was used for test article LC-MS/MS method optimization. Positive MS polarity and electrospray ionization (ESI) were applied for F429 with its transition of m/z 938.2/235.10.

Test Article F429 was spiked into blank CD-1 mouse plasma. The spiked samples contained 100, 75, 50, 37.5, 25, 10, 5, 2.5, and 0 µg/mL (blank) of F429.

### Sample Preparation for Solid Phase Extraction Evaluation

All mouse plasma samples were diluted two-fold with in-house blank CD-1 mouse plasma. Then, aliquots (50 µL) of mouse plasma and spiked F429 mouse plasma samples were added into 200 µL of water, then combined with 250 µL of pH 7.4, 10 mM phosphate buffer solution and mixed well. The final volume of the prepared solution was 500 µL, which was ready for solid phase extraction sample preparation.

### Plasma Analysis - Standards and Unknowns

The Strata X 96-well plates solid phase extraction cartridge (8E-S100-TGB, Phenomenex) was placed on a vacuum manifold with the vacuum set to 5 mm Hg for sample preparation. The plate was first conditioned with 1.0 mL of acetonitrile following the equilibration with 1.0 mL of water. Then 500 µL of samples were loaded onto the plate and later were washed twice with 0.5 mL of 5% acetonitrile in water. The receiver was replaced with a 96-deep well collection plate. The cartridge was then eluted with 80% acetonitrile in water containing 1% ammonium hydroxide twice. A 100-µL aliquot of each of the eluted samples was transferred to vials for analysis by LC-MS/MS. Quantification of the test article, F429, in mouse plasma was performed against calibration curves generated by spiking the test article into blank mouse plasma (100, 75, 50, 37.5, 25, 10, 5, and 2.5 µg/mL final concentrations).

The HPLC conditions for test article F429 is provided in Table 14 and the gradient program is provided in Table 15 below.

**Table 14**

| | |
|---|---|
| Instrument: | Perkin Elmer Series 200 autosampler and MicroPumps |
| Column: | Synergi Polar-RP C18 80A, 50 x 2.1 mm. 4 µ |
| Aqueous Reservoir (A): | 0.1 % formic acid in water |
| Organic Reservoir (B): | 0.1% formic acid in acetonitrile |
| Flow Rate: | 300 µL/min |
| Inj. Vol.: | 10µL |
| Run Time: | 4.5 min (F429 at 3.12 min) |
| Autosampler Wash: | 1:1:1 water:acetonitrile:isopropanol with 0.2% formic acid |

**Table 15**

| Time (min) | Grad. Curve | %A | % B | Waste | MS |
|---|---|---|---|---|---|
| 0 | 1 | 80 | 20 | close | |
| 1.5 | 1 | 80 | 20 | | close |
| 3.0 | 1 | 0 | 100 | | |
| 3.1 | 1 | 80 | 20 | | |
| 4.0 | 1 | 80 | 20 | close | |

The Mass Spectrometer Conditions for Test Article F429 is provided in Table 16 and the Voltages and Ions Monitored data are presented in Table 17 below.

**Table 16**

| | |
|---|---|
| Instrument: | PE Sciex AP14000 |
| Interface: | Electrospray ("Turbo Ion Spray") |
| Mode: | Multiple Reaction Monitoring (MRM) |
| Gases (arbitrary units): | CUR 20, CAD 12, NEB 12 |
| TEM: | 500°C |

**Table 17**

| Analyte | Polarity | Precursor Ion | Product Ion | Ion Spray Voltage | Declustering Potential | Entrance Potential | Collision Energy | Collision Cell Exit Potential |
|---|---|---|---|---|---|---|---|---|
| F429 | Positive | 938.20 | 235.10 | 5000 | 156 | 10 | 85 | 32 |

### Analytical Method Evaluation

An eight-point standard curve in triplicate at each concentration level was interspersed with the samples during each run. At least the two closest determinations for each concentration were used to generate the calibration curve. The acceptance criteria were that five out of eight standards must be within ±30% for the stability plasma sample analysis. Results of the calibration curve are in Table 18.

### Data Analysis

The plasma stability of the test article, F429, was evaluated after incubation in CD-1 mouse plasma.

### RESULTS-Plasma Sample Analysis

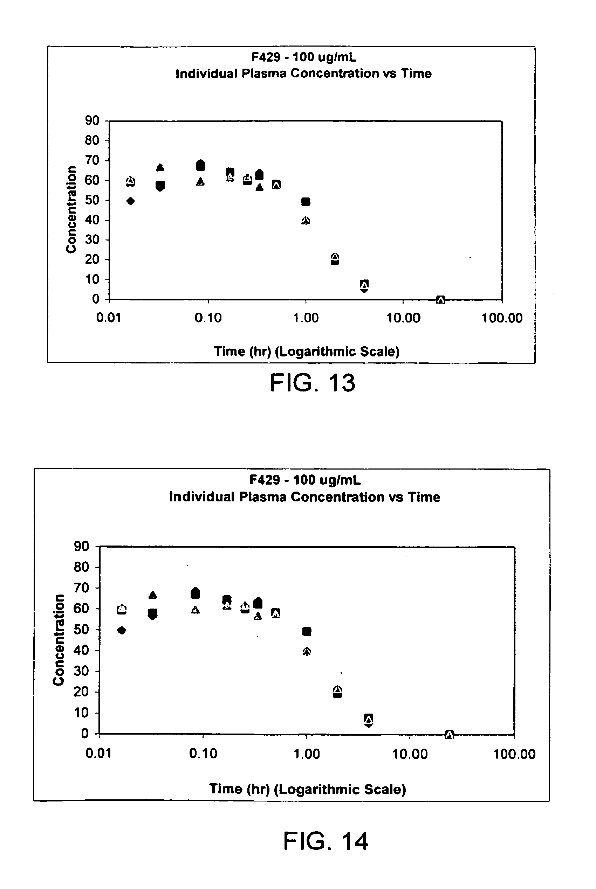

Individual rat plasma concentrations of Test Article F429 vs. time data, as well as mean data for each dosing group, are shown in Tables 19 and 20. The plots of the individual and mean plasma concentration of F429 versus time following the incubation of 100 µg/mL F429 are shown in Figure 13 and Figure 14. All samples that were below the limit of quantification were assigned a value of zero. All data are expressed as µg/mL of the F429 free drug.

### Data Analysis and Half-life

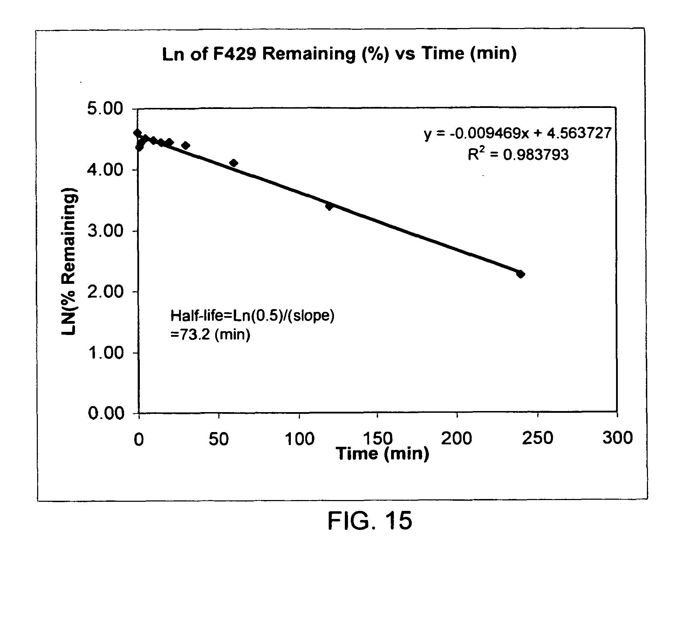

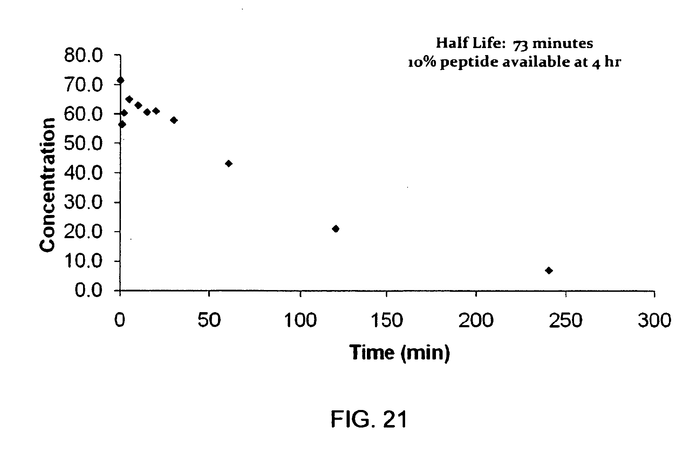

The remaining percentage of Test Article F429 in different time points from time zero are listed in Table 21. The logarithm of the remaining Test Article F429 percentage is shown in Figure 15. The half-life of F429 was calculated to be 73.2 min. (Figure 21).

**Table 18**

| F429 Standard | Nominal Conc.(ug/mL) | Calculated Conc.(ug/mL) | Accuracy (%) | CV(%) |
|---|---|---|---|---|
| STD8 | 2.50 | 2.43 | 97.4 | 4.16 |
| | | 2.28 | 91.1 | |
| | | 2.48 | 99.2 | |
| STD7 | 5.00 | 6.18 | 124 | 13.2 |
| | | 4.89 | 97.8 | |
| | | 5.28 | 106 | |
| STD6 | 10.0 | 9.95 | 99.5 | 2.25 |
| | | 10.2 | 102 | |
| | | 9.75 | 97.5 | |
| STD5 | 25.0 | 26.3 | 105 | 5.97 |
| | | 24.0 | 95.8 | |
| | | 23.5 | 94.1 | |
| STD4 | 37.5 | 39.1 | 104 | 4:28 |
| | | 35.9 | 95.8 | |
| | | 37.3 | 99.5 | |
| STD3 | 50.0 | 53.9 | 108 | 8.85 |
| | | 48.7 | 97.4 | |
| | | 45.1 | 90.2 | |
| STD2 | 75.0 | 75.4 | 101 | 8.48 |
| | | 74.8 | 99.8 | |
| | | 64.1 | 85.5 | |
| STD1 | 100 | 113 | 113 | 9.40 |
| | | 104 | 104 | |
| | | 94.2 | 94.2 | |

**Table 19**

| Incubation Time (min) | Incubation Time (hr) | Sampe (n=3) | Calcutated Conc. | Mean (ug/mL) | SD | CV (%) |
|---|---|---|---|---|---|---|
| 0 | 0.00 | 1 | 65.5 | 71.5 | 5.68 | 7.95 |
| | | 2 | 72.2 | | | |
| | | 3 | 76.8 | | | |
| 1 | 0.02 | 1 | 49.7 | 56.5 | 5.95 | 10.5 |
| | | 2 | 59.3 | | | |
| | | 3 | 60.6 | | | |
| 2 | 0.03 | 1 | 56.6 | 60.4 | 5.55 | 9.19 |
| | | 2 | 57.9 | | | |
| | | 3 | 66.8 | | | |
| 5 | 0.08 | 1 | 68.6 | 65.1 | 4.74 | 7.29 |
| | | 2 | 67.0 | | | |
| | | 3 | 59.7 | | | |
| 10 | 0.17 | 1 | 63.0 | 63.0 | 1.30 | 2.06 |
| | | 2 | 64.3 | | | |
| | | 3 | 61.7 | | | |
| 15 | 0.25 | 1 | 60.4 | 60.7 | 0.95 | 1.56 |
| | | 2 | 60.0 | | | |
| | | 3 | 61.8 | | | |
| 20 | 0.33 | 1 | 63.9 | 61.1 | 3.69 | 6.04 |
| | | 2 | 62.4 | | | |
| | | 3 | 56.9 | | | |
| 30 | 0.50 | 1 | 58.0 | 58.0 | 0.30 | 0.52 |
| | | 2 | 58.3 | | | |
| | | 3 | 57.7 | | | |
| 60 | 1.00 | 1 | 39.9 | 43.2 | 5.40 | 12.5 |
| | | 2 | 49.4 | | | |
| | | 3 | 40.2 | | | |
| 120 | 2.00 | 1 | 21.7 | 21.1 | 1.27 | 6.05 |
| | | 2 | 19.6 | | | |
| | | 3 | 21.9 | | | |
| 240 | 4.00 | 1 | 5.41 | 6.90 | 1.34 | 19.5 |
| | | 2 | 8.02 | | | |
| | | 3 | 7.26 | | | |
| 1440 | 24.00 | 1 | blq | 0.00 | 0.00 | 0.00 |
| | | 2 | blq | | | |
| | | 3 | blq | | | |

| | | | | | | |
|---|---|---|---|---|---|---|
| * blq=blow limit of quantitation | | | | | | |

**TABLE 20**

| Peak Name: F429 | | | | | | | | | | |
|---|---|---|---|---|---|---|---|---|---|---|
| No Internal Standard | | | | | | | | | | |
| Q1/Q3 Masses: 938 20/235 10 amu | | | | | | | | | | |
| | | | | | | | | | | |
| Fit | Quadratic Weighting 1 /(x *x) | | | | | | | | | |
| a0 | 16.1 | | | | | | | | | |
| a1 | 255 | | | | | | | | | |
| a2 | 0.97 | | | | | | | | | |
| Correlation coe | 0 9945 | | | | | | | | | |
| | | | | | | | | | | |

| Sample Name | File Type | File Name | Dilution Factor | Area | Conc. (ug/mL) | IS Area | Use Record | Record Modified | Calc. Conc. (ug/mL) | Accuracy (%) |
|---|---|---|---|---|---|---|---|---|---|---|
| mp | Unknown | December 09 2005 | 1 | 0.00E+00 | N/A | N/A | | 0 | No Peak | N/A |
| mp | Unknown | December 09 2005 | 1 | 0.00E+00 | N/A | N/A | | 0 | No Peak | N/A |
| mp | Unknown | December 09 2005 | 1 | 0.00E+00 | N/A | N/A | | 0 | No Peak | N/A |
| b | Unknown | December 09 2005 | 1 | 0.00E+00 | N/A | N/A | | 0 | No Peak | N/A |
| std-25 ugml | Standard | December 09 2005 | 1 | 6.43E+02 | 2.5 | N/A | 1 | 0 | 2.43 | 97.4 |
| stud-5 ugml | Standard | December 09 2005 | 1 | 1.63E+03 | 5 | N/A | 1 | 0 | 6.18 | 124 |
| std-10 ugml | Standard | December 09 2005 | 1 | 2.65E+03 | 10 | N/A | 1 | 0 | 9.95 | 99.5 |
| std-25 ugml | Standard | December 09 2005 | 1 | 7.40E+03 | 25 | N/A | 1 | 0 | 26.3 | 105 |
| std-37.5 ugml | Standard | December 09 2005 | 1 | 1.15E+04 | 37.5 | N/A | 1 | 0 | 39.1 | 104 |
| std-50 ugml | Standard | December 09 2005 | 1 | 1.66E+04 | 50 | N/A | 1 | 0 | 53.9 | 108 |
| std-75 ugml | Standard | December 09 2005 | 1 | 2.48E+04 | 75 | N/A | 1 | 0 | 75.4 | 101 |
| std-100 ugml | Standard | December 09 2005 | 1 | 4.12E+04 | 100 | N/A | 1 | 0 | 113 | 113 |
| mp | Unknown | December 09 2005 | 1 | 0.00E+00 | N/A | N/A | | 0 | No Peak | N/A |
| 0 min-1 | Unknown | December 09 2005 | 2 | 9.41 E+03 | N/A | N/A | | 0 | 65.5 | N/A |
| 0 min-2 | Unknown | December 09 2005 | 2 | 1.05E+04 | N/A | N/A | | 0 | 72.2 | N/A |
| 0 min-3 | Unknown | December 09 2005 | 2 | 1.13E+04 | N/A | N/A | | 0 | 76.8 | N/A |
| 24 hr-1 | Unknown | December 09 2005 | 2 | 3 99E+02 | N/A | N/A | | 0 | 2.98 | N/A |
| 24 hr-2 | Unknown | December 09 2005 | 2 | 0.00E+00 | N/A | N/A | | 0 | No Peak | N/A |
| 24 hr-3 | Unknown | December 09 2005 | 2 | 0.00E+00 | N/A | N/A | | 0 | No Peak | N/A |
| 4 hr-1 | Unknown | December 09 2005 | 2 | 7.14E+02 | N/A | N/A | | 0 | 5.41 | N/A |
| 4 hr-2 | Unknown | December 09 2005 | 2 | 1.06E+03 | N/A | N/A | | 0 | 8.02 | N/A |
| 4 hr-3 | Unknown | December 09 2005 | 2 | 9.55E+02 | N/A | N/A | | 0 | 7.26 | N/A |
| 2 hr-1 | Unknown | December 09 2005 | 2 | 2.90E+03 | N/A | N/A | | 0 | 21.7 | N/A |
| 2 hr-2 | Unknown | December 09 2005 | 2 | 2.61E+03 | N/A | N/A | | 0 | 19.6 | N/A |
| 2 hr-3 | Unknown | December 09 2005 | 2 | 2.92E+03 | N/A | N/A | | 0 | 21.9 | N/A |
| 1 hr-1 | Unknown | December 09 2005 | 2 | 5.49E+03 | N/A | N/A | | 0 | 39.9 | N/A |
| 1 hr-2 | Unknown | December 09 2005 | 2 | 6.91E+03 | N/A | N/A | | 0 | 49.4 | N/A |
| 1 hr-3 | Unknown | December 09 2005 | 2 | 5.54E+03 | N/A | N/A | | 0 | 40.2 | N/A |
| 30min-1 | Unknown | December 09 2005 | 2 | 8.24E+03 | N/A | N/A | | 0 | 58 | N/A |
| 30min-2 | Unknown | December 09 2005 | 2 | 8.27E+03 | N/A | N/A | | 0 | 58.3 | N/A |
| 30min-3 | Unknown | December 09 2005 | 2 | 8.18E+03 | N/A | N/A | | 0 | 57.7 | N/A |
| mp | Unknown | December 09 2005 | 1 | 0.00E+00 | N/A | N/A | | 0 | No Peak | N/A |
| mp | Unknown | December 09 2005 | 1 | 0.00E+00 | N/A | N/A | | 0 | No Peak | N/A |
| b | Unknown | December 09 2005 | 1 | 0.00E+00 | N/A | N/A | | 0 | No Peak | N/A |
| std-2.5 ugml | Standard | December 09 2005 | 1 | 6.03E+02 | 2.5 | N/A | 1 | 0 | 2.28 | 91.1 |
| std-5 ugml | Standard | December 09 2005 | 1 | 1.29E+03 | 5 | N/A | 1 | 0 | 4.89 | 97.8 |
| std-10 ugml | Standard | December 09 2005 | 1 | 2.71E+03 | 10 | N/A | 1 | 0 | 10.2 | 102 |
| std-25 ugml | Standard | December 09 2005 | 1 | 6.69E+03 | 25 | N/A | 1 | 0 | 24 | 95.8 |
| std-37.5 ugml | Standard | December 09 2005 | 1 | 1.04E+04 | 37.5 | N/A | 1 | 0 | 35.9 | 95.8 |
| std-50 ugml | Standard | December 09 2005 | 1 | 1.47E+04 | 50 | N/A | 1 | 0 | 48.7 | 97.4 |
| std-75 ugml | Standard | December 09 2005 | 1 | 2.45E+04 | 75 | N/A | 1 | 0 | 74.8 | 99.8 |
| std-100 ugml | Standard | December 09 2005 | 1 | 3.70E+04 | 100 | N/A | 1 | 0 | 104 | 104 |
| mp | Unknown | December 09 2005 | 1 | 000E+00 | N/A | N/A | | 0 | No Peak | N/A |
| 20min-1 | Unknown | December 09 2005 | 2 | 9.17E+03 | N/A | N/A | | 0 | 63.9 | N/A |
| 20min-2 | Unknown | December 09 2005 | 2 | 8 93E+03 | N/A | N/A | | 0 | 62.4 | N/A |
| 20min-3 | Unknown | December 09 2005 | 2 | 8.07E+03 | N/A | N/A | | 0 | 56.9 | N/A |
| 15min-1 | Unknown | December 09 2005 | 2 | 8.61E+03 | N/A | N/A | | 0 | 60.4 | N/A |
| 15min-2 | Unknown | December 09 2005 | 2 | 8.54E+03 | N/A | N/A | | 0 | 60 | N/A |
| 15min-3 | Unknown | December 09 2005 | 2 | 8.83E+03 | N/A | N/A | | 0 | 61.8 | N/A |
| 10min-1 | Unknown | December 09 2005 | 2 | 9.02E+03 | N/A | N/A | | 0 | 63 | N/A |
| 10min-2 | Unknown | December 09 2005 | 2 | 9.23E+03 | N/A | N/A | | 0 | 64.3 | N/A |
| 10min-3 | Unknown | December 09 2005 | 2 | 8.81E+03 | N/A | N/A | | 0 | 61.7 | N/A |
| 5min-1 | Unknown | December 09 2005 | 2 | 9.92E+03 | N/A | N/A | | 0 | 68.6 | N/A |
| 5min-2 | Unknown | December 09 2005 | 2 | 9.65E+03 | N/A | N/A | | 0 | 67 | N/A |
| 5min-3 | Unknown | December 09 2005 | 2 | 8.51E+03 | N/A | N/A | | 0 | 59.7 | N/A |
| 2min-1 | Unknown | December 09 2005 | 2 | 8.02E+03 | N/A | N/A | | 0 | 56.6 | N/A |
| 2min-2 | Unknown | December 09 2005 | 2 | 8.22E+03 | N/A | N/A | | 0 | 57.9 | N/A |
| 2min-3 | Unknown | December 09 2005 | 2 | 9.62E+03 | N/A | N/A | | 0 | 66.8 | N/A |
| 1min-1 | Unknown | December 09 2005 | 2 | 6.96E+03 | N/A | N/A | | 0 | 49.7 | N/A |
| 1min-1 | Unknown | December 09 2005 | 2 | 844E+03 | N/A | N/A | | 0 | 59.3 | N/A |
| 1min-1 | Unknown | December 09 2005 | 2 | 8.64E+-3 | N/A | N/A | | 0 | 60.6 | N/A |
| mp | Unknown | December 09 2005 | 1 | 0.00E+00 | N/A | N/A | | 0 | No Peak | N/A |
| mp | Unknown | December 09 2005 | 1 | 0.00E+00 | N/A | N/A | | 0 | No Peak | N/A |
| blank 1-1 | Unknown | December 09 2005 | 2 | 0.00E+00 | N/A | N/A | | 0 | No Peak | N/A |
| blank 1-2 | Unknown | December 09 2005 | 2 | 0.00E+00 | N/A | N/A | | 0 | No Peak | N/A |
| blank 1-3 | Unknown | December 09 2005 | 2 | 0.00E+00 | N/A | N/A | | 0 | No Peak | N/A |
| b | Unknown | December 09 2005 | 1 | 0.00E+00 | N/A | N/A | | 0 | No Peak | N/A |
| std-2.5 ugml | Standard | December 09 2005 | 1 | 6.55E+02 | 2.5 | N/A | 1 | 0 | 2.48 | 99.2 |
| std-5 ugml | Standard | December 09 2005 | 1 | 1.39E+03 | 5 | N/A | 1 | 0 | 5.28 | 106 |
| std-37.5 ugml | Standard | December 09 2005 | 1 | 1.09E+04 | 37.5 | N/A | 1 | 0 | 37.3 | 99.5 |
| std-10 ugml | Standard | December 09 2005 | 1 | 2.60E+03 | 10 | N/A | 1 | 0 | 9.75 | 97.5 |
| std-25 ugml | Standard | December 09 2005 | 1 | 6.56E+03 | 25 | N/A | 1 | 0 | 23.5 | 94.1 |
| std-50 ugml | Standard | December 09 2005 | 1 | 1.35E+04 | 50 | N/A | 1 | 0 | 45.1 | 90.2 |
| std-75 ugml | Standard | December 09 2005 | 1 | 2.04E+04 | 75 | N/A | 1 | 0 | 64.1 | 85.5 |
| std-100 ugml | Standard | December 09 2005 | 1 | 3.27E+04 | 100 | N/A | 1 | 0 | 94.2 | 94.2 |
| mp | Unknown | December 09 2005 | 1 | 0.00E+00 | N/A | N/A | | 0 | No Peak | N/A |
| mp | Unknown | December 09 2005 | 1 | 0.00E+00 | N/A | N/A | | 0 | No Peak | N/A |

**TABLE 21**

| **Incubation Time (min)** | **Sampe (n=3)** | **Calculated Conc.** | **Mean (ug/mL)** | **Remaining (%)** | **Ln** |
|---|---|---|---|---|---|
| 0 | 1 | 65.5 | 71.5 | 100 | 4.61 |
| | 2 | 72.2 | | | |
| | 3 | 76.8 | | | |
| 1 | 1 | 49.7 | 56.5 | 79.1 | 4.37 |
| | 2 | 59.3 | | | |
| | 3 | 60.6 | | | |
| 2 | 1 | 56.6 | 60.4 | 84.5 | 4.44 |
| | 2 | 57.9 | | | |
| | 3 | 66.8 | | | |
| 5 | 1 | 68.6 | 65.1 | 91.0 | 4.51 |
| | 2 | 67.0 | | | |
| | 3 | 59.7 | | | |
| 10 | 1 | 63.0 | 63.0 | 88.1 | 4.48 |
| | 2 | 64.3 | | | |
| | 3 | 61.7 | | | |
| 15 | 1 | 60.4 | 60.7 | 84.9 | 4.44 |
| | 2 | 60.0 | | | |
| | 3 | 61.8 | | | |
| 20 | 1 | 63.9 | 61.1 | 85.4 | 4.45 |
| | 2 | 62.4 | | | |
| | 3 | 56.9 | | | |
| 30 | 1 | 58.0 | 58.0 | 81.1 | 4.40 |
| | 2 | 58.3 | | | |
| | 3 | 57.7 | | | |
| 60 | 1 | 39.9 | 43.2 | 60.4 | 4.10 |
| | 2 | 49.4 | | | |
| | 3 | 40.2 | | | |
| 120 | 1 | 21.7 | 21.1 | 29.5 | 3.38 |
| | 2 | 19.6 | | | |
| | 3 | 21.9 | | | |
| 240 | 1 | 5.41 | 6.90 | 9.65 | 2.27 |
| | 2 | 8.02 | | | |
| | 3 | 7.26 | | | |
| 1440 | 1 | blq | 0.00 | 0.0 | NC |
| | 2 | blq | | | |
| | 3 | blq | | | |

### DISCUSSION

Test Article F429 ion of [M+3H]³⁺ of m/z 938.45 was monitored in positive MS polarity. A Strata X 96-well SPE plate was used for sample preparation. An eight-point standard calibration curve with the range of 2.5 to 100 µg/mL was used for F429 plasma analysis. The analytical method is highly specific with a lower limit of quantitation (LLOQ) at 2.5 gg/mL.

### Conclusion

The Test Article F429 plasma stability was performed at 37°C, and its stability half-life in CD-1 mouse plasma was 73.2 min. Test Article F429 still could be found at 4 hours (9.65% remaining). The analytical method has very good specificity.

### EXAMPLE 10

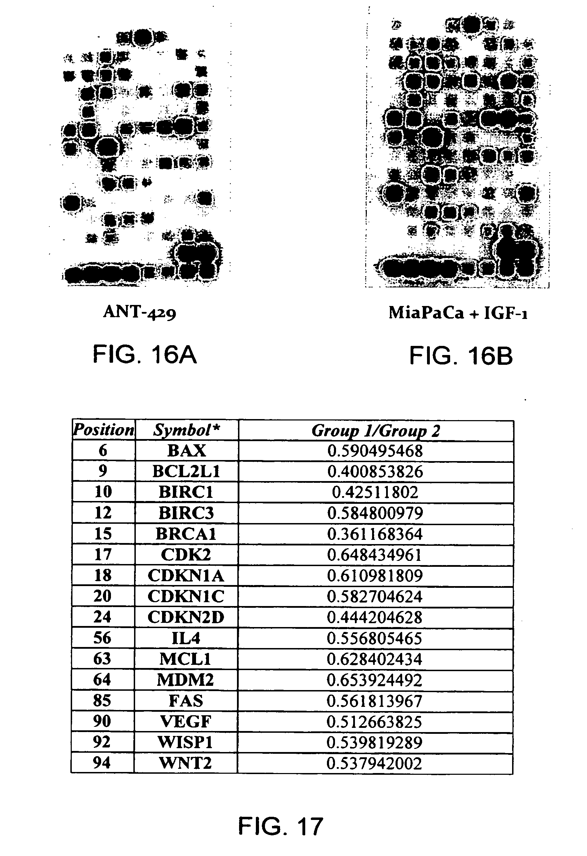

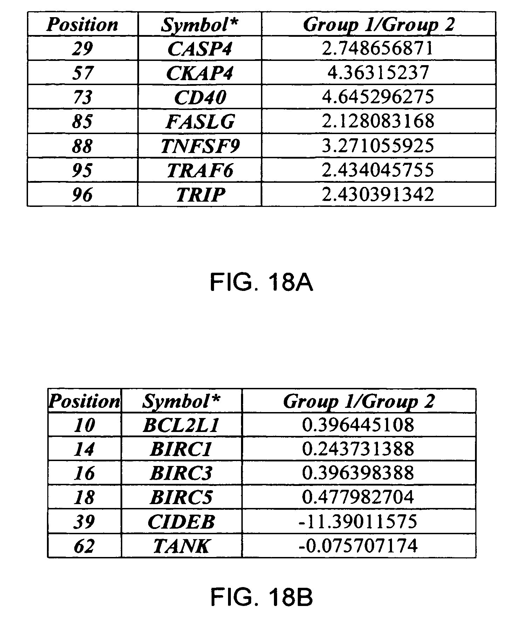

MiaPaCa cells were treated with IGF-1 (25ng/ml) +/- ANT-429 for 24 hours and then washed 3X with PBS, scrap and process for gene expression. Figures 16A and 16B are gene arrays in which gene expression changes were analyzed between MiaPaCa cells grown with IGF-1 as compared to those with ANT-429. Figure 17 provides a list of genes which were shown to be down-regulated in ANT-429 treated cells. ANT-429 was shown to modulate apoptosis genes in a study which examined changes in apoptosis gene expression between MiaPaCa cells grown with IGF-1 vs. treatment with ANT-429. Figures 18A and 18B provide a list of genes that were up-regulated or down-regulated when treated with ANT-429.

### EXAMPLE 11

### Animal Studies Inhibition

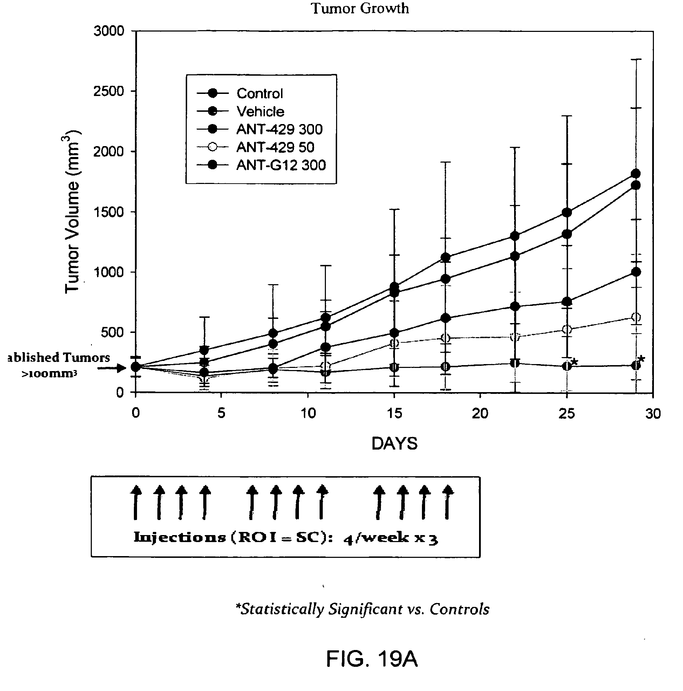

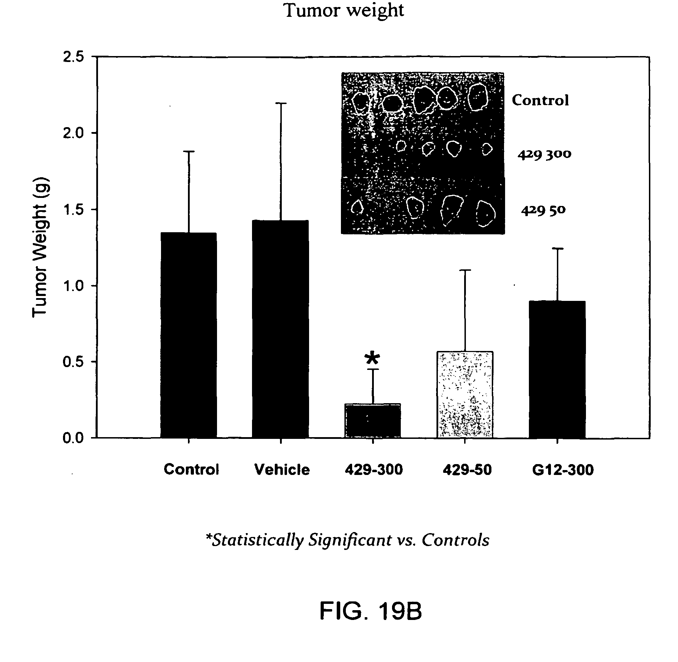

Nude mice were inoculated with MiaPaCa cells (one million cells in matrigel) on the flank and followed until tumors reached a volume of greater than 100 mm³. Animals were randomized and placed into 5 treatment groups: control (no injections), vehicle, ANT-G12, HP to a different target at 300 ug/injection (12 mg/kg), ANT-429 at 50 ug/injection (2.5 mg/kg), and ANT-429 at 300 ug/injection (12 mg/kg). Animals were injected by the subcutaneous route 4 times per week for 3 weeks and followed for an additional 2 weeks without injections. Tumors were measured twice weekly. Animals were sacrificed and the tumors excised, weighed and photographed. The results demonstrate active growth in the control and vehicle groups. There was significant inhibition of growth in the ANT-429 group at 15 mg/kg including one complete remission. Two out of five of the animals responded to treatment with 2.5 mg/kg group including one complete remission (Figures 19A and 19B). These results demonstrate that ANT-429 inhibits tumor growth.

### EXAMPLE 12

### Toxicity Studies

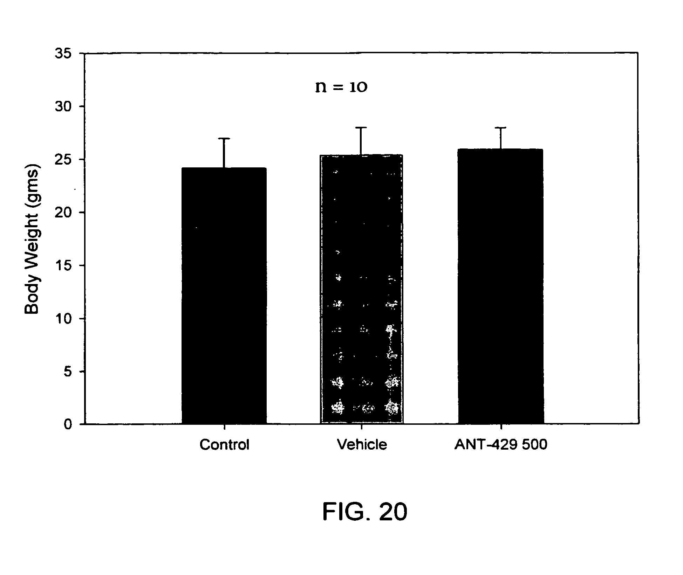

Nude mice were inoculated with MiaPaCa cells (one million cells in matrigel) on the flank and followed until tumors reached a volume of greater than 100 mm³. Animals were randomized and placed into 3 treatment groups: control (no injections), vehicle, ANT-429 and vehicle at 500 ug/injection (20 mg/kg). Animals were injected by the subcutaneous route 4 times per week for 3 weeks and followed for an additional 2 weeks without injections. Tumors and body weight were measured twice weekly. None of the animals treated with ANT-429 demonstrated any apparent adverse effects (Figure 20). These results demonstrate that ANT-429 is not toxic *in vivo.*

### CONSTRUCTION

The description of the various aspects of the invention provided herein is to be construed according to the following principles.

The use of the terms "a" and "an" and "the" and similar referents in the context of describing the invention are to be construed to cover both the singular and the plural, unless otherwise indicated herein or clearly contradicted by context.

Unless otherwise stated, all exact values provided herein are representative of corresponding approximate values (e.g., all exact exemplary values provided with respect to a particular factor or measurement can be considered to also provide a corresponding approximate measurement, modified by "about," where appropriate).

Recitation of ranges of values herein are merely intended to serve as a shorthand method of referring individually to each separate value falling in the range that is within the same order of magnitude and same level of significance (i.e., all similarly significant figures) as the lower end point of the range, unless otherwise indicated herein, and each separate value is incorporated into the specification as if it were individually recited herein. Thus, for example, a range of 1-100 herein provides support for each integer between (and including) 1-100 (i.e., 1, 2, 3, 4, ... 98, 99, and 100) and a range of 0.1-1 provides support for each value in the same order of magnitude and level of significance as 0.1 between and including these endpoints (i.e., 0.1, 0.2, 0.3, ... 0.9, 1.0).

The description herein of any aspect or embodiment of the invention using terms such as "comprising", "having," "including," or "containing" with reference to an element or elements is intended to provide support for a similar aspect or embodiment of the invention that "consists of, "consists essentially of, or "substantially comprises" that particular element or elements, unless otherwise stated or clearly contradicted by context (e.g., a composition described herein as comprising a particular element should be understood as also describing a composition consisting of that element, unless otherwise stated or clearly contradicted by context). A protein or peptide that consists of a particular sequence or peptide derivative described herein typically retains sufficient structural similarity to the referenced sequence of peptide derivative to allow the protein or peptide to exhibit similar biological properties of the sequence or peptide derivative (e.g., IGF-1R binding, IGF-1R antagonism, etc.).

In some cases formulas have been used herein to describe a family of sequences. It will be understood that this is done for the sake of convenience only and that each sequence falling within the parameters of the formula is to be considered also implicitly to be individually disclosed herein as an aspect of the disclosure.

All headings and sub-headings are used herein for convenience only and should not be construed as limiting the invention in any way.

The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

The citation and incorporation of patent documents herein is done for convenience only and does not reflect any view of the validity, patentability, and/or enforceability of such patent documents.

This invention includes all modifications and equivalents of the subject matter recited in the claims and/or aspects of the invention included herein as permitted by applicable law.

### EQUIVALENTS

Those skilled in the art will recognize, or be able to ascertain using no more than routine experimentation, numerous equivalents to the specific procedures described herein. Such equivalents are considered to be within the scope of the present invention and are covered by the following claims. The appropriate components, processes, and methods of those patents, applications and other documents may be selected for the present invention and embodiments thereof.

## Claims

1. An isolated peptide, comprising:
a peptide capable of binding Insulin-like growth factor 1 Receptor (IGF-1R),
wherein the sequence of said peptide comprises an amino acid sequence having at least 98% identity to SEQ ID. NO: 18 (F429).

2. The peptide of claim 1, wherein the sequence of said peptide comprises SEQ ID. NO: 18 (F429), and preferably wherein the sequence of said peptide consists of SEQ ID. NO: 18 (F429).

3. A pharmaceutical composition, comprising:
a peptide capable of binding IGF-1R in an amount that is effective to reduce angiogenesis and/or cancer progression in a mammalian host, wherein the sequence of said peptide comprises an amino acid sequence having at least 98% identity to SEQ ID. NO: 18 (F429).

4. The pharmaceutical composition of claim 3, wherein said mammalian host is a human host.

5. The pharmaceutical composition of claim 4, wherein the sequence of said peptide comprises SEQ ID. NO: 18 (F429), and preferably wherein the sequence of said peptide consists of SEQ ID. NO: 18 (F429).

6. A peptide capable of binding IGF-1R, wherein the sequence of said peptide comprises an amino acid sequence having a least 98% identity to SEQ ID. NO: 18 (F429) for use in a method of treating cancer.

7. The peptide of claim 6, wherein said cancer is one wherein IGF-1 and/or IGF-1R is expressed.

8. The peptide of claim 7, wherein the sequence of said peptide comprises SEQ ID. NO: 18 (F429), and preferably wherein the sequence of said peptide consists of SEQ ID. NO: 18 (F429).

9. A peptide according to claim 1 or 2 for use in therapy.

10. Use of a peptide according to claim 1 or 2 in the preparation of a medicament for the treatment of cancer.

11. A peptide antagonist of IGF-1R comprising the sequence FYxxLxxL and having 98% identity to SEQ ID NO: 18 (F429) for use in a method of treating cancer wherein IGF-1 and/or IGF-1R are expressed.

12. A peptide capable of binding IGF-1R in an amount that is effective to reduce angiogenesis and/or cancer progression in a mammalian host, wherein said peptide comprises the sequence FYxxLxxL and has 98% identity to SEQ ID NO: 18 (F429) for use in therapy.

13. Use of a peptide capable of binding IGF-1R in an amount that is effective to reduce angiogenesis and/or cancer progression in a mammalian host, wherein said peptide comprises the sequence FYxxLxxL and has 98% identity to SEQ ID NO: 18 (F429) in the preparation of a medicament for the treatment of cancer.

14. The peptide of claim 11 or 12, or the use of claim 13, wherein the peptide comprises SEQ ID. NO: 18 (F429).

## Patentansprüche

1. Isoliertes Peptid, umfassend:
ein Peptid, das zur Bindung von Insulin-ähnlichem Wachstumsfaktor-Rezeptor 1 (IGF-1 R) fähig ist,
wobei die Sequenz des Peptids eine Aminosäuresequenz umfasst, die zu wenigstens 98% Identität zu SEQ ID NO: 18 (F429) aufweist.

2. Peptid gemäß Anspruch 1, wobei die Sequenz des Peptids SEQ ID NO: 18 (F429) umfasst, und wobei die Sequenz des Peptids vorzugsweise in SEQ ID NO: 18 (F429) besteht.

3. Pharmazeutische Zusammensetzung, umfassend:
ein Peptid, das zur Bindung von IGF-1R in einer Menge fähig ist, die darin wirksam ist, die Angiogenese und/oder das Fortschreiten von Krebs in einem Säugerwirt zu verringern, wobei die Sequenz des Peptids eine Aminosäuresequenz umfasst, die zu wenigstens 98% Identität zu SEQ ID NO: 18 (F429) aufweist.

4. Pharmazeutische Zusammensetzung gemäß Anspruch 3, wobei der Säugerwirt ein menschlicher Wirt ist.

5. Pharmazeutische Zusammensetzung gemäß Anspruch 4, wobei die Sequenz des Peptids SEQ ID NO: 18 (F429) umfasst, und wobei die Sequenz des Peptids vorzugsweise in SEQ ID NO: 18 (F429) besteht.

6. Peptid, das zur Bindung von IGF-1R fähig ist, wobei die Sequenz des Peptids eine Aminosäuresequenz umfasst, die zu wenigstens 98% Identität zu SEQ ID NO: 18 (F429) aufweist, zur Verwendung bei einer Methode zur Behandlung von Krebs.

7. Peptid gemäß Anspruch 6, wobei der Krebs ein solcher ist, in welchem IGF-1 und/oder IGF-1 R exprimiert wird.

8. Peptid gemäß Anspruch 7, wobei die Sequenz des Peptids SEQ ID NO: 18 (F429) umfasst, und wobei die Sequenz des Peptids vorzugsweise in SEQ ID NO: 18 (F429) besteht.

9. Peptid gemäß Anspruch 1 oder 2, zur Verwendung in der Therapie.

10. Verwendung eines Peptids gemäß Anspruch 1 oder 2 in der Herstellung eines Medikaments für die Behandlung von Krebs.

11. Peptid-Antagonist von IGF-1R, umfassend die Sequenz FYxxLxxL und der zu wenigstens 98% Identität zu SEQ ID NO: 18 (F429) aufweist, zur Verwendung bei einer Methode zur Behandlung eines Krebs, in welchem IGF-1 und/oder IGF-1R exprimiert werden.

12. Peptid, das zur Bindung von IGF-1R in einer Menge fähig ist, die darin wirksam ist, die Angiogenese und/oder das Fortschreiten von Krebs in einem Säugerwirt zu verringern, wobei das Peptid die Sequenz FYxxLxxL ausweist und es zu wenigstens 98% Identität zu SEQ ID NO: 18 (F429) aufweist, zur Verwendung in der Therapie.

13. Verwendung eines Peptids, das zur Bindung von IGF-1R in einer Menge fähig ist, die darin wirksam ist, die Angiogenese und/oder das Fortschreiten von Krebs in einem Säugerwirt zu verringern, wobei das Peptid die Sequenz FYxxLxxL ausweist und es zu wenigstens 98% Identität zu SEQ ID NO: 18 (F429) aufweist, in der Herstellung eines Medikaments für die Behandlung von Krebs.

14. Peptid gemäß Anspruch 11 oder 12, oder die Verwendung gemäß Anspruch 13, wobei das Peptid SEQ ID NO: 18 (F429) umfasst.

## Revendications

1. Peptide isolé, comprenant :
un peptide capable de se lier à un récepteur du facteur de croissance 1 analogue à l'insuline (IGF-1R), dans lequel la séquence dudit peptide comprend une séquence d'acides aminés ayant une identité d'au moins 98% avec SEQ ID N° : 18 (F429).

2. Peptide selon la revendication 1, dans lequel la séquence dudit peptide comprend SEQ ID N° : 18 (F429) et de préférence, dans lequel la séquence dudit peptide consiste en SEQ ID N° : 18 (F429).

3. Composition pharmaceutique comprenant :
un peptide capable de se lier à l'IGF-1R en une quantité qui est efficace pour réduire l'angiogenèse et/ou la progression du cancer chez un hôte mammifère, la séquence dudit peptide comprenant une séquence d'acides aminés ayant une identité d'au moins 98% avec SEQ ID N° : 18 (F429).

4. Composition pharmaceutique selon la revendication 3, dans laquelle ledit hôte mammifère est un hôte humain.

5. Composition pharmaceutique selon la revendication 4, dans laquelle la séquence dudit peptide comprend SEQ ID N° : 18 (F429) et de préférence dans laquelle la séquence dudit peptide consiste en SEQ ID N° : 18 (F429).

6. Peptide capable de se lier à l'IGF-1R, dans lequel la séquence dudit peptide comprend une séquence d'acides aminés ayant une identité d'au moins 98% avec SEQ ID N° : 18 (F429) à utiliser dans un procédé de traitement du cancer.

7. Peptide selon la revendication 6, ledit cancer étant un cancer dans lequel IGF-1 et/ou IGF-1R est exprimé.

8. Peptide selon la revendication 7, dans lequel la séquence dudit peptide comprend SEQ ID N° : 18 (F429) et de préférence, la séquence dudit peptide consiste en SEQ ID N° : 18 (F429).

9. Peptide selon la revendication 1 ou 2 à utiliser en traitement.

10. Utilisation d'un peptide selon la revendication 1 ou 2 dans la préparation d'un médicament pour le traitement du cancer.

11. Antagoniste peptidique de l'IGF-1R comprenant la séquence FYxxLxxL et ayant une identité de 98% avec SEQ ID N° : 18 (F429) à utiliser dans un procédé de traitement du cancer dans lequel IGF-1 et/ou IGF-1R sont exprimés.

12. Peptide capable de se lier à l'IGF-1R en une quantité qui est efficace pour réduire l'angiogenèse et/ou la progression du cancer chez un hôte mammifère, ledit peptide comprenant la séquence FYxxLxxL et ayant une identité de 98% avec SEQ ID N° : 18 (F429) à utiliser en traitement.

13. Utilisation d'un peptide capable de se lier à l'IGF-1R en une quantité qui est efficace pour réduire l'angiogenèse et/ou la progression du cancer chez un hôte mammifère, ledit peptide comprenant la séquence FYxxLxxL et ayant une identité de 98% avec SEQ ID N° : 18 (F429) dans la préparation d'un médicament pour le traitement du cancer.

14. Peptide selon la revendication 11 ou 12 ou utilisation selon la revendication 13, le peptide comprenant SEQ ID N° : 18 (F429).
